# EUROPEAN PATENT APPLICATION

(11) **EP 4 725 486 A2**
(43) Date of publication of application: **15.04.2026**
(21) Application number: 25224025.4
(22) Date of filing: 01.09.2023
(51) Int. Cl.: A61K 31/499

(54) **CRYSTALLINE FORMS OF 5-(3,4-DIFLUOROBENZYL)-8-((1R,4R)-4-METHYLCYCLOHEXYL)-6,9-DIOXO-2,5,8-TRIAZASPIRO[3.5]NONANE-2-CARBALDEHYDE**

(30) Priority: 02.09.2022 WO PCT/CN2022/116765
(62) Divisional of application: 23783204.3
(71) Applicant: Cytokinetics, Inc., South San Francisco, CA 94080 (US)
(72) Inventor: CHUANG, Chihyuan, San Francisco, 94080 (US); WANG, Xiaolin, Beijing, 100176 (CN)
(74) Representative: Carpmaels & Ransford LLP

(57) **Abstract**

Provided herein are crystalline forms of 5-(3,4-difluorobenzyl)-8-((1r,4r)-4-methylcyclohexyl)-6,9-dioxo-2,5,8-triazaspiro[3.5]nonane-2-carbaldehyde, compositions thereof, methods of preparation thereof, and methods of their uses.

## Description

### CROSS-REFERENCE TO RELATED APPLICATIONS

This application claims priority to and benefit of PCT Application No. CN2022/116765 filed September 2, 2022, the disclosure of which is hereby incorporated by reference in its entirety.

### FIELD

Provided herein are polymorphs of 5-(3,4-difluorobenzyl)-8-((1r,4r)-4-methylcyclohexyl)-6,9-dioxo-2,5,8-triazaspiro[3.5]nonane-2-carbaldehyde, compositions thereof, methods of preparation thereof, and methods of their uses.

### BACKGROUND

The cardiac sarcomere is composed of a network of contractile and structural proteins that regulate cardiac muscle function. The components of the cardiac sarcomere present targets for the treatment of various cardiac diseases and conditions, for example by increasing contractility or facilitating complete relaxation to modulate systolic and diastolic function, respectively. The force and speed of cardiac muscle contraction is a major determinant of organ function and is modulated by the cyclical interactions of actin and myosin. Regulation of actin and myosin binding is determined by a network of myofilament regulatory proteins and the level of intracellular Ca²⁺. The troponin complex and tropomyosin are thin filament proteins which govern the availability of actin binding sites, and the essential and regulatory light chains, and myosin binding protein C modulate the position and mechanical properties of myosin.

Abnormalities in the cardiac sarcomere have been identified as the driving cause for a variety of cardiac diseases and conditions, such as hypertrophic cardiomyopathy (HCM) and heart failure with preserved ejection fraction (HFpEF). Mutations in the proteins of the sarcomere cause disease by rendering the cardiac muscle either 'hyper' or 'hypo' contractile. Modulators of the cardiac sarcomere can be used to rebalance contractility and stop or reverse the course of disease.

Current agents that target the cardiac sarcomere, such as inotropes (drugs that increase the contractile ability of the heart) are poorly selective for cardiac tissue, which leads to recognized adverse effects that limit their use. These adverse effects include cell damage caused by an increased rate of energy expenditure, exacerbation of relaxation abnormalities, and potential arrhythmogenic side effects that may result from increased cytosolic Ca²⁺ and cyclic AMP concentrations in the inotropically stimulated myocardium. Given the limitations of current agents, new approaches are needed to improve cardiac function in HCM and HFpEF.

There remains a great need for agents that exploit new mechanisms of action and which may have better outcomes in terms of relief of symptoms, safety, and patient mortality, both short-term and long-term. New agents with an improved therapeutic index over current agents will provide a means to achieve these clinical outcomes. The selectivity of agents directed at the cardiac sarcomere (for example, by targeting cardiac myosin) has been identified as an important means to achieve this improved therapeutic index. 5-(3,4-difluorobenzyl)-8-((1r,4r)-4-methylcyclohexyl)-6,9-dioxo-2,5,8-triazaspiro[3.5]nonane-2-carbaldehyde (compound 1) is a selective allosteric inhibitor of cardiac myosin that have little to no effect on smooth muscle myosin. Benefits of this compound include a wider therapeutic index, less impact on cardiac relaxation, better pharmacokinetics, and better safety and therefore it provides a potential treatment for cardiac diseases and conditions.

To move a drug candidate such as compound 1 to a viable pharmaceutical product, it is important to understand whether the drug candidate has polymorph forms, as well as the relative stability and interconversions of these forms under conditions likely to be encountered upon large-scale production, transportation, storage and pre-usage preparation. The ability to control and produce a stable polymorph with a robust manufacturing process can be key for regulatory approval and marketing. Large scale production processes to prepare compound 1 with high purity can be improved by using particular crystalline forms. Accordingly, there is a need for new crystalline forms of compound 1 with desirable chemical and physical stabilities, and formulations and uses of the same.

### BRIEF SUMMARY

In one aspect, provided herein are polymorphs of 5-(3,4-difluorobenzyl)-8-((1r,4r)-4-methylcyclohexyl)-6,9-dioxo-2,5,8-triazaspiro[3.5]nonane-2-carbaldehyde (compound 1).

In another aspect, provided herein are methods of preparing polymorphs of compound 1.

In another aspect, provided herein are compositions containing the polymorphs of compound 1 as described herein.

In another aspect, provided herein are methods of treating a heart disease in a subject in need thereof using polymorphs of compound 1.

### DESCRIPTION OF THE DRAWINGS

FIG. 1A shows an experimental X-ray powder diffraction (XRPD) pattern of crystalline Form I of 5-(3,4-difluorobenzyl)-8-((1r,4r)-4-methylcyclohexyl)-6,9-dioxo-2,5,8-triazaspiro[3.5]nonane-2-carbaldehyde.
FIG. 1B shows differential scanning calorimetry (DSC) and thermographic analysis (TGA) graphs of crystalline Form I of 5-(3,4-difluorobenzyl)-8-((1r,4r)-4-methylcyclohexyl)-6,9-dioxo-2,5,8-triazaspiro[3.5]nonane-2-carbaldehyde.
FIG. 1C shows a Gravimetric Vapor Sorption (GVS) graph of crystalline Form I of 5-(3,4-difluorobenzyl)-8-((1r,4r)-4-methylcyclohexyl)-6,9-dioxo-2,5,8-triazaspiro[3.5]nonane-2-carbaldehyde.
FIG. 1D shows an overlay of XRPD patterns of crystalline Form I of 5-(3,4-difluorobenzyl)-8-((1r,4r)-4-methylcyclohexyl)-6,9-dioxo-2,5,8-triazaspiro[3.5]nonane-2-carbaldehyde before and after storage for 7 days at 40°C/75% RH and 25°C/97% RH (from top to bottom: Form I after being stored for 7 days at 40°C/75% RH, Form I after being stored for 7 days at 25°C/97% RH, Form I before being stored for 7 days at 40°C/75% RH and 25°C/97% RH).
FIG. 1E shows XRPD patterns of crystalline Form I of 5-(3,4-difluorobenzyl)-8-((1r,4r)-4-methylcyclohexyl)-6,9-dioxo-2,5,8-triazaspiro[3.5]nonane-2-carbaldehyde before and after GVS measurement (top: after GVS; bottom: before GVS).
FIG. 2A shows an experimental XRPD pattern of crystalline Form II of 5-(3,4-difluorobenzyl)-8-((1r,4r)-4-methylcyclohexyl)-6,9-dioxo-2,5,8-triazaspiro[3.5]nonane-2-carbaldehyde.
FIG. 2B shows DSC and TGA graphs of crystalline Form II of 5-(3,4-difluorobenzyl)-8-((1r,4r)-4-methylcyclohexyl)-6,9-dioxo-2,5,8-triazaspiro[3.5]nonane-2-carbaldehyde.
FIG. 2C shows a GVS graph of crystalline Form II of 5-(3,4-difluorobenzyl)-8-((1r,4r)-4-methylcyclohexyl)-6,9-dioxo-2,5,8-triazaspiro[3.5]nonane-2-carbaldehyde.
FIG. 2D shows an overlay of XRPD patterns of crystalline Form II of 5-(3,4-difluorobenzyl)-8-((1r,4r)-4-methylcyclohexyl)-6,9-dioxo-2,5,8-triazaspiro[3.5]nonane-2-carbaldehyde before and after storage for 8 days at 40°C/75% RH and 25°C/97% RH (from top to bottom: Form II after storage for 8 days at 25°C/97% RH, Form II after storage for 8 days at 40°C/75% RH, Form II before storage for 8 days at 40°C/75% RH and 25°C/97% RH).
FIG. 2E shows an overlay of XRPD patterns of crystalline Form II of 5-(3,4-difluorobenzyl)-8-((1r,4r)-4-methylcyclohexyl)-6,9-dioxo-2,5,8-triazaspiro[3.5]nonane-2-carbaldehyde before and after GVS measurement (top: after GVS; bottom: before GVS).
FIG. 3A shows an experimental XRPD pattern of crystalline Form III of 5-(3,4-difluorobenzyl)-8-((1r,4r)-4-methylcyclohexyl)-6,9-dioxo-2,5,8-triazaspiro[3.5]nonane-2-carbaldehyde.
FIG. 3B shows DSC and TGA graphs of crystalline Form III of 5-(3,4-difluorobenzyl)-8-((1r,4r)-4-methylcyclohexyl)-6,9-dioxo-2,5,8-triazaspiro[3.5]nonane-2-carbaldehyde.
FIG. 3C shows an overlay XRPD patterns of crystalline Form III of 5-(3,4-difluorobenzyl)-8-((1r,4r)-4-methylcyclohexyl)-6,9-dioxo-2,5,8-triazaspiro[3.5]nonane-2-carbaldehyde before and after storage for 7 days at 40°C/75% RH, and the XRPD pattern of crystalline Form II (from top to bottom: Form III after storage for 7 days at 40°C/75% RH, Form III before storage for 7 days at 40°C/75% RH, Form II).

### DETAILED DESCRIPTION

### Definitions

As used herein and in the appended claims, the singular forms "a", "an" and "the" include plural forms, unless the context clearly dictates otherwise.

As used herein, and unless otherwise specified, the terms "about" and "approximately," when used in connection with doses, amounts, or weight percent of ingredients of a composition or a dosage form, mean a dose, amount, or weight percent that is recognized by those of ordinary skill in the art to provide a pharmacological effect equivalent to that obtained from the specified dose, amount, or weight percent. Specifically, where applicable, the terms "about" and "approximately," when used in this context, contemplate a dose, amount, or weight percent within 15% of the specified dose, amount, or weight percent.

As used herein, the term "polymorph", "polymorphic", "polymorphic form", or "crystalline form" refers to a crystalline form of a compound. Different polymorphs may have different physical properties such as, for example, melting temperatures, heats of fusion, solubilities, dissolution rates, and/or vibrational spectra as a result of the arrangement or conformation of the molecules or ions in the crystal lattice. The differences in physical properties exhibited by polymorphs may affect pharmaceutical parameters, such as storage stability, compressibility, density (important in formulation and product manufacturing), and dissolution rate (an important factor in bioavailability). Differences in stability can result from changes in chemical reactivity (*e.g.,* differential oxidation, such that a dosage form discolors more rapidly when comprised of one polymorph than when comprised of another polymorph), mechanical changes (*e.g.,* tablets crumble on storage as a kinetically favored polymorph converts to thermodynamically more stable polymorph), or both (*e.g.,* tablets of one polymorph are more susceptible to breakdown at high humidity). As a result of solubility/dissolution differences, in the extreme case, some polymorphic transitions may result in lack of potency or, at the other extreme, toxicity. In addition, the physical properties of a crystalline form may be important in processing; for example, one polymorph might be more likely to form solvates or might be difficult to filter and wash free of impurities (*e.g.,* particle shape and size distribution might be different between polymorphs).

As used herein, "therapeutically effective amount" indicates an amount that results in a desired pharmacological and/or physiological effect for the condition. The effect may be therapeutic in terms of a partial or complete cure for the condition and/or adverse effect attributable to the condition.

As used herein, the term "pharmaceutically acceptable carrier," and cognates thereof, refers to adjuvants, binders, diluents, *etc.* known to the skilled artisan that are suitable for administration to an individual (*e.g.,* a mammal or non-mammal). Combinations of two or more carriers are also contemplated. The pharmaceutically acceptable carrier(s) and any additional components, as described herein, should be compatible for use in the intended route of administration (*e.g.,* oral, parenteral) for a particular dosage form, as would be recognized by the skilled artisan.

The terms "treat," "treating," and "treatment" are meant to include alleviating or abrogating a disorder, disease, or condition, or one or more of the symptoms associated with the disorder, disease, or condition; or to slowing the progression, spread or worsening of a disease, disorder or condition or of one or more symptoms thereof. Often, the beneficial effects that a subject derives from a therapeutic agent do not result in a complete cure of the disease, disorder or condition.

The term "subject" refers to an animal, including, but not limited to, a primate (*e.g.,* human), mammal, monkey, cow, pig, sheep, goat, horse, dog, cat, rabbit, rat, or mouse. The terms "subject" and "patient" are used interchangeably herein in reference, for example, to a mammalian subject, such as a human.

As used herein, the term "substantially as shown in" when referring, for example, to an XRPD pattern, a DSC graph, a TGA graph, or a GVS graph, includes a pattern or graph that is not necessarily identical to those depicted herein, but that falls within the limits of experimental error or deviations when considered by one of ordinary skill in the art.

As used herein, the term "substantially free of" means that the composition comprising the crystalline form contains less than 50%, less than 40%, less than 30%, less than 20%, less than 15%, less than 10%, less than 5%, less than 4%, less than 3%, less than 2%, or less than 1% by weight of the indicated substance or substances.

### Polymorphs

In one aspect, provided herein are polymorphs of 5-(3,4-difluorobenzyl)-8-((1r,4r)-4-methylcyclohexyl)-6,9-dioxo-2,5,8-triazaspiro[3.5]nonane-2-carbaldehyde, also called compound **1**, having the structure shown below,

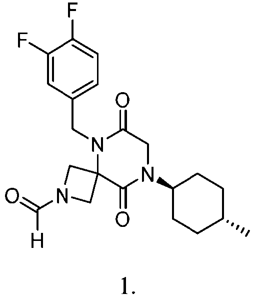

The polymorphs may have properties such as bioavailability and stability under certain conditions that are suitable for medical or pharmaceutical uses.

A polymorph of compound 1 may provide the advantages of bioavailability and stability and may be suitable for use as an active agent in a pharmaceutical composition. Variations in the crystal structure of a pharmaceutical drug substance may affect the dissolution rate (which may affect bioavailability, *etc.*)*,* manufacturability (*e.g.,* ease of handling, ease of purification, ability to consistently prepare doses of known strength, *etc.*) and stability (*e.g.,* thermal stability, shelf life (including resistance to degradation), *etc*.) of a pharmaceutical drug product. Such variations may affect the methods of preparation or formulation of pharmaceutical compositions in different dosage or delivery forms, such as solid oral dosage forms including tablets and capsules. Compared to other forms such as non-crystalline or amorphous forms, polymorphs may provide desired or suitable hygroscopicity or lack thereof, particle size control, dissolution rate, solubility, purity, physical and chemical stability, manufacturability, yield, reproducibility, and/or process control. Thus, polymorphs of compound 1 may provide advantages of improving the manufacturing process of the active agent or the stability or storability of a drug product form of the active agent, or having suitable bioavailability and/or stability as an active agent.

The use of certain conditions, such as the use of different solvents and/or temperatures, has been found to produce different polymorphs of compound 1 or a solvate thereof, including crystalline Forms I-III described herein, which may exhibit one or more favorable characteristics described herein. The processes for the preparation of the polymorphs described herein and characterization of these polymorphs are described in greater detail below.

### Form I

In some embodiments, provided herein is crystalline Form I of 5-(3,4-difluorobenzyl)-8-((1r,4r)-4-methylcyclohexyl)-6,9-dioxo-2,5,8-triazaspiro[3.5]nonane-2-carbaldehyde.

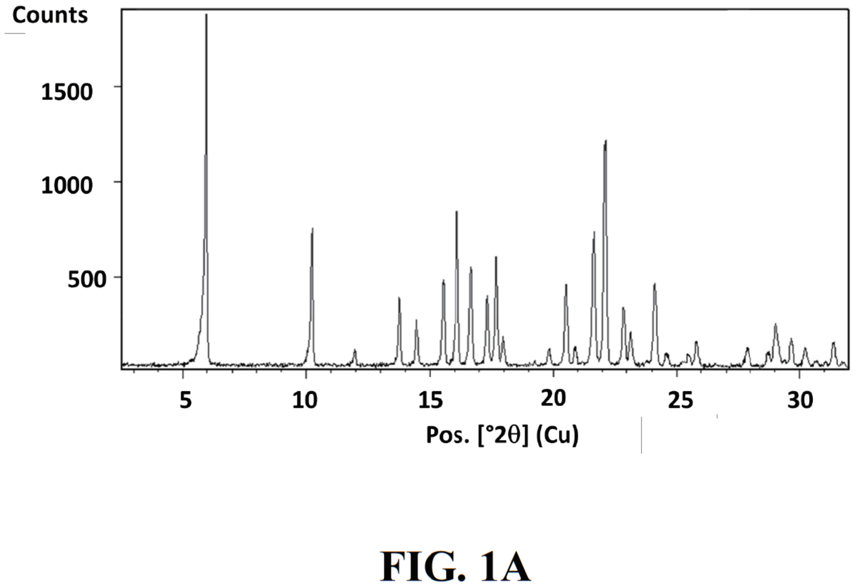

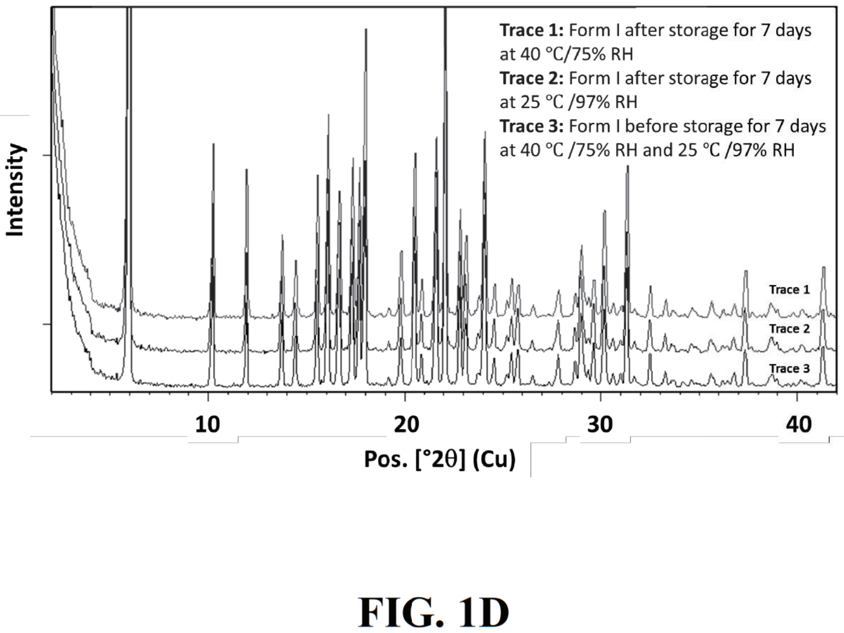

In some embodiments, Form I has an XRPD pattern substantially as shown in FIG. 1A.

Angles 2-theta and relative peak intensities observed for Form I using XRPD are shown in Table 1.

**TABLE 1**

| **Angle / 2θ** | **Intensity / %** |
|---|---|
| 6.0 | 100.0 |
| 10.2 | 9.5 |
| 11.9 | 6.7 |
| 13.7 | 5.5 |
| 14.4 | 4.1 |
| 15.5 | 8.7 |
| 16.0 | 14.2 |
| 16.6 | 9.8 |
| 17.3 | 9.9 |
| 17.6 | 9.9 |
| 17.9 | 14.4 |
| 19.8 | 3.9 |
| 20.5 | 12.0 |
| 20.9 | 2.7 |
| 21.6 | 14.6 |
| 22.1 | 29.1 |
| 22.8 | 8.0 |
| 23.1 | 5.6 |
| 24.1 | 14.3 |
| 24.5 | 2.3 |
| 25.4 | 2.7 |
| 25.7 | 3.0 |
| 27.8 | 2.6 |
| 28.6 | 2.0 |
| 29.0 | 6.1 |
| 29.6 | 3.5 |
| 30.1 | 6.2 |

In some embodiments, crystalline Form I has an XRPD pattern displaying at least two, at least three, at least four, at least five, at least six, at least seven, at least eight, at least nine, or at least ten of the peaks at angles 2-theta with the greatest intensity in the XRPD pattern substantially as shown in FIG. 1A or as provided in Table 1. It should be understood that relative intensities can vary depending on a number of factors, including sample preparation, mounting, and the instrument and analytical procedure and settings used to obtain the spectrum. Relative peak intensities and peak assignments can vary within experimental error. In some embodiments, peak assignments listed herein, including for crystalline Form I, can vary by about ±0.6 degrees, ±0.4 degrees, ±0.2 degrees, or ±0.1 degrees 2-theta.

In some embodiments, crystalline Form I has an XRPD pattern comprising peaks at angles 2-theta of 6.0±0.2, 10.2±0.2, 21.6±0.2, and 22.1±0.2 degrees. In some embodiments, the crystalline Form I has an XRPD pattern comprising additional peaks at angles 2-theta of 17.9±0.2 and 24.1±0.2 degrees. In some embodiments, crystalline Form I has an XRPD pattern further comprising additional peaks at angles 2-theta of 16.0±0.2, 16.6±0.2, 17.3±0.2, 17.6±0.2, and 20.5±0.2 degrees. In some embodiments, crystalline Form I has an XRPD pattern comprising peaks at angles 2-theta of 6.0±0.2, 10.2±0.2, 11.9±0.2, 13.7±0.2, 14.4±0.2, 15.5±0.2, 16.0±0.2, 16.6±0.2, 17.3±0.2, 17.6±0.2, 17.9±0.2, 19.8±0.2, 20.5±0.2, 20.9±0.2, 21.6±0.2, 22.1±0.2, 22.8±0.2, 23.1±0.2, 24.1±0.2, 24.5±0.2, 25.4±0.2, 25.7±0.2, 27.8±0.2, 28.6±0.2, 29.0±0.2, 29.6±0.2, and 30.1±0.2 degrees. It is to be understood that additional peaks in the XRPD pattern other than those shown in FIG. 1A or as provided in Table 1 may be observed, for instance, due to the presence of impurities, solvent, or other polymorphs or amorphic forms present in the test sample.

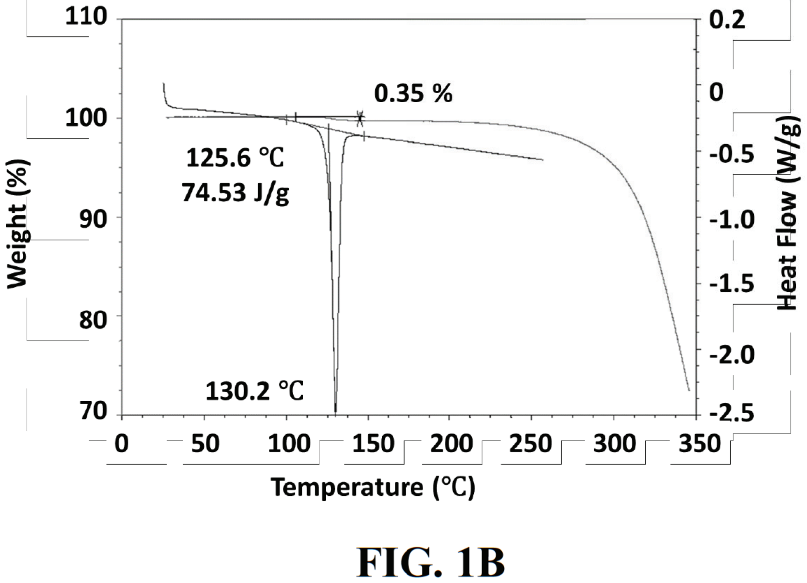

In some embodiments, Form I has a differential scanning calorimetry (DSC) graph substantially as shown in FIG. 1B. In some embodiments, Form I is characterized as having an endotherm onset at about 125.6 °C as determined by DSC. In some embodiments, Form I is characterized as having an endotherm onset at 125.6±2 °C (e.g., 125.6±1.9 °C, 125.6±1.8 °C, 125.6±1.7 °C, 125.6±1.6 °C, 125.6±1.5 °C, 125.6±1.4 °C, 125.6±1.3 °C, 125.6±1.2 °C, 125.6±1.1 °C, 125.6±1.0 °C, 125.6±0.9 °C, 125.6±0.8 °C, 125.6±0.7 °C, 125.6±0.6 °C, 125.6±0.5 °C, 125.6±0.4 °C, 125.6±0.3 °C, 125.6±0.2 °C, or 125.6±0.1 °C) as determined by DSC. In some embodiments, Form I is characterized as having an endotherm peak at about 130.2 °C as determined by DSC. In some embodiments, Form I is characterized as having an endotherm peak at 130.2±2 °C (e.g., 130.2±1.9 °C, 130.2±1.8 °C, 130.2±1.7 °C, 130.2±1.6 °C, 130.2±1.5 °C, 130.2±1.4 °C, 130.2±1.3 °C, 130.2±1.2 °C, 130.2±1.1 °C, 130.2±1.0 °C, 130.2±0.9 °C, 130.2±0.8 °C, 130.2±0.7 °C, 130.2±0.6 °C, 130.2±0.5 °C, 130.2±0.4 °C, 130.2±0.3 °C, 130.2±0.2 °C, or 130.2±0.1 °C) as determined by DSC.

In some embodiments, Form I has a thermographic analysis (TGA) graph substantially as shown in FIG. 1B. In some embodiments, Form I exhibits a weight loss of about 0.35% or 0.35%±0.05% (e.g., 0.35%±0.04%, 0.35%±0.03%, 0.35%±0.02%, or 0.35%±0.01%) between 105 °C and 145 °C as determined by TGA.

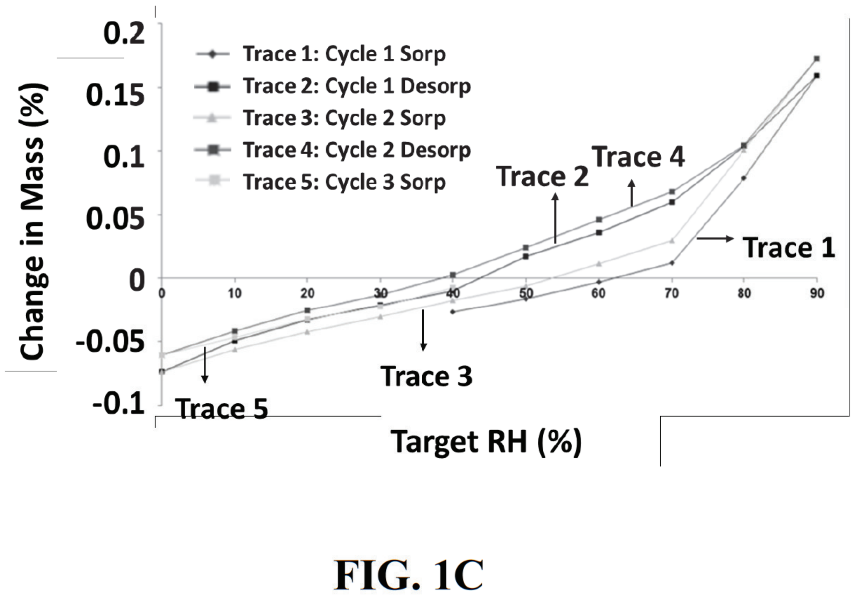

In some embodiments, Form I has a Gravimetric Vapor Sorption (GVS) graph substantially as shown in FIG. 1C.

In some embodiments, when stored for a period of 7 days under two different temperature/RH conditions (40°C/75% RH and 25°C/97% RH), Form I shows substantially no changes or no changes as determined by XRPD. In some embodiments, when stored for a period of 7 days under 40°C/75% RH, Form I shows substantially no changes or no changes as determined by XRPD. In some embodiments, when stored for a period of 7 days under 25°C/97% RH, Form I shows substantially no changes or no changes as determined by XRPD.

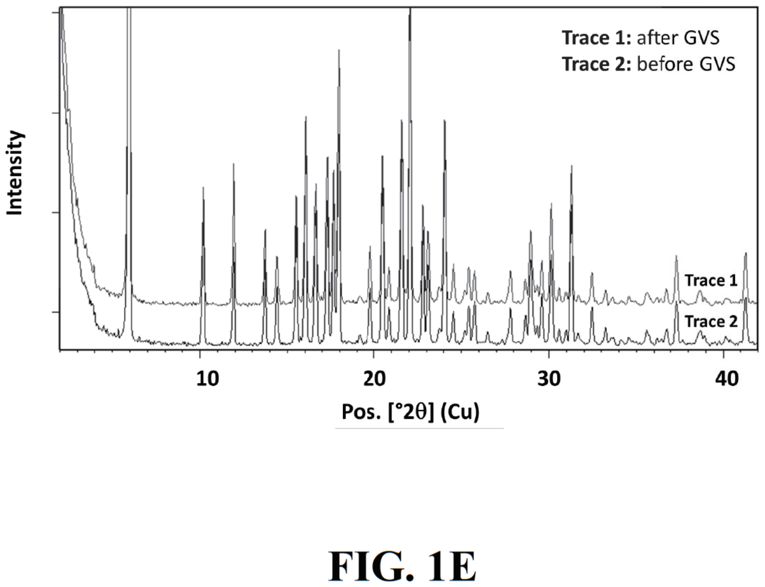

In some embodiments, Form I shows substantially no changes or no changes before and after the GVS measurement as determined by XRPD, as shown in FIG. 1E.

In some embodiments, Form I is 5-(3,4-difluorobenzyl)-8-((1r,4r)-4-methylcyclohexyl)-6,9-dioxo-2,5,8-triazaspiro[3.5]nonane-2-carbaldehyde with at least 90%, at least 91%, at least 92%, at least 93%, at least 94%, at least 95%, at least 96%, at least 97%, at least 98%, or at least 99% purity with respect to other forms, including salt, solvate, or amorphous forms, of the compound, and the purity remains substantially unchanged or unchanged when stored for a period of 7 days under 40°C/75% RH and/or under 25°C/97% RH, as determined by HPLC.

In some embodiments of Form I, at least one, at least two, at least three, at least four, at least five, at least six, at least seven, or all of the following (a)-(h) apply:
(a) Form I has an XRPD pattern comprising peaks at angles 2-theta of 6.0±0.2, 10.2±0.2, 21.6±0.2, 22.1±0.2, degrees; an XRPD pattern comprising additional peaks at angles 2-theta of 17.9±0.2 and 24.1±0.2 degrees; an XRPD pattern further comprising additional peaks at angles 2-theta of 16.0±0.2, 16.6±0.2, 17.3±0.2, 17.6±0.2, and 20.5±0.2 degrees; or an XRPD pattern comprising peaks at angles 2-theta of 6.0±0.2, 10.2±0.2, 11.9±0.2, 13.7±0.2, 14.4±0.2, 15.5±0.2, 16.0±0.2, 16.6±0.2, 17.3±0.2, 17.6±0.2, 17.9±0.2, 19.8±0.2, 20.5±0.2, 20.9±0.2, 21.6±0.2, 22.1±0.2, 22.8±0.2, 23.1±0.2, 24.1±0.2, 24.5±0.2, 25.4±0.2, 25.7±0.2, 27.8±0.2, 28.6±0.2, 29.0±0.2, 29.6±0.2, and 30.1±0.2 degrees;
(b) Form I has an XRPD pattern substantially as shown in FIG. 1A;
(c) Form I has a DSC graph substantially as shown in FIG. 1B;
(d) Form I is characterized as having an endotherm onset at 125.6±2 °C as determined by DSC;
(e) Form I is characterized as having an endotherm peak at 130.2±2 °C as determined by DSC;
(f) Form I has a TGA graph substantially as shown in FIG. 1B;
(g) Form I has a weight loss of about 0.35% or 0.35%±0.05% between 105 °C and 145 °C as determined by TGA; and
(h) Form I has a GVS graph substantially as shown in FIG. 1C.

### Form II

In some embodiments, provided herein is the crystalline Form II of 5-(3,4-difluorobenzyl)-8-((1r,4r)-4-methylcyclohexyl)-6,9-dioxo-2,5,8-triazaspiro[3.5]nonane-2-carbaldehyde. Sample and single crystal data for Form II is provided in Table 2-D.

**Table 2-D**

| | | | | | |
|---|---|---|---|---|---|
| Crystallization solvents | 2-Methoxyethanol | | | | |
| Crystallization method | Evaporation | | | | |
| Molecular formula | C₂₁H₂₅F₂N₃O₃ | | | | |
| Molecular weight | 405.44 | | | | |
| Crystal system | Triclinic | | | | |
| Space group | *P*-1 | *a* | 14.6622(3) Å | α | 68.271(2)° |
| | | *b* | 14.6944(3) Å | β | 72.639(2)° |
| | | *c* | 16.3761(2) Å | γ | 69.935(2)° |
| V | 3018.67(11) Å³ | | | | |
| Z | 6 | | | | |
| Density (calculated) | 1.338 Mg/m³ | | | | |
| Absorption coefficient, µ | 0.861 mm⁻¹ | | | | |
| Source, λ | 1.54184 Å | | | | |
| *F(000)* | 1284 | | | | |
| *T* | 100(2) K | | | | |
| Crystal | Colorless fragment, 0.250 x 0.120 x 0.100 mm | | | | |
| Wavelength | 1.54184 Å | | | | |

The crystal structure of Form II was solved in the triclinic centrosymmetric space group P-1 and refined with a final R1 [I>2σ(I)] value of 3.91%.

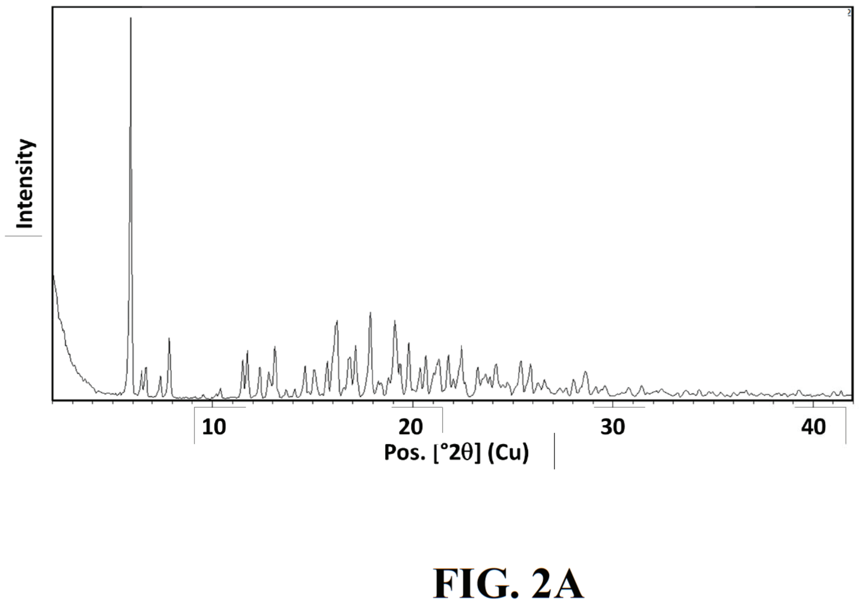

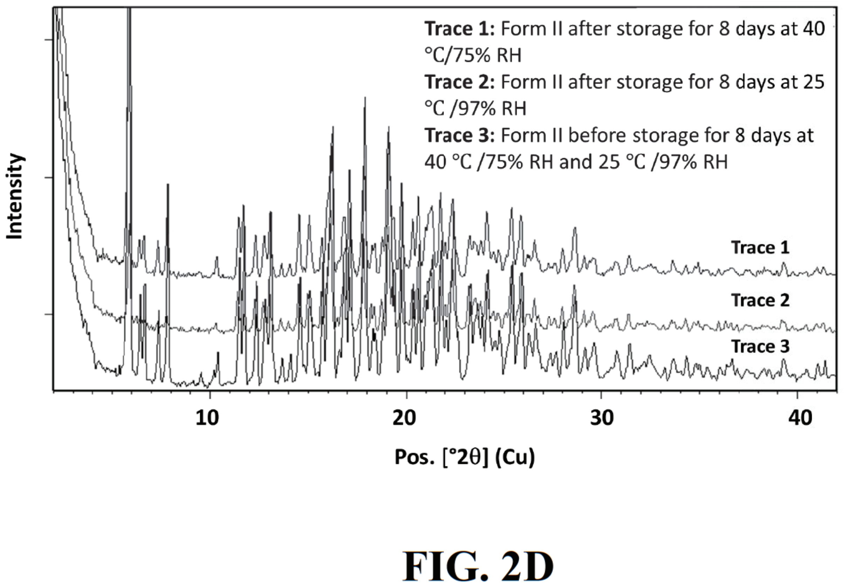

In some embodiments, Form II has an XRPD pattern substantially as shown in FIG. 2A.

Angles 2-theta and relative peak intensities observed for Form II using XRPD are shown in Table 2C.

**TABLE 2C**

| **Angle / 2θ** | **Intensity / %** |
|---|---|
| 5.9 | 100.0 |
| 6.4 | 4.7 |
| 6.7 | 8.2 |
| 7.4 | 4.7 |
| 7.8 | 15.9 |
| 10.4 | 2.6 |
| 11.5 | 8.4 |
| 11.7 | 12.9 |
| 12.4 | 8.4 |
| 12.8 | 6.9 |
| 13.1 | 14.1 |
| 13.7 | 2.3 |
| 14.1 | 2.4 |
| 14.6 | 7.4 |
| 15.1 | 7.8 |
| 15.7 | 8.0 |
| 16.2 | 20.0 |
| 16.6 | 2.3 |
| 16.8 | 10.7 |
| 17.2 | 13.9 |
| 17.9 | 24.5 |
| 18.4 | 4.1 |
| 18.8 | 4.7 |
| 19.1 | 20.9 |
| 19.4 | 8.6 |
| 19.8 | 14.8 |
| 20.3 | 7.9 |
| 20.7 | 11.4 |
| 21.0 | 5.8 |
| 21.3 | 10.6 |
| 21.8 | 11.7 |
| 22.0 | 4.5 |
| 22.4 | 13.9 |
| 23.3 | 8.2 |
| 23.6 | 6.4 |
| 23.9 | 5.5 |
| 24.2 | 9.2 |
| 24.5 | 3.1 |
| 24.8 | 3.8 |
| 25.2 | 3.3 |
| 25.4 | 9.9 |
| 25.9 | 9.1 |
| 26.3 | 4.2 |
| 26.6 | 4.9 |
| 27.3 | 2.4 |
| 27.7 | 2.6 |
| 28.1 | 5.1 |
| 28.4 | 2.8 |
| 28.7 | 7.1 |
| 29.1 | 2.9 |
| 29.6 | 3.2 |
| 30.8 | 2.7 |

In some embodiments, crystalline Form II has an XRPD pattern displaying at least two, at least three, at least four, at least five, at least six, at least seven, at least eight, at least nine, or at least ten of the peaks at angles 2-theta with the greatest intensity in the XRPD pattern substantially as shown in FIG. 2A or as provided in Table 2C. It should be understood that relative intensities can vary depending on a number of factors, including sample preparation, mounting, and the instrument and analytical procedure and settings used to obtain the spectrum. Relative peak intensities and peak assignments can vary within experimental error. In some embodiments, peak assignments listed herein, including for crystalline Form II, can vary by about ±0.6 degrees, ±0.4 degrees, ±0.2 degrees, or ±0.1 degrees 2-theta.

In some embodiments, crystalline Form II has an XRPD pattern comprising peaks at angles 2-theta of 5.9±0.2, 11.5±0.2, 11.7±0.2, 17.9±0.2, and 19.1±0.2 degrees. In some embodiments, crystalline Form II has an XRPD pattern comprising additional peaks at angles 2-theta of 7.8±0.2 and 16.2±0.2 degrees. In some embodiments, crystalline Form II has an XRPD pattern further comprising additional peaks at angles 2-theta of 13.1±0.2 and 19.8±0.2 degrees. In some embodiments, crystalline Form II has an XRPD pattern comprising peaks at angles 2-theta of 5.9±0.2, 6.4±0.2, 6.7±0.2, 7.4±0.2, 7.8±0.2, 10.4±0.2, 11.5±0.2, 11.7±0.2, 12.4±0.2, 12.8±0.2, 13.1±0.2, 13.7±0.2, 14.1±0.2, 14.6±0.2, 15.1±0.2, 15.7±0.2, 16.2±0.2, 16.6±0.2, 16.8±0.2, 17.2±0.2, 17.9±0.2, 18.4±0.2, 18.8±0.2, 19.1±0.2, 19.4±0.2, 19.8±0.2, 20.3±0.2, 20.7±0.2, 21.0±0.2, 21.3±0.2, 21.8±0.2, 22.0±0.2, 22.4±0.2, 23.3±0.2, 23.6±0.2, 23.9±0.2, 24.2±0.2, 24.5±0.2, 24.8±0.2, 25.2±0.2, 25.4±0.2, 25.9±0.2, 26.3±0.2, 26.6±0.2, 27.3±0.2, 27.7±0.2, 28.1±0.2, 28.4±0.2, 28.7±0.2, 29.1±0.2, 29.6±0.2, and 30.8±0.2 degrees. In some embodiments, an XRPD pattern may also be calculated from the single crystal data acquired for Form II. It is to be understood that additional peaks in the XRPD pattern other than those shown in FIG. 2A or as provided in Table 2C may be observed, for instance, due to the presence of impurities, solvent, or other polymorphs or amorphic forms present in the test sample.

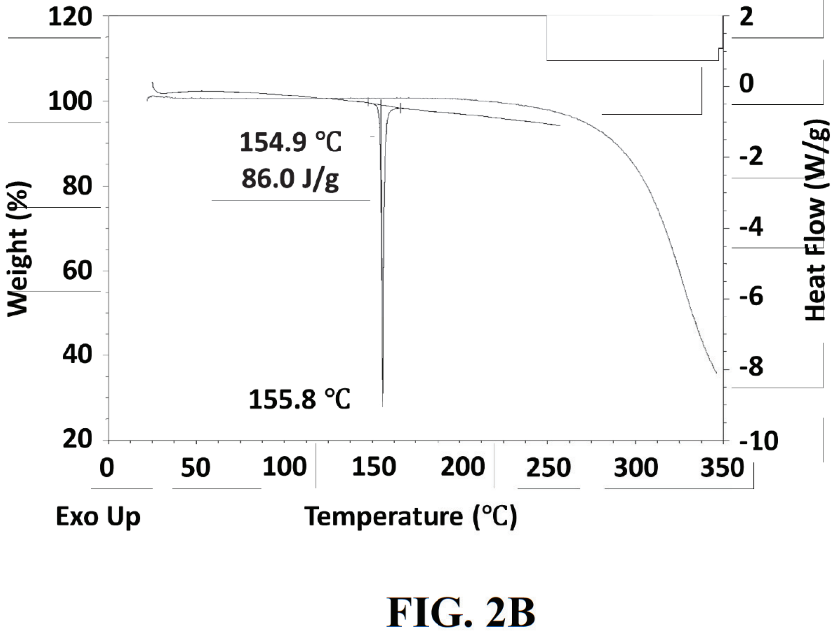

In some embodiments, Form II has a differential scanning calorimetry (DSC) graph substantially as shown in FIG. 2B. In some embodiments, Form II is characterized as having an endotherm onset at about 154.9 °C as determined by DSC. In some embodiments, Form II is characterized as having an endotherm onset at 154.9±2 °C (e.g., 154.9±1.9 °C, 154.9±1.8 °C, 154.9±1.7 °C, 154.9±1.6 °C, 154.9±1.5 °C, 154.9±1.4 °C, 154.9±1.3 °C, 154.9±1.2 °C, 154.9±1.1 °C, 154.9±1.0 °C, 154.9±0.9 °C, 154.9±0.8 °C, 154.9±0.7 °C, 154.9±0.6 °C, 154.9±0.5 °C, 154.9±0.4 °C, 154.9±0.3 °C, 154.9±0.2 °C, or 154.9±0.1 °C) as determined by DSC. In some embodiments, Form II is characterized as having an endotherm peak at about 155.8 °C as determined by DSC. In some embodiments, Form II is characterized as having an endotherm peak at 155.8±2 °C (e.g., 155.8±1.9 °C, 155.8±1.8 °C, 155.8±1.7 °C, 155.8±1.6 °C, 155.8±1.5 °C, 155.8±1.4 °C, 155.8±1.3 °C, 155.8±1.2 °C, 155.8±1.1 °C, 155.8±1.0 °C, 155.8±0.9 °C, 155.8±0.8 °C, 155.8±0.7 °C, 155.8±0.6 °C, 155.8±0.5 °C, 155.8±0.4 °C, 155.8±0.3 °C, 155.8±0.2 °C, or 155.8±0.1 °C) as determined by DSC.

In some embodiments, Form II has a thermographic analysis (TGA) graph substantially as shown in FIG. 2B. In some embodiments, Form II exhibits less than 0.1% (e.g., less than 0.09%, less than 0.08%, less than 0.07%, less than 0.06%, less than 0.05%, less than 0.04%, less than 0.03%, less than 0.02%, less than 0.01%) weight loss prior to degradation as determined by TGA.

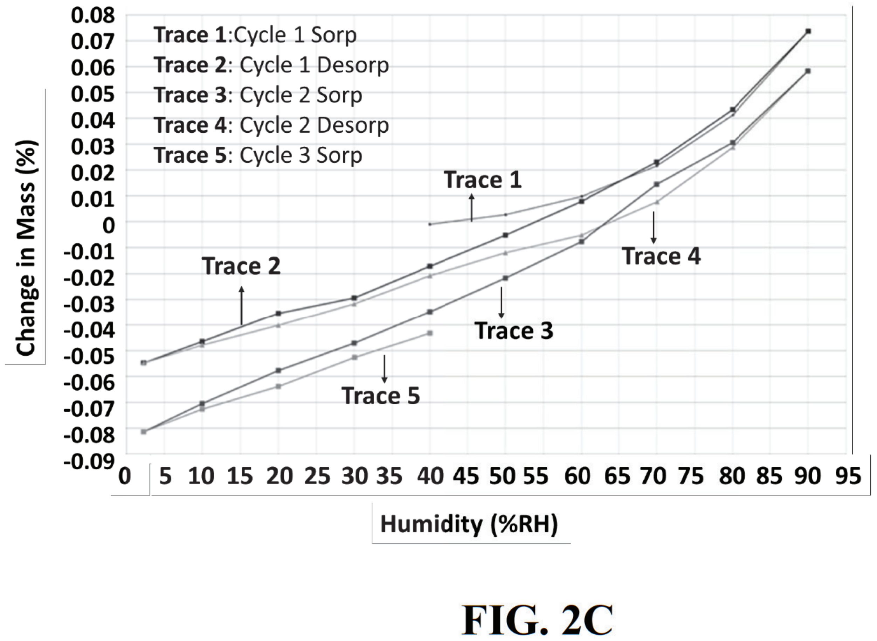

In some embodiments, Form II has a Gravimetric Vapor Sorption (GVS) graph substantially as shown in FIG. 2C.

In some embodiments, when stored for a period of 8 days under two different temperature/RH conditions (40°C/75% RH and 25°C/97% RH), Form II shows substantially no changes or no changes as determined by XRPD. In some embodiments, when stored for a period of 7 days under 40°C/75% RH, Form II shows substantially no changes or no changes as determined by XRPD. In some embodiments, when stored for a period of 7 days under 25°C/97% RH, Form II shows substantially no changes or no changes as determined by XRPD.

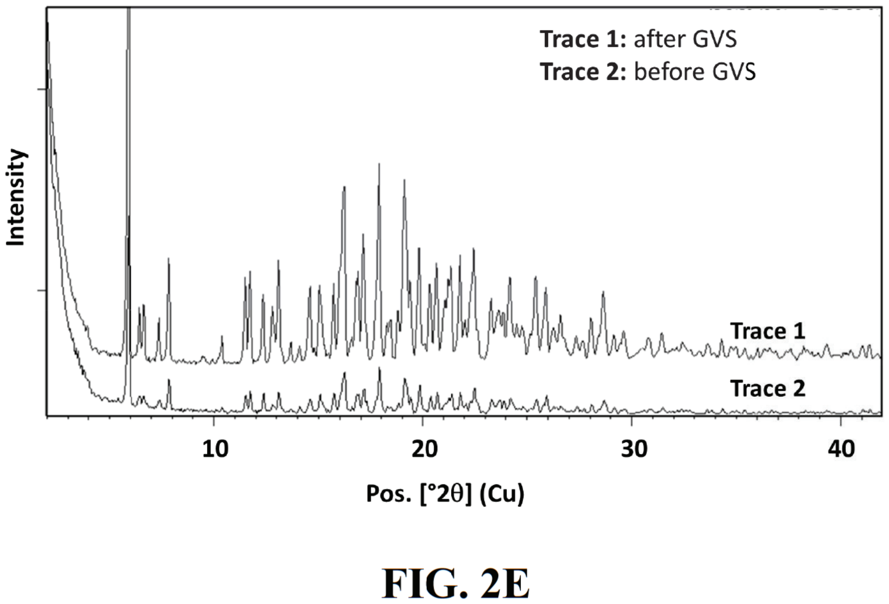

In some embodiments, Form II shows substantially no changes or no changes before and after the GVS measurement as determined by XRPD, as shown in FIG. 2E.

In some embodiments, Form II is 5-(3,4-difluorobenzyl)-8-((1r,4r)-4-methylcyclohexyl)-6,9-dioxo-2,5,8-triazaspiro[3.5]nonane-2-carbaldehyde with at least 90%, at least 91%, at least 92%, at least 93%, at least 94%, at least 95%, at least 96%, at least 97%, at least 98%, or at least 99% purity with respect to other forms, and the purity remains substantially unchanged or unchanged when stored for a period of 8 days under two different temperature/RH conditions (40°C/75% RH and/or 25°C/97% RH), as determined by HPLC.

Form II exhibits high solubility and high stability, which are desirable for pharmaceutical use. These properties can provide advantages such as improved consistency of dosing, improved release of the drug upon administration, longer shelf life, and greater ease of storage and packaging. Form II has a high solubility in a variety of common solvents. In some embodiments, Form II has a solubility of at least 100 mg/mL (e.g., at least 90 mg/mL, at least 80 mg/mL, at least 70 mg/mL, at least 60 mg/mL, or at least 50 mg/mL) in methanol, acetone, DMSO, acetonitrile and THF. In some embodiments, Form II has a solubility of about 24 mg/mL (e.g., 24±5 mg/mL, 24±4 mg/mL, 24±3 mg/mL, 24±2 mg/mL, or 24±1 mg/mL) in ethanol.

Form II exhibits a high stability in a variety of solvents. In some embodiments, Form II shows no visual signs of degradation after storage in a solvent for 24 hours, as determined by HPLC, wherein the solvent is selected from the group consisting of water, methanol, isopropanol, 2-BuOH, acetone, MIBK, DMSO, MeCN, THF, 2-methyl-THF, and toluene. In some embodiments, Form II shows no visual signs of degradation after storage in a solvent for 24 hours, as determined by HPLC, wherein the solvent is selected from the group consisting of methanol, acetone, DMSO, acetonitrile and THF. In some embodiments, Form II initially shows visual signs of degradation in a solvent but shows no further degradation after storage in the solvent for 24 hours, as determined by HPLC, wherein the solvent is selected from the group consisting of ethanol, *tert*-butyl methyl ether, and isopropyl acetate.

In some embodiments of Form II, at least one, at least two, at least three, at least four, at least five, at least six, at least seven, or all of the following (a)-(h) apply:
(a) Form II has an XRPD pattern comprising peaks at angles 2-theta of 5.9±0.2, 11.5±0.2, 11.7±0.2, 17.9±0.2, and 19.1±0.2 degrees; an XRPD pattern comprising additional peaks at angles 2-theta of 7.8±0.2 and 16.2±0.2 degrees; an XRPD pattern further comprising additional peaks at angles 2-theta of 13.1±0.2 and 19.8±0.2 degrees; or an XRPD pattern comprising peaks at angles 2-theta of 5.9±0.2, 6.4±0.2, 6.7±0.2, 7.4±0.2, 7.8±0.2, 10.4±0.2, 11.5±0.2, 11.7±0.2, 12.4±0.2, 12.8±0.2, 13.1±0.2, 13.7±0.2, 14.1±0.2, 14.6±0.2, 15.1±0.2, 15.7±0.2, 16.2±0.2, 16.6±0.2, 16.8±0.2, 17.2±0.2, 17.9±0.2, 18.4±0.2, 18.8±0.2, 19.1±0.2, 19.4±0.2, 19.8±0.2, 20.3±0.2, 20.7±0.2, 21.0±0.2, 21.3±0.2, 21.8±0.2, 22.0±0.2, 22.4±0.2, 23.3±0.2, 23.6±0.2, 23.9±0.2, 24.2±0.2, 24.5±0.2, 24.8±0.2, 25.2±0.2, 25.4±0.2, 25.9±0.2, 26.3±0.2, 26.6±0.2, 27.3±0.2, 27.7±0.2, 28.1±0.2, 28.4±0.2, 28.7±0.2, 29.1±0.2, 29.6±0.2, and 30.8±0.2 degrees;
(b) Form II has an XRPD pattern substantially as shown in FIG. 2A;
(c) Form II has a DSC graph substantially as shown in FIG. 2B;
(d) Form II is characterized as having an endotherm onset at 154.9±2 °C as determined by DSC;
(e) Form II is characterized as having an endotherm peak at 155.8±2 °C as determined by DSC;
(f) Form II has a TGA graph substantially as shown in FIG. 2B;
(g) Form II has less than 0.1% weight loss prior to degradation as determined by TGA; and
(h) Form II has a GVS graph substantially as shown in FIG. 2C

### Form III

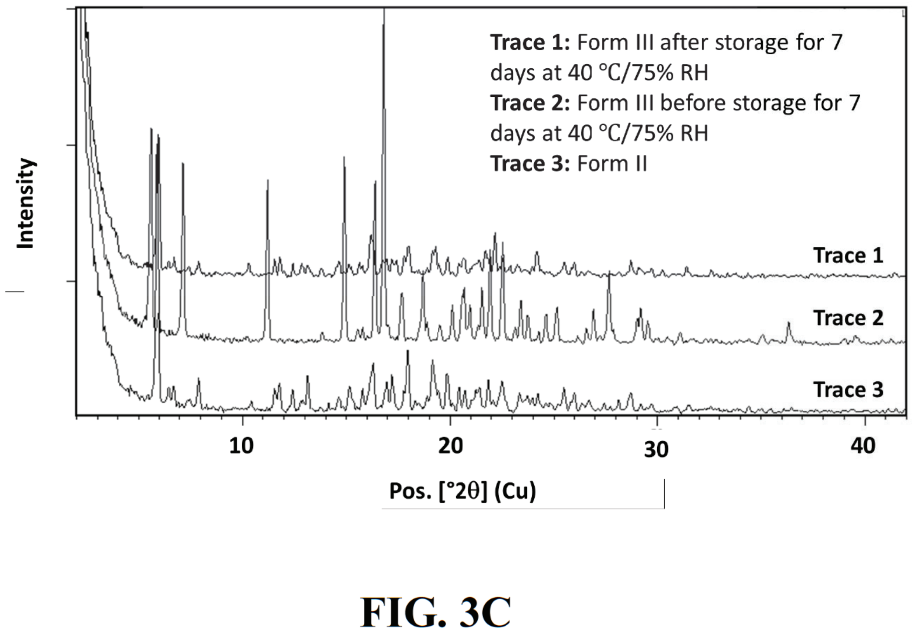

Crystalline Form III of 5-(3,4-difluorobenzyl)-8-((1r,4r)-4-methylcyclohexyl)-6,9-dioxo-2,5,8-triazaspiro[3.5]nonane-2-carbaldehyde 1,4-dioxane solvate was isolated and subjected to baseline characterization to understand the nature of the solid. In some embodiments, the crystalline Form III was assigned as an unstable, hemi-dioxane solvate that converted to crystalline Form II after storage at 40°C/75% RH conditions for 7 days, as determined by XRPD and shown in FIG. 3C. In some embodiments, crystalline Form III is 5-(3,4-difluorobenzyl)-8-((1r,4r)-4-methylcyclohexyl)-6,9-dioxo-2,5,8-triazaspiro[3.5]nonane-2-carbaldehyde with 0.5 mol equiv. of 1,4-dioxane, as determined by ¹HNMR.

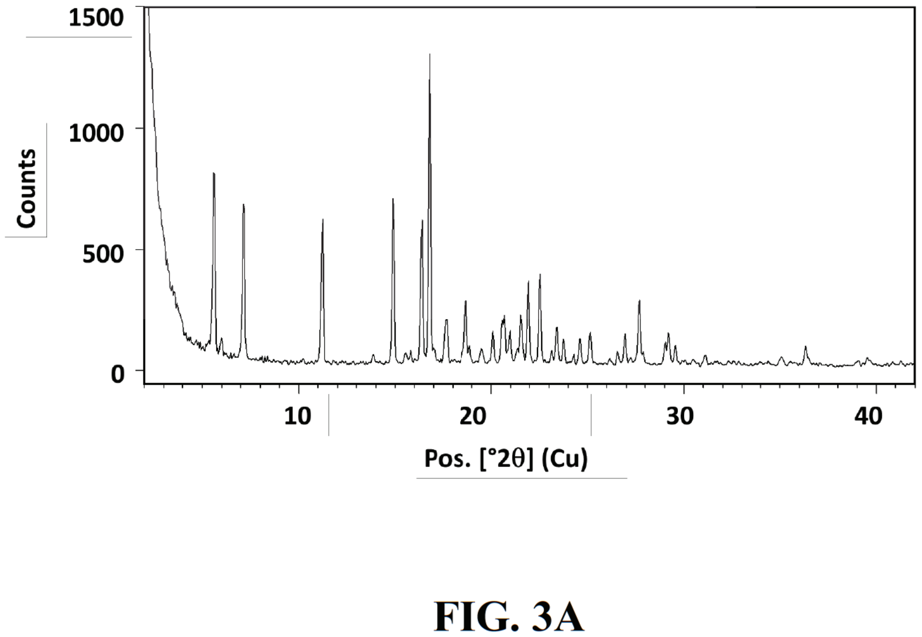

In some embodiments, Form III has an XRPD pattern substantially as shown in FIG. 3A.

Angles 2-theta and relative peak intensities observed for Form III using XRPD are shown in Table 4-A.

**Table 4-A**

| **Angle / 2θ** | **Intensity / %** |
|---|---|
| 5.6 | 60.3 |
| 6.0 | 3.3 |
| 7.2 | 51.4 |
| 11.2 | 46.8 |
| 13.9 | 2.7 |
| 14.9 | 54.8 |
| 15.5 | 3.4 |
| 15.8 | 3.3 |
| 16.4 | 45.8 |
| 16.8 | 100 |
| 17.0 | 3.6 |
| 17.7 | 14.6 |
| 18.7 | 20.4 |
| 18.9 | 4.4 |
| 19.5 | 4.9 |
| 20.1 | 10.7 |
| 20.6 | 12.7 |
| 21.0 | 11.2 |
| 21.4 | 3.8 |
| 21.6 | 14.4 |
| 21.9 | 27.3 |
| 22.5 | 30 |
| 23.2 | 3.4 |
| 23.4 | 12.5 |
| 23.8 | 8.2 |
| 24.3 | 2.2 |
| 24.6 | 8.7 |
| 25.1 | 10.4 |
| 26.2 | 1.5 |
| 26.6 | 4.1 |
| 26.9 | 9.9 |
| 27.3 | 1.8 |
| 27.7 | 21.9 |
| 27.9 | 3.5 |
| 29.0 | 6.6 |
| 29.2 | 8.9 |
| 29.6 | 6.1 |

In some embodiments, crystalline Form III has an XRPD pattern displaying at least two, at least three, at least four, at least five, at least six, at least seven, at least eight, at least nine, or at least ten of the peaks at angles 2-theta with the greatest intensity in the XRPD pattern substantially as shown in FIG. 3A. It should be understood that relative intensities can vary depending on a number of factors, including sample preparation, mounting, and the instrument and analytical procedure and settings used to obtain the spectrum. Relative peak intensities and peak assignments can vary within experimental error. In some embodiments, peak assignments including for crystalline Form III, can vary by about ±0.6 degrees, ±0.4 degrees, ±0.2 degrees, or ±0.1 degrees 2-theta.

In some embodiments, crystalline Form III has an XRPD pattern comprising peaks at angles 2-theta of 5.6±0.2, 7.2±0.2, 11.2±0.2, 14.9±0.2, and 16.8±0.2 degrees. In some embodiments, crystalline Form III has an XRPD pattern comprising additional peaks at angles 2-theta of 16.4±0.2,18.7±0.2, 21.9±0.2, 22.5±0.2, and 22.7±0.2 degrees. In some embodiments, crystalline Form III has an XRPD pattern comprising peaks at angles 2-theta of 5.6±0.2, 6.0±0.2, 7.2±0.2, 11.2±0.2, 13.9±0.2, 14.9±0.2, 15.5±0.2, 15.8±0.2, 16.4±0.2, 16.8±0.2, 17.0±0.2, 17.7±0.2, 18.7±0.2, 18.9±0.2, 19.5±0.2, 20.1±0.2, 20.6±0.2, 21.0±0.2, 21.4±0.2, 21.6±0.2, 21.9±0.2, 22.5±0.2, 23.2±0.2, 23.4±0.2, 23.8±0.2, 24.3±0.2, 24.6±0.2, 25.1±0.2, 26.2±0.2, 26.6±0.2, 26.9±0.2, 27.3±0.2, 27.7±0.2, 27.9±0.2, 29.0±0.2, 29.2±0.2, and 29.6±0.2 degrees. It is to be understood that additional peaks in the XRPD pattern other than those shown in FIG. 3A or as provided in Table 4-A may be observed, for instance, due to the presence of impurities, solvent, or other polymorphs or amorphic forms present in the test sample.

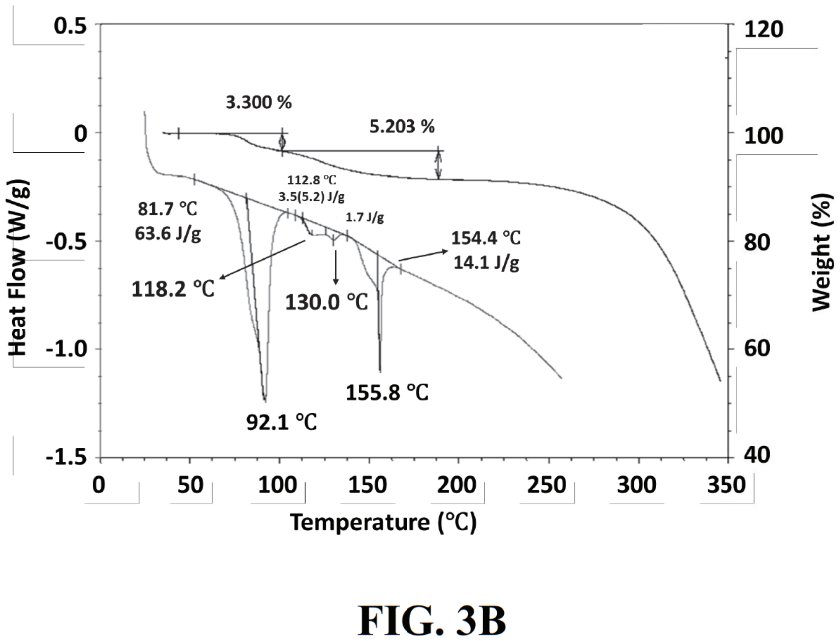

In some embodiments, Form III has a differential scanning calorimetry (DSC) graph substantially as shown in FIG. 3B. In some embodiments, Form III is characterized as having an endotherm onset at about 81.7±4 °C, an endotherm onset with two events at about 112.8±4 °C, or an endotherm onset at about 154.4±4 °C or any combinations thereof, as determined by DSC. In some embodiments, Form III is characterized as having an endotherm peak at about 92.1±4 °C, an endotherm peak at about 118.2±4 °C, an endotherm peak at about 130.0±4 °C, or an endotherm peak at about 155.8±4 °C, or any combinations thereof, as determined by DSC.

In some embodiments, Form III has a thermographic analysis (TGA) graph substantially as shown in FIG. 3B. In some embodiments, Form III exhibits a weight loss of about 3.3 % or 3.3%±0.5% (e.g., 3.3%±0.4%, 3.3%±0.3%, 3.3%±0.2%, 3.3%±0.1%) between 45 °C and 101 °C and/or a weight loss of about 5.2% or 5.2%±0.5% (e.g., 5.2%±0.4%, 5.2%±0.3%, 5.2%±0.2%, 5.2%±0.1%) between 101 °C and 189 °C, as determined by TGA.

In some embodiments of Form III, at least one, at least two, at least three, at least four, at least five, at least six, or all of the following (a)-(g) apply:
(a) Form III has an XRPD pattern comprising peaks at angles 2-theta of 5.6±0.2, 7.2±0.2, 11.2±0.2, 14.9±0.2, and 16.8±0.2 degrees; an XRPD pattern comprising peaks at angles 2-theta of 5.6±0.2, 7.2±0.2, 11.2±0.2, 14.9±0.2, 16.4±0.2, 16.8±0.2, 18.7±0.2, 21.9±0.2, 22.5±0.2, and 22.7±0.2 degrees; or an XRPD pattern comprising peaks at angles 2-theta of 5.6±0.2, 6.0±0.2, 7.2±0.2, 11.2±0.2, 13.9±0.2, 14.9±0.2, 15.5±0.2, 15.8±0.2, 16.4±0.2, 16.8±0.2, 17.0±0.2, 17.7±0.2, 18.7±0.2, 18.9±0.2, 19.5±0.2, 20.1±0.2, 20.6±0.2, 21.0±0.2, 21.4±0.2, 21.6±0.2, 21.9±0.2, 22.5±0.2, 23.2±0.2, 23.4±0.2, 23.8±0.2, 24.3±0.2, 24.6±0.2, 25.1±0.2, 26.2±0.2, 26.6±0.2, 26.9±0.2, 27.3±0.2, 27.7±0.2, 27.9±0.2, 29.0±0.2, 29.2±0.2, and 29.6±0.2 degrees;
(b) Form III has an XRPD pattern substantially as shown in FIG. 3A;
(c) Form III has a DSC graph substantially as shown in FIG. 3B;
(d) Form III is characterized as having an endotherm onset at about 81.7±4.0 °C, an endotherm onset at about 112.8±4.0 °C, or an endotherm onset at about 154.4±4.0 °C, or any combination thereof, as determined by DSC;
(e) Form III is characterized as having an endotherm peak at about 92.1±4.0 °C, an endotherm peak at about 118.2±4.0 °C, an endotherm peak at about 130.0±4.0 °C, or an endotherm onset at about 155.8±4.0 °C, or any combination thereof, as determined by DSC;
(f) Form III has a TGA graph substantially as shown in FIG. 3B; and
(g) Form III has a weight loss of about 3.3% or 3.3%±0.5% between 45 °C and 101 °C and/or a weight loss of about 5.2% or 5.2%±0.5% between 101 °C and 189 °C, as determined by TGA.

### Compositions

Also provided herein are compositions containing polymorphs described herein, such as Form I, Form II, Form III or a mixture thereof. In some embodiments, the composition contains Form I. In some embodiments, the composition contains Form II. In some embodiments, the composition contains Form III. In some embodiments, the composition contains a mixture of Form I and Form II. In some embodiments, the composition further comprises a pharmaceutically acceptable carrier.

In some embodiments, provided is a composition containing Form I of 5-(3,4-difluorobenzyl)-8-((1r,4r)-4-methylcyclohexyl)-6,9-dioxo-2,5,8-triazaspiro[3.5]nonane-2-carbaldehyde. In some embodiments, the composition is substantially free of at least one or both of crystalline Forms II and III of 5-(3,4-difluorobenzyl)-8-((1r,4r)-4-methylcyclohexyl)-6,9-dioxo-2,5,8-triazaspiro[3.5]nonane-2-carbaldehyde. In some embodiments, the composition is substantially free of amorphous or non-crystalline form of 5-(3,4-difluorobenzyl)-8-((1r,4r)-4-methylcyclohexyl)-6,9-dioxo-2,5,8-triazaspiro[3.5]nonane-2-carbaldehyde. In some embodiments, the composition is substantially free of salts of 5-(3,4-difluorobenzyl)-8-((1r,4r)-4-methylcyclohexyl)-6,9-dioxo-2,5,8-triazaspiro[3.5]nonane-2-carbaldehyde.

In some embodiments of the composition containing Form I of 5-(3,4-difluorobenzyl)-8-((1r,4r)-4-methylcyclohexyl)-6,9-dioxo-2,5,8-triazaspiro[3.5]nonane-2-carbaldehyde, at least about 0.1%, at least about 0.3%, at least about 0.5%, at least about 0.8%, at least about 1.0%, at least about 5.0%, at least about 10%, at least about 20%, at least about 30%, at least about 40%, at least about 50%, at least about 60%, at least about 70%, at least about 80%, at least about 85%, at least about 90%, at least about 95%, at least about 96%, at least about 97%, at least about 98%, at least about 99%, or at least 99.9% by weight of the total composition is Form I. In some embodiments of the composition containing Form I of 5-(3,4-difluorobenzyl)-8-((1r,4r)-4-methylcyclohexyl)-6,9-dioxo-2,5,8-triazaspiro[3.5]nonane-2-carbaldehyde, at least about 0.1%, at least about 0.3%, at least about 0.5%, at least about 0.8%, at least about 1.0%, at least about 5.0%, at least about 10%, at least about 20%, at least about 30%, at least about 40%, at least about 50%, at least about 60%, at least about 70%, at least about 80%, at least about 85%, at least about 90%, at least about 95%, at least about 96%, at least about 97%, at least about 98%, at least about 99%, or at least 99.9% by weight of 5-(3,4-difluorobenzyl)-8-((1r,4r)-4-methylcyclohexyl)-6,9-dioxo-2,5,8-triazaspiro[3.5]nonane-2-carbaldehyde exists in Form I.

In some embodiments, provided is a composition containing Form II of 5-(3,4-difluorobenzyl)-8-((1r,4r)-4-methylcyclohexyl)-6,9-dioxo-2,5,8-triazaspiro[3.5]nonane-2-carbaldehyde. In some embodiments, the composition is substantially free of at least one or both of crystalline Forms I and III of 5-(3,4-difluorobenzyl)-8-((1r,4r)-4-methylcyclohexyl)-6,9-dioxo-2,5,8-triazaspiro[3.5]nonane-2-carbaldehyde. In some embodiments, the composition is substantially free of amorphous or non-crystalline form of 5-(3,4-difluorobenzyl)-8-((1r,4r)-4-methylcyclohexyl)-6,9-dioxo-2,5,8-triazaspiro[3.5]nonane-2-carbaldehyde. In some embodiments, the composition is substantially free of salts of 5-(3,4-difluorobenzyl)-8-((1r,4r)-4-methylcyclohexyl)-6,9-dioxo-2,5,8-triazaspiro[3.5]nonane-2-carbaldehyde.

In some embodiments of the composition containing Form II of 5-(3,4-difluorobenzyl)-8-((1r,4r)-4-methylcyclohexyl)-6,9-dioxo-2,5,8-triazaspiro[3.5]nonane-2-carbaldehyde, at least about 0.1%, at least about 0.3%, at least about 0.5%, at least about 0.8%, at least about 1.0%, at least about 5.0%, at least about 10%, at least about 20%, at least about 30%, at least about 40%, at least about 50%, at least about 60%, at least about 70%, at least about 80%, at least about 85%, at least about 90%, at least about 95%, at least about 96%, at least about 97%, at least about 98%, at least about 99%, or at least 99.9% by weight of the total composition is Form II. In some embodiments of the composition containing Form II of 5-(3,4-difluorobenzyl)-8-((1r,4r)-4-methylcyclohexyl)-6,9-dioxo-2,5,8-triazaspiro[3.5]nonane-2-carbaldehyde, at least about 0.1%, at least about 0.3%, at least about 0.5%, at least about 0.8%, at least about 1.0%, at least about 5.0%, at least about 10%, at least about 20%, at least about 30%, at least about 40%, at least about 50%, at least about 60%, at least about 70%, at least about 80%, at least about 85%, at least about 90%, at least about 95%, at least about 96%, at least about 97%, at least about 98%, at least about 99%, or at least 99.9% by weight of 5-(3,4-difluorobenzyl)-8-((1r,4r)-4-methylcyclohexyl)-6,9-dioxo-2,5,8-triazaspiro[3.5]nonane-2-carbaldehyde exists in Form II.

In some embodiments, provided is a composition containing Form III of 5-(3,4-difluorobenzyl)-8-((1r,4r)-4-methylcyclohexyl)-6,9-dioxo-2,5,8-triazaspiro[3.5]nonane-2-carbaldehyde. In some embodiments, the composition is substantially free of at least one or both of crystalline Forms I and II of 5-(3,4-difluorobenzyl)-8-((1r,4r)-4-methylcyclohexyl)-6,9-dioxo-2,5,8-triazaspiro[3.5]nonane-2-carbaldehyde. In some embodiments, the composition is substantially free of amorphous or non-crystalline form of 5-(3,4-difluorobenzyl)-8-((1r,4r)-4-methylcyclohexyl)-6,9-dioxo-2,5,8-triazaspiro[3.5]nonane-2-carbaldehyde. In some embodiments, the composition is substantially free of salts of 5-(3,4-difluorobenzyl)-8-((1r,4r)-4-methylcyclohexyl)-6,9-dioxo-2,5,8-triazaspiro[3.5]nonane-2-carbaldehyde.

In some embodiments of the composition containing Form III of 5-(3,4-difluorobenzyl)-8-((1r,4r)-4-methylcyclohexyl)-6,9-dioxo-2,5,8-triazaspiro[3.5]nonane-2-carbaldehyde, at least about 0.1%, at least about 0.3%, at least about 0.5%, at least about 0.8%, at least about 1.0%, at least about 5.0%, at least about 10%, at least about 20%, at least about 30%, at least about 40%, at least about 50%, at least about 60%, at least about 70%, at least about 80%, at least about 85%, at least about 90%, at least about 95%, at least about 96%, at least about 97%, at least about 98%, at least about 99%, or at least 99.9% by weight of the total composition is Form III. In some embodiments of the composition containing Form III of 5-(3,4-difluorobenzyl)-8-((1r,4r)-4-methylcyclohexyl)-6,9-dioxo-2,5,8-triazaspiro[3.5]nonane-2-carbaldehyde, at least about 0.1%, at least about 0.3%, at least about 0.5%, at least about 0.8%, at least about 1.0%, at least about 5.0%, at least about 10%, at least about 20%, at least about 30%, at least about 40%, at least about 50%, at least about 60%, at least about 70%, at least about 80%, at least about 85%, at least about 90%, at least about 95%, at least about 96%, at least about 97%, at least about 98%, at least about 99%, or at least 99.9% by weight of 5-(3,4-difluorobenzyl)-8-((1r,4r)-4-methylcyclohexyl)-6,9-dioxo-2,5,8-triazaspiro[3.5]nonane-2-carbaldehyde exists in Form III.

In some embodiments, provided is a tablet or capsule containing one or more of the crystalline Forms described herein (*e.g.,* Form I, II, III or a mixture thereof), and one or more pharmaceutically acceptable carriers. In some embodiments, provided is a tablet or capsule containing substantially pure crystalline Form I of 5-(3,4-difluorobenzyl)-8-((1r,4r)-4-methylcyclohexyl)-6,9-dioxo-2,5,8-triazaspiro[3.5]nonane-2-carbaldehyde, and one or more pharmaceutically acceptable carriers. In some embodiments, provided is a tablet or capsule containing substantially pure crystalline Form II of 5-(3,4-difluorobenzyl)-8-((1r,4r)-4-methylcyclohexyl)-6,9-dioxo-2,5,8-triazaspiro[3.5]nonane-2-carbaldehyde, and one or more pharmaceutically acceptable carriers. In some embodiments, provided is a tablet or capsule containing substantially pure crystalline Form III of 5-(3,4-difluorobenzyl)-8-((1r,4r)-4-methylcyclohexyl)-6,9-dioxo-2,5,8-triazaspiro[3.5]nonane-2-carbaldehyde, and one or more pharmaceutically acceptable carriers. In some embodiments, provided is a tablet or capsule containing a mixture of Form I and Form II of 5-(3,4-difluorobenzyl)-8-((1r,4r)-4-methylcyclohexyl)-6,9-dioxo-2,5,8-triazaspiro[3.5]nonane-2-carbaldehyde, and one or more pharmaceutically acceptable carriers.

### Method of Preparation

### Form I

In some embodiments, provided is a method of preparing crystalline Form I of 5-(3,4-difluorobenzyl)-8-((1r,4r)-4-methylcyclohexyl)-6,9-dioxo-2,5,8-triazaspiro[3.5]nonane-2-carbaldehyde, the method comprising: (a) mixing 5-(3,4-difluorobenzyl)-8-((1r,4r)-4-methylcyclohexyl)-6,9-dioxo-2,5,8-triazaspiro[3.5]nonane-2-carbaldehyde with a solvent; and (b) stirring the mixture of step (a) to form a suspension. In some embodiments, step (b) comprises stirring the mixture of step (a) at a temperature from 0 °C to 30 °C, 0 °C to 20 °C, 0 °C to 10 °C, or about 5 °C. In some embodiments, the solvent is selected from the group consisting of ethanol, 1-propanol, 2-propanol, ethyl acetate, methyl isobutyl ketone (MIBK), ethyleneglycol, a mixture of N,N-dimethylformamide (DMF) and water, a mixture of N-methylpyrrolidone (NMP) and water, a mixture of 1,4-dioxane and water. In some embodiments, the method further comprises adding additional solvent. In some embodiments, the method further comprises adding anti-solvent to facilitate the formation of the suspension. In some embodiments, the method further comprises filtering the suspension of step (b). In some embodiments, the method further comprises filtering the suspension of step (b) after 5 to 9 days, 6 to 8 days, or about 7 days.

In some embodiments, provided is a method of preparing crystalline Form I of 5-(3,4-difluorobenzyl)-8-((1r,4r)-4-methylcyclohexyl)-6,9-dioxo-2,5,8-triazaspiro[3.5]nonane-2-carbaldehyde, the method comprising: (a) mixing 5-(3,4-difluorobenzyl)-8-((1r,4r)-4-methylcyclohexyl)-6,9-dioxo-2,5,8-triazaspiro[3.5]nonane-2-carbaldehyde with a solvent; and (b) subjecting the mixture generated in step (a) to heat/cool cycles. In some embodiments, the solvent is selected from the group consisting of methanol, acetone, ethyl acetate, isopropyl acetate, propyl acetate, methyl ethyl ketone (MEK), tetrahydrofuran (THF), dichloromethane, acetonitrile, dimethyl sulfoxide (DMSO), nitromethane, n-heptane, water, cyclohexane, ethyleneglycol, 2 methyltetrahydrofuran, a mixture of DMSO and water, a mixture of DMF and water, a mixture of NMP and water, a mixture of methanol and water, a mixture of 1-propanol and water, a mixture of 2-methoxyethanol and water, a mixture of ethyl acetate and cyclohexane, a mixture of toluene and n-heptane, a mixture of diethyl ether and n-heptane, and a mixture of MTBE and n-heptane. In some embodiments, the solvent is selected from the group consisting of methanol, acetone, ethyl acetate, isopropyl acetate, propyl acetate, methyl ethyl ketone (MEK), tetrahydrofuran (THF), dichloromethane, acetonitrile, dimethyl sulfoxide (DMSO), nitromethane, n-heptane, water, cyclohexane, ethyleneglycol, 2-methyltetrahydrofuran, DMSO:H₂O (1:1 v/v), DMF and water (1:1 v/v), NMP and water (1:1 v/v), methanol and water (1:1 v/v), 1-propanol: water (1:1 v/v), 2-methoxyethanol: water (1:1 v/v), ethyl acetate and cyclohexane (1:1 v/v), toluene: n-heptane (1:1 v/v), diethyl ether and n-heptane (1:1 v/v), MTBE and n-heptane (1:1 v/v). In some embodiments, the heat/cool cycles comprises cycles between room temperature and a temperature higher than room temperature. In some embodiments, the heat/cool cycles comprises cycles between room temperature and a temperature higher than room temperature, which is from 30 °C to 70 °C, 40°C to 60 °C, or about 50 °C. In some embodiments, the duration of each condition of the heat/cool cycle is from 1 to 6 hours, 2 to 5 hours, or about 4 hours. In some embodiments wherein biphasic layers are formed in step (a), the method further comprises sonicating the mixture of step (a) and/or subjecting the mixture of step (a) to evaporation. In some embodiments wherein biphasic layers are formed in step (a), the method further comprises sonicating the mixture of step (a) wherein the sonication duration is from 0.5 hours to 3 hours, or about 1.5 hours. In some embodiments wherein gums are formed in step (a), the method further comprising adding a solvent (e.g., a hydrocarbon solvent such as cyclohexane) to the mixture of step (a) and sonicating the mixture of step (a). In some embodiments wherein gums are formed in step (a), the method further comprising adding a solvent (e.g., a hydrocarbon solvent such as cyclohexane) to the mixture of step (a) and sonicating the mixture of step (a) wherein the sonication duration is from 2 hours to 6 hours, 3 hours to 5 hours, or about 4 hours. In some embodiments, the method further comprises filtering a solid generated in step (b). In some embodiments, the method further comprises filtering a solid generated in step (b) after 5 to 9 days, 6 to 8 days, or about 7 days.

In some embodiments, provided is a method of preparing crystalline Form I of 5-(3,4-difluorobenzyl)-8-((1r,4r)-4-methylcyclohexyl)-6,9-dioxo-2,5,8-triazaspiro[3.5]nonane-2-carbaldehyde, the method comprising: (a) wetting 5-(3,4-difluorobenzyl)-8-((1r,4r)-4-methylcyclohexyl)-6,9-dioxo-2,5,8-triazaspiro[3.5]nonane-2-carbaldehyde with a solvent; and (b) grinding the mixture of step (a). In some embodiments, step (b) comprises grinding the mixture of step (a) for a time period of from 1 hour to 3 hours, 1.5 hours to 2.5 hours, or about 2 hours. In some embodiments, the step (b) comprises grinding the mixture of step (a) at a speed of from 3000 rpm to 7000 rpm, 4000 rpm to 6000 rpm, or about 5000 rpm. In some embodiments wherein gums are formed in step (a), the method further comprises drying the gums *in-vacuo.* In some embodiments wherein gums are formed in step (a), the method further comprises drying the gums *in-vacuo* at a temperature which is 10 °C higher or lower than room temperature, 5 °C higher or lower than room temperature, 3 °C higher or lower than the room temperature, or about the room temperature. In some embodiments wherein gums are formed in step (a), the method further comprises drying the gums *in-vacuo* for 5 to 15 hours, 8 to 12 hours, or about 10 hours. In some embodiments wherein gums are formed in step (a), the method further comprises drying the gums *in-vacuo.* In some embodiments wherein gums are formed in step (a), the method further comprises drying the gums *in-vacuo* at a temperature which is 10 °C higher or lower than room temperature, 5 °C higher or lower than the room temperature, 3 °C higher or lower than the room temperature, or about the room temperature. In some embodiments wherein gums are formed in step (a), the method further comprises drying the gums *in-vacuo* for 1 day to 5 days, 2 days to 4 days, or about 3 days. In some embodiments wherein brittle materials are involved, the method further comprises drying the mixture containing brittle materials *in-vacuo.* In some embodiments wherein brittle materials are involved, the method further comprises drying the mixture containing brittle materials *in-vacuo* at a temperature which is 10 °C higher or lower than room temperature, 5 °C higher or lower than the room temperature, 5 °C higher or lower than the room temperature, 3 °C higher or lower than the room temperature, or about the room temperature. In some embodiments wherein brittle materials are involved, the method further comprises drying the mixture containing brittle materials *in-vacuo* for 5 to 15 hours, 8 to 12 hours, or about 10 hours. In some embodiments, the solvent is selected from the group consisting of a mixture of methanol and water, a mixture of ethanol and water, a mixture of 1-propanol and water, a mixture of 2-propanol and water, a mixture of 2-butanol and water, a mixture of 2-methoxyethan-1-ol and water, a mixture of acetone and water, a mixture of 1,4-dioxane and water, and a mixture of DMSO and water. In some embodiments, the solvent is selected from the group consisting of methanol and water (1:1 and 15:85 v/v), ethanol and water (1:1 and 15:85 v/v), 1-propanol and water (98:2 and 15:85 v/v), 2-propanol and water (98:2, 1:1, and 15:85 v/v), 2-butanol and water (15:85 v/v), 2-methoxyethan-1-ol and water (1:1 v/v), acetone and water (85:15 v/v), 1,4-dioxane and water (1:1 v/v), and DMSO and water (85:15, 1:1, and 15:85 v/v). Form I can also be prepared with the procedures provided in Example 1 and/or 5 herein.

In some embodiments, provided is a method of preparing crystalline Form I of 5-(3,4-difluorobenzyl)-8-((1r,4r)-4-methylcyclohexyl)-6,9-dioxo-2,5,8-triazaspiro[3.5]nonane-2-carbaldehyde, the method comprising: (1) forming a mixture of 5-(3,4-difluorobenzyl)-8-((1r,4r)-4-methylcyclohexyl)-6,9-dioxo-2,5,8-triazaspiro[3.5]nonane-2-carbaldehyde and a first solvent; (2) heating the mixture of step (1) to a first temperature; (3) cooling down the mixture of step (2) to a second temperature; and (4) filtering the mixture of step (3) at the second temperature to obtain crystalline solids. In some embodiments, the first solvent comprises a non-polar organic solvent and EtOAc. In some embodiments, the first solvent comprises cyclohexane and EtOAc. In some embodiments, the first solvent comprises petroleum ether and EtOAc. In some embodiments, the first solvent is a mixture of cyclohexane and EtOAc. In some embodiments, the first solvent is a mixture of a non-polar organic solvent and EtOAc. In some embodiments, the first solvent is a mixture of petroleum ether and EtOAc. In some embodiment, the first solvent is a mixture of cyclohexane and EtOAc wherein the volumetric ratio between cyclohexane and EtOAc is from 3:1 to 7:1, from 4:1 to 6:1, or about 5:1. In some embodiments, the first temperature is from 50 °C to 110 °C, 60°C to 100 °C, 70°C to 90 °C, or about 80 °C. In some embodiments, the second temperature is 10 °C higher or lower than room temperature, 5 °C higher or lower than the room temperature, 5 °C higher or lower than the room temperature, 3 °C higher or lower than the room temperature, or about the room temperature. In some embodiments, the method further comprises (5) concentrating the filtrate of step (4) under reduced pressure and (6) recrystallizing the mixture of step (5) with a second solvent at a third temperature to obtain crystalline solid. In some embodiments, the second solvent comprise petroleum ether and EtOAc. In some embodiments, the second solvent is a mixture of petroleum ether and EtOAc. In some embodiments, the second solvent is a mixture of petroleum ether and EtOAc wherein the volumetric ratio between petroleum ether and EtOAc is from 5:1 to 15:1, from 7:1 to 13:1, from 8:1 to 12:1, from 9:1 to 11:1, or about 10:1. In some embodiments, the third temperature is 10 °C higher or lower than room temperature, 5 °C higher or lower than the room temperature, 5 °C higher or lower than the room temperature, 3 °C higher or lower than the room temperature, or about the room temperature.

Form I can also be prepared with the procedures provided in Example 1 and/or 5 herein.

### Form II

In some embodiments, provided is a method of preparing crystalline Form II of 5-(3,4-difluorobenzyl)-8-((1r,4r)-4-methylcyclohexyl)-6,9-dioxo-2,5,8-triazaspiro[3.5]nonane-2-carbaldehyde, the method comprising: (1) mixing 5-(3,4-difluorobenzyl)-8-((1r,4r)-4-methylcyclohexyl)-6,9-dioxo-2,5,8-triazaspiro[3.5]nonane-2-carbaldehyde with a solvent; and (2) stirring the mixture generated in step (1) to form a suspension. In some embodiments, step (2) comprises stirring the mixture of step (1) at a temperature from 0 °C to 30 °C, 0 °C to 20 °C, 0 °C to 10 °C, or about 5 °C. In some embodiments, the solvent is selected from the group consisting of n-heptane, diethyl ether, propyl acetate, ethyl acetate, isopropyl acetate, MIBK, 2-propanol, ethanol, MTBE, 2-methyl-1-propanol, toluene, 1,2-dimethoxyethane, tetrahydrofuran, 2-methoxyethanol, methanol, a mixture of isopropanol and water, a mixture of 2-propanol and water, a mixture of chloroform and n-heptane, a mixture of dichloromethane and n-heptane, a mixture of cyclohexane and heptane, and a mixture of THF and water. In some embodiments, the solvent is selected from the group consisting of of n-heptane, diethyl ether, propyl acetate, ethyl acetate, isopropyl acetate, methyl isobutyl ketone (MIBK), 2-propanol, ethanol, MTBE, 2-methyl-1-propanol, toluene, 1,2-dimethoxyethane, tetrahydrofuran, 2-methoxyethanol, methanol, iPA:water (95:5, v/v), 2-propanol and water (95:5 v/v), chloroform and n-heptane (1:1 v/v), dichloromethane and n-heptane (1:1 v/v), cyclohexane and n-heptane (1:1 v/v), and THF and water (1:1 v/v),. In some embodiments, the method further comprises adding additional solvent. In some embodiments, the method further comprises adding anti-solvent to facilitate the formation of suspension. In some embodiments, the method further comprises filtering a suspension in step (2). In some embodiments, the method further comprises filtering a suspension in step (2) after 5 to 9 days, 6 to 8 days, or about 7 days.

In some embodiments, provided is a method of preparing crystalline Form II of 5-(3,4-difluorobenzyl)-8-((1r,4r)-4-methylcyclohexyl)-6,9-dioxo-2,5,8-triazaspiro[3.5]nonane-2-carbaldehyde, the method comprising: (a) mixing 5-(3,4-difluorobenzyl)-8-((1r,4r)-4-methylcyclohexyl)-6,9-dioxo-2,5,8-triazaspiro[3.5]nonane-2-carbaldehyde with a solvent; and (b) subjecting the mixture generated in step (a) to heat/cool cycles. In some embodiments, the solvent is selected from the group consisting of ethanol, 1-propanol, 2-propanol, ethyl acetate, propyl acetate, isopropyl acetate, MIBK, 2-methyl-1-propanol, 1,2 dimethoxyethane, 2-methoxyethanol, DMF, diethyl ether, MTBE, n-heptane, cyclohexane, a mixture of ethanol and water, a mixture of 2-propanol and water, a mixture of 1,2-dimethoxyethane and water, a mixture of DMF and water, a mixture of NMP and water, a mixture of diethyl ether and n-heptane, a mixture of ethyl acetate and n-heptane, a mixture of propyl acetate and n-heptane, a mixture of isopropyl acetate and n-heptane, a mixture of MIBK and n-heptane, a mixture of MEK and n-heptane, a mixture of MTBE and n-heptane, a mixture of 2-methyl-1-propanol and n-heptane, a mixture of THF and n-heptane, a mixture of 2-propanol and n-heptane, a mixture of acetone and n-heptane, a mixture of chloroform and n-heptane. In some embodiments, the solvent is selected from the group consisting of ethanol, 1-propanol, 2-propanol, ethyl acetate, propyl acetate, isopropyl acetate, MIBK, 2-methyl-1-propanol, 1,2 dimethoxyethane, 2-methoxyethanol, DMF, diethyl ether, MTBE, n-heptane, cyclohexane, ethanol and water (1:1 v/v), 2-propanol and water (95:5 and 1:1 v/v), 1,2-dimethoxyethane and water (1:1 v/v), a mixture of DMF and water (1:1 v/v), a mixture of NMP and water (1:1 v/v), a mixture of diethyl ether and n-heptane (1:1 v/v), a mixture of ethyl acetate and n-heptane (1:1 v/v), a mixture of propyl acetate and n-heptane (1:1 v/v), a mixture of isopropyl acetate and n-heptane (1:1 v/v), a mixture of MIBK and n-heptane (1:1 v/v), a mixture of MEK and n-heptane (1:1 v/v), a mixture of MTBE and n-heptane (1:1 v/v), a mixture of 2-methyl-1-propanol and n-heptane (1:1 v/v), a mixture of THF and n-heptane (1:1 v/v), a mixture of 2-propanol and n-heptane (1:1 v/v), a mixture of acetone and n-heptane (1:1 v/v), and a mixture of chloroform and n-heptane (1:1 v/v). In some embodiments, the heat/cool cycles comprise cycles between room temperature and a temperature higher than room temperature. In some embodiments, the heat/cool cycles comprises cycles between room temperature and a temperature higher than room temperature, which is from 30 °C to 70 °C, 40°C to 60 °C, or about 50 °C. In some embodiments, the duration of each condition is from 1 to 6 hours, 2 to 5 hours, or about 4 hours. In some embodiments wherein biphasic layers are formed in step (a), the method further comprises sonicating the mixture of step (a) and/or subjecting the mixture of step (a) to evaporation. In some embodiments wherein biphasic layers are formed in step (a), the method further comprises sonicating the mixture of step (a) wherein the sonication duration is from 0.5 hours to 3 hours, or about 1.5 hours. In some embodiments wherein gums are formed in step (a), the method further comprising adding cyclohexane to the mixture of step (a) and sonicating the mixture of step (a). In some embodiments wherein gums are formed in step (a), the method further comprising adding cyclohexane to the mixture of step (a) and sonicating the mixture of step (a) wherein the sonication duration is from 2 hours to 6 hours, 3 hours to 5 hours, or about 4 hours. In some embodiments, the method further comprises filtering a solid generated in step (b). In some embodiments, the method further comprises filtering a solid generated in step (b) after 5 to 9 days, 6 to 8 days, or about 7 days.

In some embodiments, provided is a method of preparing crystalline Form II of 5-(3,4-difluorobenzyl)-8-((1r,4r)-4-methylcyclohexyl)-6,9-dioxo-2,5,8-triazaspiro[3.5]nonane-2-carbaldehyde, the method comprising: (a) mixing 5-(3,4-difluorobenzyl)-8-((1r,4r)-4-methylcyclohexyl)-6,9-dioxo-2,5,8-triazaspiro[3.5]nonane-2-carbaldehyde with a solvent; (b) stirring the mixture generated in step (a); and (c) collecting the aliquots of the mixture generated in step (b). In some embodiments, the step (b) comprises stirring the mixture of step (a) at a temperature from about 65 °C to 85 °C, from 70 °C to 80°C, or about 75 °C. In some embodiments, the step (b) comprises stirring the mixture of step (a) at a speed from 300 rpm to 700 rpm, from 400 rpm to 600 rpm, or about 500 rpm. In some embodiments, the step (b) comprises stirring the mixture of step (a) for 5 to 15 hours, 8 to 12 hours, or about 10 hours. In some embodiments wherein biphasic layers are formed in step (b), the method further comprises adding additional solvent to the mixture of step (c) until solution is formed, cooling the solution and stirring the solution at the cooled temperature. In some embodiments wherein biphasic layers are formed in step (b) and additional solvent is added to the mixture of step (c) until solution is formed, the temperature which the solution is cooled to and is stirred at is from 3 °C to 7°C, from 4°C to 6°C, or about 5°C. In some embodiments wherein biphasic layers are formed in step (b), the method further comprises adding additional solvent to the mixture of step (c) until solution is formed and cooling the solution at a speed from 0.08 °C/min to 0.12 °C/min, from 0.09 °C/min to 0.10 °C/min, or about 0.1 °C/min. In some embodiments, the solvent is selected from the group consisting of n-heptane, cyclohexane, a mixture of methanol and water, a mixture of ethanol and water, a mixture of 1-propanol and water, a mixture of 2-propanol and water, a mixture of 2-butanol and water, and a mixture of acetone and water. In some embodiments, the solvent is selected from the group consisting of n-heptane, cyclohexane, methanol and water (1:1 and 15:85 v/v), ethanol and water (1:1 and 85:15 v/v), 1-propanol and water (1:1 and 15:85 v/v), 2-propanol and water(1:1 and 15:85 v/v), 2-butanol and water (20:80 v/v), and acetone and water (1:1 v/v). In some embodiments, the method further comprises adding additional solvent. In some embodiments, the method further comprises adding anti-solvent to facilitate the formation of suspension. In some embodiments, the method further comprises filtering a suspension in step (c). In some embodiments, the method further comprises filtering a suspension in step (c) after 13 to 17 days, 14 to 16 days, or about 15 days. In some embodiments, the method further comprises filtering a suspension in step (c) after 16 to 20 days, 17 to 19 days, or about 18 days.

In some embodiments, provided is a method of preparing crystalline Form II of 5-(3,4-difluorobenzyl)-8-((1r,4r)-4-methylcyclohexyl)-6,9-dioxo-2,5,8-triazaspiro[3.5]nonane-2-carbaldehyde, the method comprising: (a) wetting 5-(3,4-difluorobenzyl)-8-((1r,4r)-4-methylcyclohexyl)-6,9-dioxo-2,5,8-triazaspiro[3.5]nonane-2-carbaldehyde with a solvent; and (b) grinding the mixture of step (a). In some embodiments, the step (b) comprises grinding the mixture of step (a) for certain amount time, which is from 1 hour to 3 hours, 1.5 hours to 2.5 hours, or about 2 hours. In some embodiments, the step (b) comprises grinding the mixture of step (a) at a speed from 3000 rpm to 7000 rpm, 4000 rpm to 6000 rpm, or about 5000 rpm. In some embodiments wherein gums are formed in step (a), the method further comprises drying the gums *in-vacuo.* In some embodiments wherein gums are formed in step (a), the method further comprises drying the gums *in-vacuo* at a temperature which is 10 °C higher or lower than the room temperature, 5 °C higher or lower than the room temperature, 3 °C higher or lower than the room temperature, or about the room temperature. In some embodiments wherein gums are formed in step (a), the method further comprises drying the gums *in-vacuo* for 5 to 15 hours, 8 to 12 hours, or about 10 hours. In some embodiments wherein gums are formed in step (a), the method further comprises drying the gums *in-vacuo.* In some embodiments wherein gums are formed in step (a), the method further comprises drying the gums *in-vacuo* at a temperature which is 10 °C higher or lower than the room temperature, 5 °C higher or lower than the room temperature, 3 °C higher or lower than the room temperature, or about the room temperature. In some embodiments wherein gums are formed in step (a), the method further comprises drying the gums *in-vacuo* for 1 days to 5 days, 2 days to 4 days, or about 3 days. In some embodiments wherein brittle materials are involved, the method further comprises drying the mixture containing brittle materials *in-vacuo.* In some embodiments wherein brittle materials are involved, the method further comprises drying the mixture containing brittle materials *in-vacuo* at a temperature which is 10 °C higher or lower than the room temperature, 5 °C higher or lower than the room temperature, 3 °C higher or lower than the room temperature, or about the room temperature. In some embodiments wherein brittle materials are involved, the method further comprises drying the mixture containing brittle materials *in-vacuo* for 5 to 15 hours, 8 to 12 hours, or about 10 hours. In some embodiments, the solvent is selected from the group consisting of a mixture of methanol and water, a mixture of ethanol and water, a mixture of 1-propanol and water, a mixture of 2-propanol and water, a mixture of 2-butanol and water, a mixture of acetone and water, a mixture of 1,4 dioxane and water, a mixture of THF and water, a mixture of acetonitrile and water, a mixture of DMSO and water, a mixture of dichloromethane and n-heptane. In some embodiments, the solvent is selected from the group consisting of methanol and water (97:3, 85:15 and 1:1 v/v), ethanol and water (97:3 and 85:15 v/v), 1-propanol and water (85:15 and 1:1 v/v), 2-propanol and water (98:2, 85:15 and 1:1 v/v), 2-butanol and water (98:2 v/v), acetone and water (1:1 v/v), 1,4 dioxane and water (99:1 and 1:1 v/v), THF and water (99:1 v/v), acetonitrile and water (1:1 v/v), DMSO and water (15:85 v/v), and dichloromethane and n-heptane (1:1 v/v).

In some embodiments, provided is a method of preparing crystalline Form II of 5-(3,4-difluorobenzyl)-8-((1r,4r)-4-methylcyclohexyl)-6,9-dioxo-2,5,8-triazaspiro[3.5]nonane-2-carbaldehyde, the method comprising: (1) forming a mixture of 5-(3,4-difluorobenzyl)-8-((1r,4r)-4-methylcyclohexyl)-6,9-dioxo-2,5,8-triazaspiro[3.5]nonane-2-carbaldehyde and a first solvent; (2) heating the mixture of step (1) to a first temperature; (3) adding a second solvent to the mixture of step (2) at the first temperature; (4) stirring the mixture of step (3) at the first temperature; (5) cooling the mixture of step (4) to a second temperature; (6) stirring the mixture of step (5) at the second temperature; and (7) filtering the mixture of step (6) at the second temperature to obtain crystalline solids. In some embodiments, the first solvent comprises ethanol and water. In some embodiments, the first solvent is ethanol. In some embodiments, the second solvent comprises water. In some embodiments, the second solvent is water. In some embodiments, the first temperature is from 50 °C to 110 °C, from 60 °C to 100 °C, from 70 °C to 90 °C, or about 80 °C. In some embodiments, the first temperature is 78 °C. In some embodiments, the first temperature is 85 °C. In some embodiments, the first temperature is from 60 °C to 120 °C, from 70 °C to 110 °C, from 80 °C to 100 °C, or about 90 °C. In some embodiments, the second temperature is 10 °C higher or lower than the room temperature, 5 °C higher or lower than the room temperature, 3 °C higher or lower than the room temperature, or about the room temperature. In some embodiments wherein the first temperature is from 85 °C to 90 °C, step (5) further comprises adding a crystalline seed of crystalline Form II to the mixture of step (4) at an intermediate temperature between the first temperature and the second temperature. In some embodiments wherein a crystalline seed of Form II is added to the mixture of step (4) at an intermediate temperature, the intermediate temperature is from 40 °C to 60 °C, from 45 °C to 55 °C, or about 50 °C. In some embodiments wherein a crystalline seed of Form II is added to the mixture of step (4) at an intermediate temperature, the intermediate temperature is from 60 °C to 80 °C, from 65 °C to 75 °C, or about 70 °C. In some embodiments, step (2) further comprises adding the second solvent during the heating process. In some embodiments wherein step (2) comprises adding the second solvent during the heating process, the second solvent is added at temperature from 45 °C and 55 °C, or about 50 °C and/or is added at temperature from 60 °C to 70°C, or about 65 °C. In some embodiments, the method further comprises drying the solid in step (7) under air or under vacuum. In some embodiments, the method further comprises drying the solid in step (7) below 50 °C. In some embodiments, the method further comprises drying the solid in step (7) at a temperature which is 10 °C higher or lower than the room temperature, 5 °C higher or lower than the room temperature, 3 °C higher or lower than the room temperature, or about the room temperature. In some embodiments the method further comprises drying the solid in step (7) under air or under vacuum for 1 days to 5 days, 2 days to 4 days, or about 3 days.

Form II can also be prepared with the procedures provided in Example 2 and/or 5.

### Form III

In some embodiments, provided is a method of preparing crystalline Form III of 5-(3,4-difluorobenzyl)-8-((1r,4r)-4-methylcyclohexyl)-6,9-dioxo-2,5,8-triazaspiro[3.5]nonane-2-carbaldehyde, the method comprising: (1) mixing 5-(3,4-difluorobenzyl)-8-((1r,4r)-4-methylcyclohexyl)-6,9-dioxo-2,5,8-triazaspiro[3.5]nonane-2-carbaldehyde with a solvent comprising 1,4-dioxane; and (2) stirring the mixture generated in step (1) to form a suspension. In some embodiments, the step (2) comprises stirring the mixture of step (1) at a temperature from 4 °C to 6 °C, 4.5 °C to 5.5 °C, or about 5 °C. In some embodiments, the solvent is a mixture of 1,4-dioxane and water. In some embodiments, the solvent is the mixture of 1,4-dioxane and water (v/v 1:1). In some embodiments, the method further comprises adding additional solvent. In some embodiments, the method further comprises adding anti-solvent to facilitate the formation of suspension. In some embodiments, the method further comprises filtering a suspension in step (2). In some embodiments, the method further comprises filtering a suspension in step (2) after 5 to 9 days, 6 to 8 days, or about 7 days.

Form III can also be prepared with the procedures provided in Example 4 and/or 5.

### Methods of Use

The crystalline forms and compositions provided herein may be used to treat or prevent a disease or condition in an individual or subject.

When used in a prophylactic manner, the crystalline forms and compositions disclosed and/or described herein may prevent a disease or disorder from developing or lessen the extent of a disease or disorder that may develop in an individual or subject at risk of developing the disease or disorder.

Without being bound by theory, the crystalline forms and compositions provided are believed to act by inhibiting myosin. This inhibition potentially decreases the number of independent myosin heads interacting with actin filaments reducing the amount of contraction. Reducing contraction of cardiac muscle can be important for the treatment of heart diseases in which over-contraction is an issue. In some embodiments, provided are methods of treating or preventing heart disease in an individual or subject, comprising administering to the individual or subject in need thereof a crystalline form or composition provided herein. In some embodiments, provided are methods of treating or preventing heart disease in a subject in need thereof comprising administering to the subject a therapeutically effective amount of a crystalline form or composition provided herein. In some embodiments, provided are methods of treating heart disease in a subject in need thereof comprising administering to the subject a therapeutically effective amount of a crystalline form or composition provided herein. In some embodiments, provided are methods of treating an established or diagnosed heart disease in a subject in need thereof comprising administering to the subject a therapeutically effective amount of a crystalline form or composition provided herein. In some embodiments, provided are methods of preventing heart disease in a subject in need thereof comprising administering to the subject a therapeutically effective amount of a crystalline form or composition provided herein.

Also provided herein is the use of a crystalline form or composition provided herein in the manufacture of a medicament for treatment of a heart disease in a subject. In some aspects, provided is a crystalline form as described herein for use in a method of treatment of the human or animal body by therapy. In some embodiments, provided herein is a crystalline form, such as Form I, II, or III, or the mixture thereof, or composition thereof, for use in a method of treatment of the human or animal body by therapy. In some embodiments, provided herein is a crystalline form, such as Form I, II, or III, or the mixture thereof, or composition thereof, for use in treating or preventing heart disease. In some embodiments, provided herein is a crystalline form, such as Form I, II, or III, or the mixture thereof, or composition thereof, for use in treating heart disease. In some embodiments, provided herein is a crystalline form, such as Form I, II, or III, or the mixture thereof, or composition thereof, for use in treating an established or diagnosed heart disease. In other embodiments, provided herein is a crystalline form, such as Form I, II, or III, or the mixture thereof, or composition thereof, for use in preventing heart disease. In some embodiments, provided herein is a crystalline form, such as Form I, II, or III, or the mixture thereof, or composition thereof, for use in treating a disease or condition associated with HCM. In some embodiments, provided herein is a crystalline form, such as Form I, II, or III, or the mixture thereof, or composition thereof, for use in treating a disease or condition associated with secondary left ventricular wall thickening. In some embodiments, provided herein is a crystalline form, such as Form I, II, or III, or the mixture thereof, or composition thereof, for use in ameliorating a symptom associated with heart disease. In other embodiments, provided herein is a crystalline form, such as Form I, II, or III, or the mixture thereof, or composition thereof, for use in reducing the risk of a symptom associated with heart disease. In other embodiments, provided herein is a crystalline form, such as Form I, II, or III, or the mixture thereof, or composition thereof, for use in treating a disease or condition associated with small left ventricular cavity, cavity obliteration, hyperdynamic left ventricular contraction, obstruction of blood flow out of the left ventricle, cardiac hypertrophy, small cardiac stroke volume, impaired relaxation of the left ventricle, high left ventricle filling pressure, myocardial ischemia, or cardiac fibrosis. In certain embodiments, provided herein is a crystalline form, such as Form I, II, or III, or the mixture thereof, or composition thereof, for use in treating a disease or condition associated with small left ventricular cavity and cavity obliteration, hyperdynamic left ventricular contraction, myocardial ischemia, or cardiac fibrosis. In some embodiments, provided herein is a crystalline form, such as Form I, II, or III, or the mixture thereof, or composition thereof, for use in treating muscular dystrophies. In some embodiments, provided herein is a crystalline form, such as Form Form I, II, or III, or the mixture thereof, or composition thereof, for use in treating a glycogen storage disease. In other embodiments, provided herein is a crystalline form, such as Form I, II, or III, or the mixture thereof, or composition thereof, for use in modulating the cardiac sarcomere, such as inhibiting the cardiac sarcomere. In yet other embodiments, provided herein is a crystalline form, such as Form I, II, or III, or the mixture thereof, or composition thereof, for use in potentiating cardiac myosin.

In some embodiments, the subject is a mammal. In some embodiments, the subject is a mouse, rat, dog, cat, pig, sheep, horse, cow, or human. In some embodiments, the subject is a human. In some embodiments, the subject has an established or diagnosed heart disease. In some embodiments, the subject has established or diagnosed hypertrophic cardiomyopathy (HCM). In some embodiments, the subject is at risk for developing heart disease. In some embodiments, the subject has a mutation that increases risk for heart disease. In some embodiments, the subject has a mutation that increases risk for hypertrophic cardiomyopathy (HCM). In some embodiments, the mutation is a sarcomeric mutation. In some embodiments, the mutation is a mutation in myosin heavy chain β (MHC-β), cardiac muscle troponin T (cTnT), tropomyosin alpha-1 chain (TPM1), myosin-binding protein C cardiac-type (MYBPC3), cardiac troponin I (cTnI), myosin essential light chain (ELC), titin (TTN), myosin regulatory light chain 2 ventricular/cardiac muscle isoform (MLC-2), cardiac muscle alpha actin, muscle LIM protein (MLP), or protein kinase AMP-activated non-catalytic subunit gamma 2 (PRKAG2). In some embodiments, the mutation is a mutation in MHC-β. In some embodiments, the subject has established or diagnosed hypertrophic cardiomyopathy without a confirmed genetic etiology.

In some embodiments, the subject has a high risk of progressive symptoms. In some embodiments, the subject has a high risk of atrial fibrillation, ventricular tachyarrhythmias, stroke, and/or sudden death. In some embodiments, the subject has a reduced exercise capacity. In some embodiments, the reduced exercise capacity is as compared to an age-matched control population. In some embodiments, the subject is eligible for surgical intervention or percutaneous ablation to treat the heart disease.

In some embodiments, the heart disease is hypertrophic cardiomyopathy (HCM). In some embodiments, the heart disease is obstructive HCM. In some embodiments, the heart disease is nonobstructive HCM. In some embodiments, the HCM is associated with a sarcomeric mutation. In some embodiments, the HCM is associated with a non-sarcomeric mutation. In some embodiments, the heart disease is obstructive or nonobstructive HCM caused by sarcomeric and/or non-sarcomeric mutations. In some embodiments, the sarcomeric mutation is a mutation in a myosin heavy chain β (MHC-β), cardiac muscle troponin T (cTnT), tropomyosin alpha-1 chain (TPM1), myosin-binding protein C cardiac-type (MYBPC3), cardiac troponin I (cTnI), myosin essential light chain (ELC), titin (TTN), myosin regulatory light chain 2 ventricular/cardiac muscle isoform (MLC-2), cardiac muscle alpha actin, or muscle LIM protein (MLP). In some embodiments, the sarcomeric mutation is a mutation in MHC-β. In some embodiments, the non-sarcomeric mutation is a mutation in protein kinase AMP-activated non-catalytic subunit gamma 2 (PRKAG2).

In some embodiments, provided herein are methods of treating a disease or condition associated with HCM, comprising administering to the individual or subject in need thereof a crystalline form or composition provided herein. In some embodiments, the disease or condition is Fabry's Disease, Danon Disease, mitochondrial cardiomyopathies, or Noonan Syndrome.

Also provided herein is the use of a crystalline form or composition provided herein in the manufacture of a medicament for treatment of a disease or condition associated with HCM.

In some embodiments, the heart disease is heart failure with preserved ejection fraction (HFpEF). In some embodiments, the heart disease is diastolic dysfunction. In some embodiments, the heart disease is cardiomyopathy. In some embodiments, the heart disease is primary or secondary restrictive cardiomyopathy. In some embodiments, the heart disease is condition or symptoms caused by coronary artery disease. In some embodiments, the heart disease is myocardial infarction or angina pectoris. In some embodiments, the heart disease is left ventricular outflow tract obstruction. In some embodiments, the heart disease is hypertensive heart disease. In some embodiments, the heart disease is congenital heart disease. In some embodiments, the heart disease is cardiac ischemia and/or coronary heart disease. In some embodiments, the heart disease is diabetic heart disease. In other embodiments, the heart disease is congestive heart failure. In some embodiments, the heart disease is right heart failure. In other embodiments, the heart disease is cardiorenal syndrome. In some embodiments, the heart disease is infiltrative cardiomyopathy. In some embodiments, the heart disease is a condition that is or is related to cardiac senescence or diastolic dysfunction due to aging. In some embodiments, the heart disease is a condition that is or is related to left ventricular hypertrophy and/or concentric left ventricular remodeling.

In some embodiments, the provided are methods of treating a disease or condition associated with secondary left ventricular wall thickening in an individual or subject, comprising administering to the individual or subject in need thereof a crystalline form or composition provided herein. In some embodiments, the disease is hypertension, valvular heart diseases (aortic stenosis, Mitral valve regurgitation), metabolic syndromes (diabetes, obesity), end stage renal disease, scleroderma, sleep apnea, amyloidosis, Fabry's disease, Friedreich Ataxia, Danon disease, Noonan syndrome, or Pompe disease.

Also provided herein is the use of a crystalline form or composition provided herein in the manufacture of a medicament for treatment of a disease or condition associated with secondary left ventricular wall thickening.

In some embodiments, provided are methods of ameliorating a symptom associated with heart disease in a subject, comprising administering to the individual or subject in need thereof a crystalline form or composition provided herein, wherein the symptom is one or more selected from poor or reduced cardiac elasticity, poor or reduced diastolic left ventricular relaxation, abnormal left atrial pressure (e.g., abnomally high left atrial pressure), paroxysmal or permanent atrial fibrillation, increased left atrial and pulmonary capillary wedge pressures, increased left ventricular diastolic pressures, syncope, ventricular relaxation during diastole, ventricular fibrosis, left ventricular hypertrophy, left ventricular mass, increased left ventricular wall thickness, left ventricular mid-cavity obstruction, increased systolic anterior motion of mitral valve, left ventricular outflow tract obstruction, chest pain, exertional dyspnea, pre-syncope, abnormal exercise capacity, and fatigue.

In some embodiments, the provided are methods of reducing the risk of a symptom associated with heart disease in a subject, comprising administering to the individual or subject in need thereof a crystalline form or composition provided herein, wherein the symptom is one or more selected from sudden cardiac death, poor or reduced cardiac elasticity, poor or reduced diastolic left ventricular relaxation, abnormal left atrial pressure (e.g., abnomally high left atrial pressure), paroxysmal or permanent atrial fibrillation, increased left atrial and pulmonary capillary wedge pressures, increased left ventricular diastolic pressures, syncope, ventricular relaxation during diastole, ventricular fibrosis, left ventricular hypertrophy, left ventricular mass, increased left ventricular wall thickness, left ventricular mid-cavity obstruction, increased systolic anterior motion of mitral valve, left ventricular outflow tract obstruction, chest pain, exertional dyspnea, pre-syncope, abnormal exercise capacity, and fatigue.

In some embodiments, the provided are methods of treating a disease or condition associated with small left ventricular cavity, cavity obliteration, hyperdynamic left ventricular contraction, obstruction of blood flow out of the left ventricle, cardiac hypertrophy, small cardiac stroke volume, impaired relaxation of the left ventricle, high left ventricle filling pressure, myocardial ischemia, or cardiac fibrosis in an individual or subject, comprising administering to the individual or subject in need thereof a crystalline form or composition provided herein.

In some embodiments, the provided are methods of treating a disease or condition associated with small left ventricular cavity and cavity obliteration, hyperdynamic left ventricular contraction, myocardial ischemia, or cardiac fibrosis in an individual or subject, comprising administering to the individual or subject in need thereof a crystalline form or composition provided herein.

Also provided herein is the use of a crystalline form or composition provided herein in the manufacture of a medicament for treatment of a disease or condition associated with small left ventricular cavity and cavity obliteration, hyperdynamic left ventricular contraction, myocardial ischemia, or cardiac fibrosis.

In some embodiments, the provided are methods of treating muscular dystrophies in an individual or subject (e.g., Duchenne muscular dystrophy), comprising administering to the individual or subject in need thereof a crystalline form or composition provided herein. Also provided herein is the use of a crystalline form or composition provided herein in the manufacture of a medicament for treatment of muscular dystrophies (*e.g.*, Duchenne muscular dystrophy).

In some embodiments, the provided are methods of treating a glycogen storage disease in an individual or subject, comprising administering to the individual or subject in need thereof a crystalline form or composition provided herein. Also provided herein is the use of a crystalline form or composition provided herein in the manufacture of a medicament for treatment of a glycogen storage disease.

Also provided are methods for modulating the cardiac sarcomere in an individual or subject which method comprises administering to an individual or subject in need thereof a therapeutically effective amount of at least one chemical entity as described herein. In some embodiments, provided are methods of inhibiting the cardiac sarcomere, comprising contacting the cardiac sarcomere with at least one chemical entity as described herein, such as a crystalline form or composition provided herein. Additionally provided herein is the use of at least one chemical entity as described herein, such as a crystalline form or composition provided herein in the manufacture of a medicament for inhibiting the cardiac sarcomere of an individual or subject.

Also provided are methods for potentiating cardiac myosin in an individual or subject which method comprises administering to an individual or subject in need thereof a therapeutically effective amount of at least one chemical entity as described herein such as a crystalline form or composition provided herein. Additionally provided herein is the use of at least one chemical entity as described herein, such as a crystalline form or composition provided herein in the manufacture of a medicament for potentiating cardiac myosin in an individual or subject.

In some embodiments, the methods provided herein further comprise monitoring the effectiveness of the treatment. Examples of indicators include, but are not limited to improvement in one or more of the following: New York Heart Association (NYHA) Functional Classification, exercise capacity, cardiac elasticity, diastolic left ventricular relaxation, left atrial pressure, paroxysmal or permanent atrial fibrillation, left atrial and pulmonary capillary wedge pressures, left ventricular diastolic pressures, syncope, ventricular relaxation during diastole, ventricular fibrosis, left ventricular hypertrophy, left ventricular mass, left ventricular wall thickness, left ventricular mid-cavity obstruction systolic anterior motion of mitral valve, left ventricular outflow tract obstruction, chest pain, exertional dyspnea, pre-syncope, abnormal exercise capacity, and fatigue. These indicators can be monitored by techniques known in the art including self-reporting; ECG, including ambulatory ECG; echocardiography; cardiac MRI; CT; biopsy; cardiopulmonary exercise testing (CPET); and actigraphy.

In some embodiments, the crystalline forms or compositions described therein reduce the contractility of a cardiomyocyte. In some embodiments, the crystalline forms or compositions reduce the contractility of a cardiomyocyte by greater than 40%, such as greater than 45%, 50%, 60%, 70%, 80%, or 90%. In some embodiments, the crystalline forms or compositions reduce the contractility of a cardiomyocyte 40%-90%, such as 40%-80%, 40-70%, 50%-90%, 50%-80% or 50%-70%. In some embodiments, the crystalline forms or compositions do not significantly alter calcium transients in the cardiomyocyte. In some embodiments, the crystalline forms or compositions decrease the ATPase activity in a cardiomyocyte. Methods of measuring contractility, ATPase activity, and calcium transients are known in the art, for example, by calcium labeling, electrophysiological recordings, and microscopic imaging. In some embodiments, the crystalline forms or compositions do not significantly inhibit or induce a cytochrome P450 (CYP) protein.

In some embodiments, provided herein are crystalline forms or copositions, wherein the elimination half-life (t_{½}; calculated as ln(2)/k, wherein the elimination rate constant, k, is calculated as the absolute value of the slope of the linear regression of logarithm of the concentration versus time for the last three data points of a concentration-time profile) is ≤ 30 hours in a human. In some embodiments, 10 hours ≤ t_{½} ≤ 30 hours in a human. In some embodiments, t_{½} is between about 10 hours and about 30 hours, between about 10 hours and about 25 hours, between about 15 hours and about 30 hours, or between about 15 hours and about 25 hours. In some embodiments, t_{½} is about 12, 15, 18, 21, 24, 27, or 30 hours. In some embodiments, the elimination half-life of a compound provided herein is such that the compound is suitable for once-daily dosing.

In some embodiments, the subject has a left ventricular wall that is thicker than normal prior to treatment. In some embodiments, the subject has a left ventricular wall thickness that is greater than 15 mm, such as greater than 18 mm, 20 mm, 22 mm, 25 mm, or 30 mm prior to treatment. In some embodiments, the left ventricular wall thickness is reduced by greater than 5%, such as greater than 8%, 10%, 12%, 15%, 20%, or 30% following treatment. Left ventricular wall thickness can be measured by methods known in the art, such as by echocardiography, CT scan, or a cardiac MRI.

In some embodiments, the subject has abnormal cardiac fibrosis prior to treatment. In some embodiments, the abnormal cardiac fibrosis is reduced by greater than 5%, such as greater than 8%, 10%, 12%, 15%, 20%, or 30% following treatment. Cardiac fibrosis can be measured by methods known in the art, such as by biopsy or a cardiac MRI.

In some embodiments, the subject has reduced exercise capacity prior to treatment. In some embodiments, the exercise capacity of the subject is increased by greater than 5%, such as greater than 8%, 10%, 12%, 15%, 20% or 30% following treatment. In some embodiments, the exercise capacity is measured by cardiopulmonary exercise testing (CPET). CPET measures changes in oxygen consumption (VO₂ max). Methods of measuring CPET and VO₂ max are well known in the art (Malhotra et al., JACC: Heart Failure, 2016, 4(8): 607-616; Guazzi et al., J Amer College Cardiol, 2017, 70 (13): 1618-1636; Rowin et al., JACC: Cariovasc Imaging, 2017, 10(11):1374-1386). In some embodiments, VO₂ max is improved by more than 1 mL/kg/m², such as more than 1.2 mL/kg/m², 1.4 mL/kg/m², 1.5 mL/kg/m², 1.7 mL/kg/m², 2 mL/kg/m², 2.2 mL/kg/m², 2.5 mL/kg/m², 3 mL/kg/m², 3.2 mL/kg/m², or 3.5 mL/kg/m² following treatment.

In some embodiments, the subject has a New York Heart Association (NYHA) Functional Classification of II, III, or IV prior to treatment. In some embodiments, the subject has a New York Heart Association (NYHA) Functional Classification of III or IV prior to treatment. In some embodiments, the subject has a New York Heart Association (NYHA) Functional Classification of IV prior to treatment. In some embodiments, the subject remains in the same NYHA functional class or has a reduced NYHA functional class following treatment.

In some embodiments, VO₂ max is improved by more than 1 mL/kg/m², such as more than 1.2 mL/kg/m², 1.4 mL/kg/m², 1.5 mL/kg/m², 1.7 mL/kg/m², or 2 mL/kg/m² and the subject has a reduced NYHA functional class following treatment. In some embodiments, VO₂ max is improved by more than 2.5 mL/kg/m², 3 mL/kg/m², 3.2 mL/kg/m², or 3.5 mL/kg/m² and the subject remains in the same NYHA functional class or has a reduced NYHA functional class following treatment.

In some embodiments, daily function and/or activity level of the subject is improved following treatment. Improved daily function and/or activity level may be measured, for example, by journaling or actigraphy, such as a wearable physical fitness monitor or activity tracker (e.g., FITBIT^{®} or FITBIT^{®}-like monitors).

In some embodiments, the subject has one or more of decreased shortness of breath, decreased chest pain, decreased arrhythmia burden, such as atrial fibrillation and ventricular arrhythmias, decreased incidence of heart failure, and decreased ventricular outflow obstruction following treatment.

### Dosages

The crystalline forms and compositions disclosed and/or described herein are administered at a therapeutically effective dosage, e.g., a dosage sufficient to provide treatment for the disease state. While human dosage levels have yet to be optimized for the chemical entities described herein, generally, a daily dose ranges from about 0.01 to 100 mg/kg of body weight; in some embodiments, from about 0.05 to 10.0 mg/kg of body weight, and in some embodiments, from about 0.10 to 1.4 mg/kg of body weight. Thus, for administration to a 70 kg person, in some embodiments, the dosage range would be about from 0.7 to 7000 mg per day; in some embodiments, about from 3.5 to 700.0 mg per day, and in some embodiments, about from 7 to 100.0 mg per day. The amount of the chemical entity administered will be dependent, for example, on the subject and disease state being treated, the severity of the affliction, the manner and schedule of administration and the judgment of the prescribing physician. For example, an exemplary dosage range for oral administration is from about 5 mg to about 500 mg per day, and an exemplary intravenous administration dosage is from about 5 mg to about 500 mg per day, each depending upon the pharmacokinetics.

A daily dose is the total amount administered in a day. A daily dose may be, but is not limited to be, administered each day, every other day, each week, every 2 weeks, every month, or at a varied interval. In some embodiments, the daily dose is administered for a period ranging from a single day to the life of the subject. In some embodiments, the daily dose is administered once a day. In some embodiments, the daily dose is administered in multiple divided doses, such as in 2, 3, or 4 divided doses. In some embodiments, the daily dose is administered in 2 divided doses.

Administration of the crystalline forms and compositions described herein can be via any accepted mode of administration for therapeutic agents including, but not limited to, oral, sublingual, subcutaneous, parenteral, intravenous, intranasal, topical, transdermal, intraperitoneal, intramuscular, intrapulmonary, vaginal, rectal, or intraocular administration. In some embodiments, the crystalline form or composition is administered orally or intravenously. In some embodiments, the crystalline form or composition disclosed and/or described herein is administered orally.

Pharmaceutically acceptable compositions include solid, semi-solid, liquid and aerosol dosage forms, such as tablet, capsule, powder, liquid, suspension, suppository, and aerosol forms. The crystalline forms disclosed and/or described herein can also be administered in sustained or controlled release dosage forms (e.g., controlled/sustained release pill, depot injection, osmotic pump, or transdermal (including electrotransport) patch forms) for prolonged timed, and/or pulsed administration at a predetermined rate. In some embodiments, the compositions are provided in unit dosage forms suitable for single administration of a precise dose.

The crystalline forms described herein can be administered either alone or in combination with one or more conventional pharmaceutical carriers or excipients (e.g., mannitol, lactose, starch, magnesium stearate, sodium saccharine, talcum, cellulose, sodium crosscarmellose, glucose, gelatin, sucrose, magnesium carbonate). If desired, the pharmaceutical composition can also contain minor amounts of nontoxic auxiliary substances such as wetting agents, emulsifying agents, solubilizing agents, pH buffering agents and the like (e.g., sodium acetate, sodium citrate, cyclodextrine derivatives, sorbitan monolaurate, triethanolamine acetate, triethanolamine oleate). Generally, depending on the intended mode of administration, the pharmaceutical composition will contain about 0.005% to 95%, or about 0.5% to 50%, by weight of a compound disclosed and/or described herein. Actual methods of preparing such dosage forms are known, or will be apparent, to those skilled in this art; for example, see Remington's Pharmaceutical Sciences, Mack Publishing Company, Easton, Pennsylvania.

In some embodiments, the compositions will take the form of a pill or tablet and thus the composition may contain, along with a crystalline form disclosed and/or described herein, one or more of a diluent (e.g., lactose, sucrose, dicalcium phosphate), a lubricant (e.g., magnesium stearate), and/or a binder (e.g., starch, gum acacia, polyvinylpyrrolidine, gelatin, cellulose, cellulose derivatives). Other solid dosage forms include a powder, marume, solution or suspension (e.g., in propylene carbonate, vegetable oils or triglycerides) encapsulated in a gelatin capsule.

Liquid pharmaceutically administrable compositions can, for example, be prepared by dissolving, dispersing or suspending etc. a crystalline form disclosed and/or described herein and optional pharmaceutical additives in a carrier (e.g., water, saline, aqueous dextrose, glycerol, glycols, ethanol or the like) to form a solution or suspension. Injectables can be prepared in conventional forms, either as liquid solutions or suspensions, as emulsions, or in solid forms suitable for dissolution or suspension in liquid prior to injection. The percentage of the crystalline form contained in such parenteral compositions depends, for example, on the physical nature of the crystalline form, the activity of the crystalline form and the needs of the subject. However, percentages of active ingredient of 0.01% to 10% in solution are employable, and may be higher if the composition is a solid which will be subsequently diluted to another concentration. In some embodiments, the composition will comprise from about 0.2 to 2% of a crystalline form disclosed and/or described herein in solution.

Pharmaceutical compositions of the crystalline forms and compositions described herein may also be administered to the respiratory tract as an aerosol or solution for a nebulizer, or as a microfine powder for insufflation, alone or in combination with an inert carrier such as lactose. In such a case, the particles of the pharmaceutical composition may have diameters of less than 50 microns, or in some embodiments, less than 10 microns.

In addition, pharmaceutical compositions can include a crystalline form disclosed and/or described herein and one or more additional medicinal agents, pharmaceutical agents, adjuvants, and the like. Suitable medicinal and pharmaceutical agents include those described herein.

### Kits

Also provided are articles of manufacture and kits containing any of the crystalline forms or compositions provided herein. The article of manufacture may comprise a container with a label. Suitable containers include, for example, bottles, vials, and test tubes. The containers may be formed from a variety of materials such as glass or plastic. The container may hold a pharmaceutical composition provided herein. The label on the container may indicate that the pharmaceutical composition is used for preventing, treating or suppressing a condition described herein, and may also indicate directions for either in vivo or in vitro use.

In one aspect, provided herein are kits containing a crystalline form or composition described herein and instructions for use. The kits may contain instructions for use in the treatment of a heart disease in an individual or subject in need thereof. A kit may additionally contain any materials or equipment that may be used in the administration of the crystalline forms or composition, such as vials, syringes, or IV bags. A kit may also contain sterile packaging.

### Combinations

The crystalline forms and compositions described herein may be administered alone or in combination with other therapies and/or therapeutic agents useful in the treatment of the aforementioned disorders, diseases, or conditions.

The crystalline forms and compositions described and/or disclosed herein may be combined with one or more other therapies to treat a heart disease, such as HCM or HFpEF. In some embodiments, the one or more therapies include therapies that retard the progression of heart failure by down-regulating neurohormonal stimulation of the heart and attempt to prevent cardiac remodeling (e.g., ACE inhibitors, angiotensin receptor blockers (ARBs), β-blockers, aldosterone receptor antagonists, or neural endopeptidase inhibitors). In some embodiments, the one or more therapies include therapies that improve cardiac function by stimulating cardiac contractility (e.g., positive inotropic agents, such as the β-adrenergic agonist dobutamine or the phosphodiesterase inhibitor milrinone). In other embodiments, the one or more therapies include therapies that reduce cardiac preload (e.g., diuretics, such as furosemide) or afterload (vasodilators of any class, including but not limited to calcium channel blockers, phosphodiesterase inhibitors, endothelin receptor antagonists, renin inhibitors, or smooth muscle myosin modulators).

The crystalline forms and compositions described and/or disclosed herein may be combined with one or more other therapies to treat HCM or HFpEF. In some embodiments, the crystalline forms and/compositions may be combined with a β-blocker, verapamil, and/or disopyramide.

### EXAMPLES

The following examples are provided to further aid in understanding the embodiments disclosed in the application, and presuppose an understanding of conventional methods well known to those persons having ordinary skill in the art to which the examples pertain. The particular materials and conditions described hereunder are intended to exemplify particular aspects of embodiments disclosed herein and should not be construed to limit the reasonable scope thereof.

The following abbreviations may be used herein:

| **Abbreviations** | **Meaning** |
|---|---|
| ¹H NMR | Proton Nuclear Magnetic Resonance |
| 1-PrOH | 1-Propanol |
| API | Active Pharmaceutical Ingredient |
| *ca.* | Approximately |
| DI water | Deionised Water |
| DMF | *N,N*-Dimethylformamide |
| DMSO | Dimethyl sulfoxide |
| D-PAS | Dip Probe Absorption Spectroscopy |
| DSC | Differential Scanning Calorimetry |
| DVS | Dynamic Vapor Sorption |
| eq | Equivalents |
| Eq. | Equivalents |
| EtOAc | Ethyl acetate |
| EtOH | Ethanol |
| GVS | Gravimetric Vapor Sorption |
| H₂O | Water |
| HCl | Hydrochloric acid |
| HPLC | High Performance Liquid Chromatography |
| IC | Ion Chromatography |
| ICH | International Council for Harmonisation of Technical Requirements for Pharmaceuticals for Human Use |
| ID | Identification |
| IPA | 2-Propanol |
| KHP | Potassium hydrogen phthalate |
| MeCN | Acetonitrile |
| MEK | Methyl ethyl ketone |
| MeOH | Methanol |
| MIBK | Methyl isobutyl ketone |
| min | Minutes |
| mol | Molar |
| MS | Mass Spectroscopy |
| N/A | Not Applicable |
| NMP | N-Methylpyrrolidone |
| NMR | Nuclear Magnetic Resonance |
| No. | Number |
| PTFE | Polytetrafluoroethylene |
| RH | Relative Humidity |
| RRT | Relative Retention Time |
| R.T. | Room Temperature |
| SCXRD | Single Crystal X-Ray Diffraction |
| TBME | *tert*-Butyl methyl ether |
| Temp | Temperature |
| TFA | Trifluoric acid |
| Tg | Glass transition temperature |
| TGA | Thermal Gravimetric Analysis |
| THF | Tetrahydrofuran |
| v/v | Volume to volume ratio |
| Vol | Volumes |
| XRPD | X-Ray Powder Diffraction |

The crystalline Forms of 5-(3,4-difluorobenzyl)-8-((1r,4r)-4-methylcyclohexyl)-6,9-dioxo-2,5,8-triazaspiro[3.5]nonane-2-carbaldehyde were characterized by various analytical techniques, including XRPD, DSC, TGA, GVS, HPLC, using the procedures described below.

### XRPD

### 1. XRPD with Bruker AXS D8 Advance

XRPD diffractograms were collected on a Bruker D8 diffractometer using Cu Kα radiation (40 kV, 40 mA) and a θ-2θ goniometer fitted with a Ge monochromator. The incident beam passes through a 2.0 mm divergence slit followed by a 0.2 mm anti-scatter slit and knife edge. The diffracted beam passes through an 8.0 mm receiving slit with 2.5° Soller slits followed by the Lynxeye Detector. The software used for data collection and analysis was Diffrac *Plus* XRD Commander and Diffrac *Plus* EVA respectively.

Samples were run under ambient conditions as flat plate specimens using powder as received. The sample was prepared on a polished, zero-background (510) silicon wafer by gently pressing onto the flat surface or packed into a cut cavity. The sample was rotated in its own plane.
The details of the standard Pharmorphix data collection method are:
- Angular range: 2 to 42° 2θ
- Step size: 0.05° 2θ
- Collection time: 0.5 s/step (total collection time: 6.40 min)
When required other methods for data collection are used with details as follows:
- Angular range: 2 to 31° 2θ
- Step size: 0.06° 2θ
- Collection time: 0.5 s/step

### 2. XRPD with PANalytical Empyrean

XRPD diffractograms were collected on a PANalytical Empyrean diffractometer using Cu Kα radiation (45 kV, 40 mA) in transmission geometry. A 0.5° slit, 4 mm mask and 0.04 rad Soller slits with a focusing mirror were used on the incident beam. A PIXcel^{3D} detector, placed on the diffracted beam, was fitted with a receiving slit and 0.04 rad Soller slits. The software used for data collection was X'Pert Data Collector using X'Pert Operator Interface. The data were analysed and presented using Diffrac *Plus* EVA or HighScore Plus.

Samples were prepared and analysed in a metal 96 well-plate in transmission mode. X-ray transparent film was used between the metal sheets on the metal well-plate and powders (approximately 1 - 2 mg) were used as received.

The scan mode for the metal plate used the gonio scan axis.

The details of the standard screening data collection method are:
Angular range: 2.5 to 32.0° 2θ
Step size: 0.0130° 2θ
Collection time: 12.75 s/step (total collection time of 2.07 min)

### X-ray Single Crystal Diffraction (XRSD) and Structural Refinement

### XRSD Data Collection and Structure Refinement Details

| | | |
|---|---|---|
| Diffractometer | XtaLAB Synergy-S, Dualflex, HyPix-6000HE | |
| Radiation source | PhotonJet (Cu) X-ray Source, CuKα | |
| Data collection method | omega scans | |
| θ range for data collection | 3.755 to 70.076° | |
| Index ranges | -17 ≤ *h* ≤ 17, -17 ≤ *k* ≤ 17, -19 ≤ *l* ≤ 19 | |
| Reflections collected | 163951 | |
| Independent reflections | 11461 [R(int) = 0.0501] | |
| Coverage of independent reflections | 99.9 % | |
| Absorption correction | Multi-Scan | |
| Max. and min. transmission | 1.00000 and 0.44904 | |
| Structure solution technique | Direct Methods | |
| Structure solution program | SHELXTL (Sheldrick, 2013) | |
| Refinement technique | Full-matrix least-squares on *F*² | |
| Refinement program | SHELXL-2014/6 (Sheldrick, 2014) | |
| Function minimized | ∑ *w*(*F*ₒ² - *F*_{c}²)² | |
| Data / restraints / parameters | 11461 / 1216 / 1061 | |
| Goodness-of-fit on *F*² | 0.991 | |
| Δ/σₘₐₓ | 0.001 | |
| Final R indices | 10519 data; I>2σ(I) | R1 = 0.0391, wR2 = 0.1042 |
| | all data | R1 = 0.0416, wR2 = 0.1063 |
| Weighting scheme | *w =* 1 / [σ²(*F*o²) + (0.0551P)²+ 0.9946P] where P = (*F*o²+2*F*c²) / 3 | |
| Extinction coefficient | 0.00131(12) | |
| Largest diff. peak and hole | 0.470 and -0.339 *e*Å⁻³ | |

### DSC

DSC data were collected on a TA Instruments Q2000 equipped with a 50 position auto-sampler. Typically, 0.5 - 3 mg of each sample, in a pin-holed aluminium pan, was heated at 10 °C/min from 25 °C to typically 260 °C. A purge of dry nitrogen at 50 ml/min was maintained over the sample. Modulated temperature DSC was carried out using an underlying heating rate of 2 °C/min and temperature modulation parameters of ±0.636 °C (amplitude) every 60 seconds (period). The full details used for mDSC are: 1) Equilibrate at -80.00°C; 2) Isothermal for 5.00 min; 3) Sampling interval 1.00 sec/pt; 4) Modulate ± 0.636°C every 60 seconds, and 5)Ramp 2.00°C/min to 260.00°C. The instrument control software was Advantage for Q Series and Thermal Advantage and the data were analysed using Universal Analysis or TRIOS.

### TGA

TGA data were collected on a TA Instruments Q500 TGA, equipped with a 16 position auto-sampler. Typically, 5 - 10 mg of each sample was loaded onto a pre-tared aluminium DSC pan and heated at 10 °C/min from ambient temperature to 350 °C. A nitrogen purge at 60 ml/min was maintained over the sample. The instrument control software was Advantage for Q Series and Thermal Advantage and the data were analysed using Universal Analysis or TRIOS.

### GVS

### 1. GVS with SMS DVS Intrinsic

Sorption isotherms were obtained using a SMS DVS Intrinsic moisture sorption analyser, controlled by DVS Intrinsic Control software. The sample temperature was maintained at 25 °C by the instrument controls. The humidity was controlled by mixing streams of dry and wet nitrogen, with a total flow rate of 200 ml/min. The relative humidity was measured by a calibrated Rotronic probe (dynamic range of 1.0 - 100 %RH), located near the sample. The weight change, (mass relaxation) of the sample as a function of %RH was constantly monitored by a microbalance (accuracy ±0.005 mg).

Typically, 5 - 30 mg of sample was placed in a tared mesh stainless steel basket under ambient conditions. The sample was loaded and unloaded at 40 %RH and 25 °C (typical room conditions). A moisture sorption isotherm was performed as outlined below (2 scans per complete cycle). The standard isotherm was performed at 25 °C at 10 %RH intervals over a 0 - 90 %RH range. Typically, a double cycle (4 scans) was carried out. Data analysis was carried out within Microsoft Excel using the DVS Analysis Suite. The method for SMS DVS Intrinsic experiments is shown below.

### Method Parameters for SMS DVS Intrinsic Experiments

| **Parameter** | **Value** |
|---|---|
| Adsorption - Scan 1 | 40 - 90 |
| Desorption, Adsorption - Scan 2 | 90 - 0, 0 - 40 |
| Intervals (%RH) | 10 |
| Number of Scans | 4 |
| Flow rate (ml/min) | 200 |
| Temperature (°C) | 25 |
| Stability (°C/min) | 0.2 |
| Sorption Time (hours) | 6 hour time out |
| Number of cycles | 2 |

### 2. Hiden IGASorp

Sorption isotherms were obtained using a Hiden IGASorp moisture sorption analyser, controlled by Isochema HISorp software. The sample temperature was maintained at 25 °C by a Grant LT ecocool 150 re-circulating water bath. The humidity was controlled by mixing streams of dry and wet nitrogen, with a total flow rate of 250 ml.min⁻¹. The relative humidity was measured by a calibrated Vaisala RH probe (dynamic range of 0 - 95 %RH), located near the sample. The weight change, (mass relaxation) of the sample as a function of %RH was constantly monitored by the microbalance (accuracy ±0.001 mg).

Typically, 20 - 30 mg of sample was placed in a tared mesh stainless steel basket under ambient conditions. The sample was loaded and unloaded at 40 %RH and 25 °C (typical room conditions). A moisture sorption isotherm was performed as outlined below (2 scans giving 1 complete cycle). The standard isotherm was performed at 25 °C at 10 %RH intervals over a 0 - 90 %RH range. Typically, a double cycle (4 scans) was carried out. Data analysis was carried out within the Isochema HISorp 2019 software and exported into Microsoft Excel to present accordingly.

### Method Parameters for Hiden IGASorp Experiments

| **Parameters** | **Value** |
|---|---|
| Adsorption - Scan 1 | 40 - 90 |
| Desorption / Adsorption - Scan 2 | 90 - 0, 0 - 40 |
| Intervals (%RH) | 10 |
| Number of Scans | 2 |
| Flow rate (ml/min) | 250 |
| Temperature (°C) | 25 |
| Stability (°C/min) | 0.05 |
| Minimum Sorption Time (mins) | 10 |
| Maximum Sorption Time (mins) | 360 |
| Equilibration Mode | Final Rate |
| Accuracy (tolerance) | +/- 0.001 mg/min (over 600 s) |

### HPLC

Purity analysis was performed on an Agilent HP1100/Infinity II 1260 series system equipped with a diode array detector and using OpenLAB software. The full method details are provided below.

### HPLC method for chemical purity determinations

| **Parameter** | **Value** | | |
|---|---|---|---|
| Type of method | Reverse phase with gradient elution | | |
| Sample Preparation | 0.25 mg/ml in acetonitrile : water 1:1 | | |
| Column | Supelco Ascentis Express C18 2.7 µm 100 × 4.6 mm | | |
| Column Temperature (°C) | 25 | | |
| Injection (µL) | 20 | | |
| Detection: Wavelength, Bandwidth (nm) | 255, 90 | | |
| Flow Rate (ml/min) | 2 | | |
| Phase A | 0.1% TFA in water | | |
| Phase B | 0.085% TFA in acetonitrile | | |
| Timetable | **Time (min)** | **% Phase A** | **% Phase B** |
| | 0 | 95 | 5 |
| | 6 | 5 | 95 |
| | 6.2 | 95 | 5 |
| | 8 | 95 | 5 |

A supplied method was successfully transferred onto an Infinity II 1260 Agilent system. A minor change was implemented in the method which was the addition of an extra minute of equilibration time at the end of the method. This additional equilibration time allows for the observation of the full gradient.

### Details on the HPLC supplied method

| **Parameter** | **Value** | | |
|---|---|---|---|
| Type of method | Reverse phase with gradient elution | | |
| Column | Supelco Ascentis, C18 2.7 µm 100 × 4.6 mm | | |
| Column Temperature (°C) | Ambient (Set to 25°C) | | |
| Injection volume (µL) | 20 | | |
| Detection: Wavelength, Bandwidth (nm) | 260,4 | | |
| | Ref: 360, 100 | | |
| Flow Rate (ml/min) | 1.0 | | |
| Phase A | 0.1% Formic acid in water | | |
| Phase B | 0.1% Formic Acid in acetonitrile | | |
| Timetable | **Time (min)** | **% Phase A** | **% Phase B** |
| | 0.0 | 70 | 30 |
| | 6.0 | 20 | 80 |
| | 7.0 | 20 | 80 |
| | 7.1 | 70 | 30 |
| | 9.0 | 70 | 30 |
| | 10.0* | 70 | 30 |

### NMR

¹H NMR spectra were collected on a Bruker 400 MHz instrument equipped with an auto-sampler and controlled by a Avance NEO nanobay console. Samples were prepared in DMSO-*d₆* solvent, unless otherwise stated. Automated experiments were acquired using ICON-NMR configuration within Topspin software, using standard Bruker-loaded experiments (¹H). Off-line analysis was performed using ACD Spectrus Processor.

### Static Stability Experiments

For short-term (8 days or fewer) stability experiments, solid material was placed into open vials at elevated storage conditions, unless otherwise stated. These conditions were achieved using saturated salt solutions at specific temperatures within sealed containers. Storage containers were pre-equilibrated prior to input of samples.

### Salt solutions used to produce static storage conditions

| **Condition** | **Saturated Salt Solution** | **Temperature (°C)** |
|---|---|---|
| 25 °C/97 %RH | Potassium sulphate | 25 |
| 40 °C/75 %RH | Sodium chloride | 40 |

For long-term (1 month or longer) stability experiments, solid material was placed into LDPE bags and sealed in a HDPE container at elevated storage conditions, unless otherwise stated.

### Example 1

### Synthesis of 5-(3,4-difluorobenzyl)-8-((1r,4r)-4-methylcyclohexyl)-6,9-dioxo-2,5,8-triazaspiro[3.5]nonane-2-carbaldehyde (Compound 1)

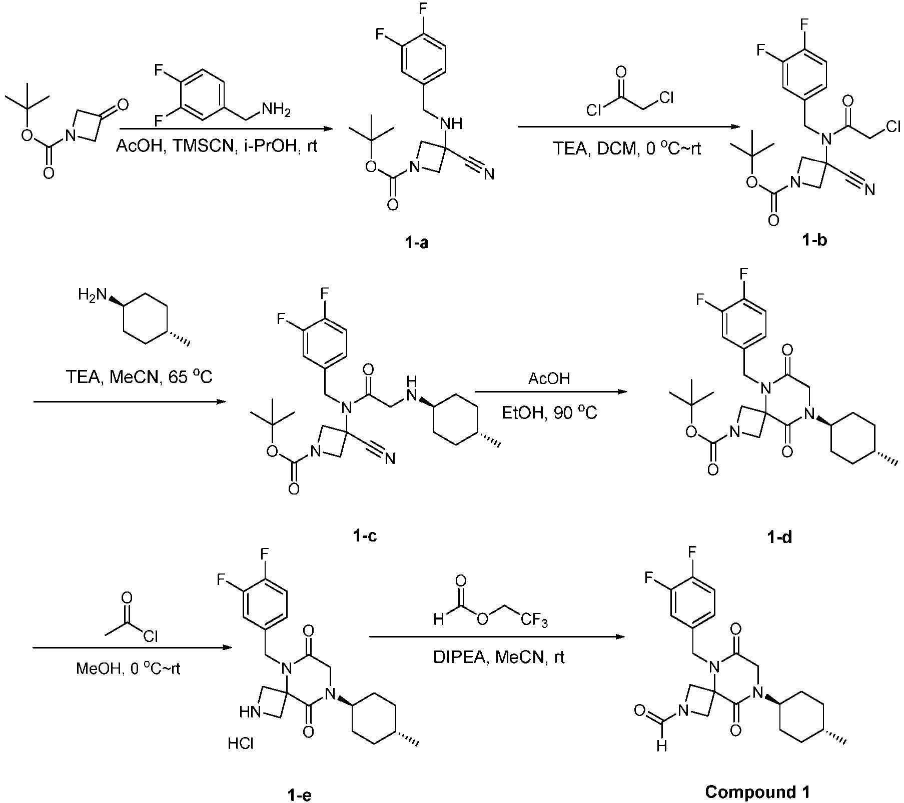

### Step 1: Synthesis of tert-butyl 3-cyano-3-{[(3,4-difluorophenyl)methyl]amino}azetidine-1-carboxylate (1-a):

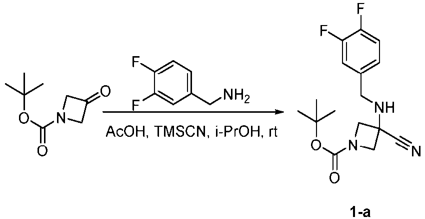

To a solution of tert-butyl 3-oxoazetidine-1-carboxylate (3.0 kg, 17523.774 mmol, 1 equiv) in i-PrOH (15.00 L) at r.t., acetic acid (0.74 kg, 12.27 mol, 0.7 equiv) and 1-(3,4-difluorophenyl)methanamine (3.01 kg, 21.03 mol, 1.2 equiv) were added. After stirred at r.t. for 1h, the resulting mixture was added trimethylsilyl cyanide (1.74 kg, 17.52 mol, 1.0 equiv). The resulting mixture was stirred overnight at r.t. The reaction was monitored by LCMS. The resulting mixture was concentrated under reduced pressure to remove 3/4 solvents, then filtered, washed with n-hexane (2 × 3 L), and dried to afford 3.6 kg (1^{st} batch) of **1-a.** The filtrate was concentrated, dissolved in i-PrOH (500 mL) and then filtered, washed with n-hexane (2 × 500 mL), and dried to afford 0.4 kg (2^{nd} batch) of **1-a.** Two batches were combined to afford 4 kg of *tert-butyl* 3-cyano-3-{[(3,4-difluorophenyl)methyl]amino}azetidine-1-carboxylate (yield:70.59%) as a white solid. LRMS (ES) m/z 268 [M+H-56]. ¹H NMR (300 MHz, Chloroform-d) δ 7.30 - 7.17 (m, 1H), 7.17 - 7.04 (m, 2H), 4.24 (d, J = 8.9 Hz, 2H), 3.92 - 3.83 (m, 2H), 3.80 (s, 2H), 2.20 (d, J = 55.9 Hz, 1H), 1.44 (s, 9H).

### Step 2: Synthesis of tert-butyl 3-{2-chloro-N-[(3,4-difluorophenyl)methyl]acetamido}-3-cyanoazetidine-1-carboxylate(1-b) :

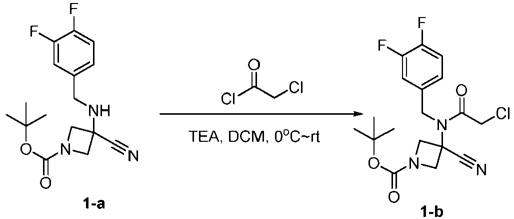

To a solution of *tert*-butyl 3-cyano-3-{[(3,4-difluorophenyl)methyl]amino}azetidine-1-carboxylate (3.0 kg, 9.28 mol, 1 equiv) in DCM (15 L) at 0 °C was added TEA (2.82 kg, 27.83 mol, 3.0 equiv). Then chloroacetyl chloride (2.62 kg, 23.20 mol, 2.5 equiv) in DCM (15 L) was added over a period of 2 h. The resulting mixture was stirred overnight at r.t.. The reaction was monitored by LCMS. The resulting mixture was washed with sat. NaHCO₃ (2 × 9 L) and brine (9 L), dried over anhydrous Na₂SO₄, concentrated under reduced pressure, purified by silica gel column chromatography, eluted with petroleum ether and EtOAc (2:1) to afford 3.0 kg of *tert-butyl* 3-{2-chloro-N-[(3,4-difluorophenyl)methyl]acetamido}-3-cyanoazetidine-1-carboxylate (yield: 81%) as a yellow solid. LRMS (ES) m/z 344 [M+H-56]. ¹H NMR (300 MHz, Chloroform-d) δ 7.26 (dt, J = 9.6, 8.3 Hz, 1H), 7.13 (ddd, J = 10.0, 7.1, 2.3 Hz, 1H), 7.08 - 6.99 (m, 1H), 4.67 (s, 2H), 4.33 (d, J = 9.8 Hz, 2H), 4.16 - 4.05 (m, 4H), 1.44 (s, 9H).

### Step 3: Synthesis of tert-butyl 3-cyano-3-{N-[(3,4-difluorophenyl)methyl]-2-{[(1r,4r)-4-methylcyclohexyl]amino}acetamido}azetidine-1-carboxylate (1-c):

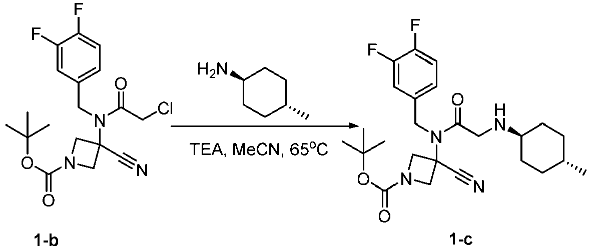

To a solution of tert-butyl 3-{2-chloro-N-[(3,4-difluorophenyl)methyl]acetamido}-3-cyanoazetidine-1-carboxylate (3.0 kg, 7.50 mol, 1 equiv) in MeCN (30 L) at r.t. were added (1r,4r)-4-methylcyclohexan-1-amine (0.93 kg, 8.25 mol, 1.1 equiv) and TEA (1.90 kg, 18.76 mol, 2.5 equiv). The resulting mixture was stirred overnight at 65 °C. Desired product could be monitored by LCMS. The resulting mixture was cooled to r.t, concentrated under reduced pressure. The resulting mixture was diluted with water (10 L), extracted with EtOAc (3 × 15 L). The combined organic layers were washed with brine (2 × 10 L), dried over anhydrous Na₂SO₄, concentrated under reduced pressure to afford 3.0 kg of *tert*-butyl 3-cyano-3-{N-[(3,4-difluorophenyl)methyl]-2-{[(1r,4r)-4-methylcyclohexyl]amino}acetamido}azetidine-1-carboxylate as a brown solid which was directly used in next step without further purification. LRMS (ES) m/z 421 [M+H-56]. ¹H NMR (300 MHz, Chloroform-d) δ 7.20 - 7.06 (m, 1H), 7.01 (ddd, J = 11.0, 7.4, 2.3 Hz, 1H), 6.96 - 6.85 (m, 1H), 4.89 (s, 2H), 4.48 (dd, J = 13.6, 9.4 Hz, 2H), 4.21 - 3.80 (m, 3H), 3.80 (s, 2H), 1.92 - 1.69 (m, 4H), 1.58 - 1.27 (m, 13H), 1.14 (qd, J = 12.7, 12.2, 3.2 Hz, 2H), 0.93 (d, J = 6.4 Hz, 3H).

### Step 4: Synthesis of tert-butyl 5-[(3,4-difluorophenyl)methyl]-6,9-dioxo-8-[(1r,4r)-4-methylcyclohexyl]-2,5,8-triazaspiro[3.5]nonane-2-carboxylate (1-d):

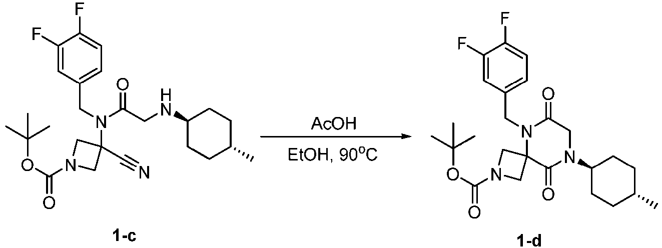

To a solution of *tert-butyl* 3-cyano-3-{N-[(3,4-difluorophenyl)methyl]-2-{[(1r,4r)-4-methylcyclohexyl]amino} acetamido}azetidine-1-carboxylate (3 kg, 6.30 mol, 1 equiv) in EtOH (30 L) at r.t. was added AcOH (7.56 kg, 125.90 mol, 20 equiv) dropwise over a period of 30 min. The resulting mixture was stirred overnight at 90 °C. The reaction was monitored by LCMS. The resulting mixture was cooled to r.t., concentrated under reduced pressure to remove 70% solvents, cooled down to 0 °C, stirred for 15 min, filtered, washed with ice cold ethanol (3 L), and dried to afford 2.4 kg of *tert-butyl* 5-[(3,4-difluorophenyl)methyl]-6,9-dioxo-8-[(1r,4r)-4-methylcyclohexyl]-2,5,8-triazaspiro[3.5]nonane-2-carboxylate (yield:75.44%) as a light yellow solid. LRMS (ES) m/z 422 [M+H-56]. ¹H NMR (300 MHz, Chloroform-d) δ 7.20 - 7.02 (m, 2H), 7.02 - 6.91 (m, 1H), 4.89 (s, 2H), 4.51 (d, J = 9.5 Hz, 2H), 4.40 (tt, J = 12.2, 3.8 Hz, 1H), 4.00 - 3.91 (m, 4H), 1.86 - 1.77 (m, 2H), 1.71 (dd, J = 12.1, 3.5 Hz, 2H), 1.52 (d, J = 12.4 Hz, 1H), 1.44 (s, 9H), 1.37 - 1.21 (m, 2H), 1.13 (qd, J = 12.6, 3.4 Hz, 2H), 0.92 (d, J = 6.4 Hz, 3H).

### Step 5: Synthesis of 5-[(3,4-difluorophenyl)methyl]-8-[(1r,4r)-4-methylcyclohexyl]-2,5,8-triazaspiro[3.5]nonane-6,9-dione hydrochloride (1-e):

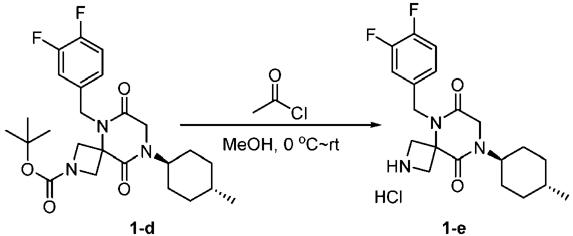

To a solution of tert-butyl 5-[(3,4-difluorophenyl)methyl]-6,9-dioxo-8-[(1r,4r)-4-methylcyclohexyl]-2,5,8-triazaspiro[3.5]nonane-2-carboxylate (3.4 kg, 7.12 mol, 1 equiv) in MeOH (34 L) at -5 °C was added acetyl chloride (1.68 kg, 21.36 mol, 3.0 equiv) dropwise over a period of 1.5 h. The resulting mixture was stirred overnight at r.t. The reaction was monitored by LCMS. The resulting mixture was concentrated under reduced pressure, purified by trituration with petroleum ether and EtOAc (10:1, 12 L) to afford 2.5 kg of 5-[(3,4-difluorophenyl)methyl]-8-[(1r,4r)-4-methylcyclohexyl]-2,5,8-triazaspiro[3.5]nonane-6,9-dione hydrochloride (yield:85%) as an off-white solid. LRMS (ES) m/z 378 [M+H]. ¹H NMR (300 MHz, DMSO-d6) δ 10.26 (s, 1H), 9.13 (s, 1H), 7.51 - 7.29 (m, 2H), 7.27 - 7.12 (m, 1H), 5.04 (s, 2H), 4.27 - 4.06 (m, 5H), 3.98 (s, 2H), 1.86 - 1.45 (m, 6H), 1.37 - 1.25 (m, 1H), 1.03 (tq, J = 12.1, 7.8, 6.0 Hz, 2H), 0.86 (d, *J* = 6.4 Hz, 3H).

### Step 6: Synthesis of 5-[(3,4-difluorophenyl)methyl]-6,9-dioxo-8-[(1r,4r)-4-methylcyclohexyl]-2,5,8-triazaspiro[3.5]nonane-2-carbaldehyde (Compound 1):

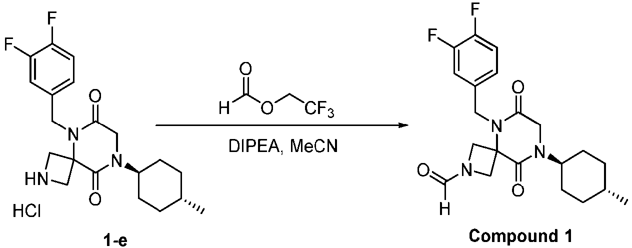

To a solution of 5-(3,4-difluorobenzyl)-8-((1r,4r)-4-methylcyclohexyl)-6,9-dioxo-2,5,8-triazaspiro[3.5]nonane-2-carbaldehyde (4.0 kg, 10.60 mol, 1 equiv) in MeCN (20 L) at r.t. were added 2,2,2-trifluoroethyl formate (1.63 kg, 12.72 mol, 1.2 equiv) and DIPEA (3.42 kg, 26.50 mol, 2.5 equiv) . The resulting mixture was stirred overnight at rt. The reaction was monitored by LCMS. The resulting mixture was concentrated under reduced pressure. The resulting mixture was diluted with EtOAc (10 L). The resulting mixture was quenched with NH₄Cl (6 L, sat.) and water (6 L), extracted with EtOAc (3x15 L). The combined organic layers were washed with NH₄Cl (aq.) (10 L) and brine (10 L), dried over anhydrous Na₂SO₄, concentrated under reduced pressure to give a crude brown oil, the crude oil was re-crystallized from cyclohexane and EtOAc (5:1, 4L, 80 °C to r.t.), filtered to afford 3 kg (1^{st} batch) 5-[(3,4-difluorophenyl)methyl]-6,9-dioxo-8-[(1r,4r)-4-methylcyclohexyl]-2,5,8-triazaspiro[3.5]nonane-2-carbaldehyde light yellow solid. The filtrate was concentrated under reduced pressure, re-crystallized with petroleum ether and EtOAc (10:1, 3 L, rt) to afford 800 g (2^{nd} batch) of light yellow solid. Two batches were combined, dried to afford 3.8 kg of Form I (m.p. at 133 °C) 5-(3,4-difluorobenzyl)-8-((1r,4r)-4-methylcyclohexyl)-6,9-dioxo-2,5,8-triazaspiro[3.5]nonane-2-carbaldehyde light yellow solid. The overall yield of this step is 97%.

### Crystalline Form I characterization

Crystalline Form I of 5-(3,4-difluorobenzyl)-8-((1r,4r)-4-methylcyclohexyl)-6,9-dioxo-2,5,8-triazaspiro[3.5]nonane-2-carbaldehyde was analyzed by XRPD, DSC, TGA, GVS, and HPLC. Table 1-A shows the angles 2-theta and relative peak intensities that were observed for Form I. FIG. 1A shows an XRPD pattern of Form I.

**Table 1-A**

| **Angle** / **2θ** | **Intensity** / **%** |
|---|---|
| 6.0 | 100.0 |
| 10.2 | 9.5 |
| 11.9 | 6.7 |
| 13.7 | 5.5 |
| 14.4 | 4.1 |
| 15.5 | 8.7 |
| 16.0 | 14.2 |
| 16.6 | 9.8 |
| 17.3 | 9.9 |
| 17.6 | 9.9 |
| 17.9 | 14.4 |
| 19.8 | 3.9 |
| 20.5 | 12.0 |
| 20.9 | 2.7 |
| 21.6 | 14.6 |
| 22.1 | 29.1 |
| 22.8 | 8.0 |
| 23.1 | 5.6 |
| 24.1 | 14.3 |
| 24.5 | 2.3 |
| 25.4 | 2.7 |
| 25.7 | 3.0 |
| 27.8 | 2.6 |
| 28.6 | 2.0 |
| 29.0 | 6.1 |
| 29.6 | 3.5 |
| 30.1 | 6.2 |

FIG. 1B shows DSC and TGA graphs of Form I. As shown in the DSC graph, an endotherm onset at about 125.6 °C and an endotherm peak at 130.2 °C were observed. As shown in the TGA graph, a weight loss of 0.4 % between 105 °C and 145 °C was observed. FIG. 1C shows a GVS graph of Form I.

The purity and stability of crystalline Form I were analyzed by XRPD and HPLC. Table 1-B shows the XRPD analysis results and the purity of crystalline Form I analyzed by HPLC before storage for 7 days at 25°C/97% RH or 7 days at 40°C/75% RH, after storage for 7 days at 25°C/97% RH, and after storage for 7 days at 40°C/75% RH. As shown in Table 1-B, Form I was 5-(3,4-difluorobenzyl)-8-((1r,4r)-4-methylcyclohexyl)-6,9-dioxo-2,5,8-triazaspiro[3.5]nonane-2-carbaldehyde with at least 96.3% purity and the purity remained substantially unchanged when stored for a period of 7 days under 40°C/75% RH and/or under 25°C/97% RH, as determined by HPLC.

**Table 1-B**

| | | |
|---|---|---|
| Before Storage | XRPD | Form I |
| | HPLC | 96.3% |
| Storage at 25 °C/ 97 %RH for 7 days | XRPD | Unchanged by XRPD |
| | HPLC | 96.6 % |
| Storage at 40 °C/ 75 %RH for 7 days | XRPD | Unchanged by XRPD |
| | HPLC | 96.4 % |

### Example 2

### Preparations of Form II

### Method 1: Conversion of Form I to Form II

2.5 g of crystalline Form I of 5-(3,4-difluorobenzyl)-8-((1r,4r)-4-methylcyclohexyl)-6,9-dioxo-2,5,8-triazaspiro[3.5]nonane-2-carbaldehyde was suspended in EtOH (5 vol, 12.5 ml). The sample was stirred at 50 °C (500 rpm). After 15 minutes, a solution was obtained. Some debris was identified that appeared to be a piece of plastic which was extracted prior to continuation.

The solution was stirred at 50 °C for a further 5 minutes and then cooled to 5 °C at 0.1 °C/minute. After stirring at 5 °C overnight, an aliquot of the suspension was filtered through a filter cartridge equipped with a frit. A stream of compressed air was gently blown over the solids. A portion of the mother liquor was placed for slow evaporation.

The bulk sample was filtered through a Buchner funnel. A small amount of the solids passed through the filter paper. The mother liquor was collected and re-filtered and added to the filter cake. The filter cake was dried under suction for 30 minutes. The solids were determined as Form II.

### Method 2

To 5-(3,4-difluorobenzyl)-8-((1r,4r)-4-methylcyclohexyl)-6,9-dioxo-2,5,8-triazaspiro[3.5]nonane-2-carbaldehyde (4.0 g 9.9 mmol) in a 100 mL round bottom flask with a magnetic stirring bar, was added ethanol (4.0 mL) and the mixture was heated to 78 °C with an oil bath. To this mixture, was added water (2 mL at a time for a total a 6 mL and dropwise after that to a total of 7.0 mL) until it turned slightly cloudy. The mixture was heated at 78 °C for 30 min before turning off the heat and the solution was slowly allowed to cool to room temperature with stirring (~300 rpm, revolutions per minute) and stirred overnight. The solid was collected by filtration and dried under the air to obtain 3.8 g (95%) of Form II 5-(3,4-difluorobenzyl)-8-((1r,4r)-4-methylcyclohexyl)-6,9-dioxo-2,5,8-triazaspiro[3.5]nonane-2-carbaldehyde as a white solid. LRMS (ES) m/z 406.2 (M+H). ¹H NMR (400 MHz, Methylene Chloride-*d*₂) δ 8.05 (s, 1H), 7.20 (q, *J* = 9.0 Hz, 1H), 7.11 (dd, *J =* 11.0, 8.0 Hz, 1H), 7.04-6.97 (m, 1H), 4.95 (d, *J* = 16.2 Hz, 1H), 4.81 (d, *J* = 16.1 Hz, 1H), 4.71 (d, *J* = 9.2 Hz, 1H), 4.50 (d, *J* = 10.7 Hz, 1H), 4.42 (td, *J* = 12.3, 6.2 Hz, 1H), 4.21 (d, *J* = 9.1 Hz, 1H), 4.10 (d, *J =* 10.7 Hz, 1H), 4.03 (s, 2H), 1.85 (d, *J =* 13.2 Hz, 2H), 1.74 (d, *J* = 9.8 Hz, 2H), 1.59 - 1.48 (m, 2H), 1.43 - 1.33 (m, 1H), 1.17 (qd, *J =* 12.7, 3.5 Hz, 2H), 0.95 (d, *J* = 6.3 Hz, 3H).

### Method 3

To 5-(3,4-difluorobenzyl)-8-((1r,4r)-4-methylcyclohexyl)-6,9-dioxo-2,5,8-triazaspiro[3.5]nonane-2-carbaldehyde (42.0 g 103.6 mmol) in a 250 mL round bottom flask with a magnetic stirring bar was added ethanol (40.0 mL) and the solution was heated to 78 °C with an oil bath. To this mixture was added water (10 mL at a time for a total of 70 mL) until it turned slightly cloudy. The mixture was heated at 78 °C for 30 min, 3.8 g of crystalline Form II material of 5-(3,4-difluorobenzyl)-8-((1r,4r)-4-methylcyclohexyl)-6,9-dioxo-2,5,8-triazaspiro[3.5]nonane-2-carbaldehyde (3.8 g, 9.4 mmol) was added, the heat was turned off and the solution allowed to slowly cool to room temperature with stirring (~300 rpm, revolutions per minute) and stirred overnight. The solid was collected by filtration and dried under the air to obtain 44.8 g (97%) of Form II 5-(3,4-difluorobenzyl)-8-((1r,4r)-4-methylcyclohexyl)-6,9-dioxo-2,5,8-triazaspiro[3.5]nonane-2-carbaldehyde as a white solid. LRMS (ES) m/z 406.2 (M+H). ¹H NMR (400 MHz, Methanol-*d*₄) δ 7.99 (s, 1H), 7.25 (dt, *J* = 11.3, 8.7 Hz, 2H), 7.11 (dd, *J* = 8.5, 4.2 Hz, 1H), 4.93 (s, 2H), 4.66 (d, *J* = 9.9 Hz, 1H), 4.42 (t, *J* = 9.7 Hz, 2H), 4.32 (tt, *J* = 12.1, 3.9 Hz, 1H), 4.15 (d, *J* = 11.1 Hz, 1H), 4.11 (s, 2H), 1.85 (d, *J* = 13.3 Hz, 2H), 1.81 - 1.71 (m, 2H), 1.71 - 1.56 (m, 2H), 1.51 - 1.35 (m, 1H), 1.14 (qd, *J* = 12.5, 3.4 Hz, 2H), 0.95 (d, *J =* 6.4 Hz, 3H).

### Method 4: Conversion from Form I to Form II

To crystalline Form I solid of 5-(3,4-difluorobenzyl)-8-((1r,4r)-4-methylcyclohexyl)-6,9-dioxo-2,5,8-triazaspiro[3.5]nonane-2-carbaldehyde (50.0 g 123.3 mmol) in a 250 mL round bottom flask with a magnetic stirring bar, ethanol (50.0 mL) was added and the solution was heated to 86 °C with an oil bath. To this mixture was added water (10 mL at a time for a total a 50 mL) until it turned slightly cloudy. The mixture was heated at 86 °C for 30 min, the heat was turned off and the mixture was seeded with small amounts of crystalline Form II of 5-(3,4-difluorobenzyl)-8-((1r,4r)-4-methylcyclohexyl)-6,9-dioxo-2,5,8-triazaspiro[3.5]nonane-2-carbaldehyde (100 mg) at about 50 °C, and the solution was slowly allowed to cool to room temperature with stirring (~300 rpm, revolutions per minute) and stirred overnight. The solid was collected by filtration and dried under the air to obtain 44.0 g of 5-(3,4-difluorobenzyl)-8-((1r,4r)-4-methylcyclohexyl)-6,9-dioxo-2,5,8-triazaspiro[3.5]nonane-2-carbaldehyde as a white solid. LRMS (ES) m/z 406.2 (M+H).

This process was repeated at a scale of 170 g, 80 g, and 100 g scale of Form I solid to produce 176 g, 74 g, and 99 g, respectively.

A 2 L three neck flask with mechanic stirring was charged with 383 g pulled from the lots above. To this mixture was added ethanol (380 mL) and water (100 mL) and the mixture was heated slowly with a heating mantle with internal temperature controller. The suspension turned into a homogeneous solution at 50 °C and additional water (200 mL, 100 mL at a time) was added. When the internal temperature reached to 65 °C, additional water (100 mL) was added. The mixture turned cloudy until it reached 80 °C. To this solution was added water (50 mL with 5 mL at a time). The mixture was then heated to 85 °C for 10 min and the heat was turned off to allow it to slowly cool to room temperature. When the mixture cooled to 70 °C, 20 mg of Form II crystalline solid was added. More solid was observed when the internal temperature reached to 40 °C and the internal temperature was maintained at 40-46 °C when more solid precipitated out of the solution. The solid was collected when the internal temperature reached to 28 °C by filtration. The mixture was dried under vacuum oven for 3 days without heating to obtain 373 g of off-white crystalline Form II. ¹H NMR (500 MHz, DMSO-*d*₆) δ 7.97 (s, 1H), 7.44 - 7.32 (m, 2H), 7.10 (dt, *J* = 7.4, 2.8 Hz, 1H), 4.83 (s, 2H), 4.51 (d, *J* = 9.6 Hz, 1H), 4.29 - 4.14 (m, 3H), 4.01 (s, 2H), 3.97 (d, *J =* 10.7 Hz, 1H), 1.77 - 1.70 (m, 2H), 1.65 - 1.51 (m, 4H), 1.34 (dtq, *J* = 14.2, 7.0, 3.4 Hz, 1H), 1.03 (qd, *J* = 12.6, 3.9 Hz, 2H), 0.87 (d, *J =* 6.5 Hz, 3H).

### Method 5: Conversion from Form I to Form II

2.05 kg of Form I of 5-(3,4-difluorobenzyl)-8-((1r,4r)-4-methylcyclohexyl)-6,9-dioxo-2,5,8-triazaspiro[3.5]nonane-2-carbaldehyde obtained in Step 6 of Example 1, in a 10 L round-bottom flask with stirred bar was added 2000 mL of ethanol and heated to reflux (inner temperature is 90 °C). After 5 min of stirring at reflux, to this mixture was added water (2000 mL in total: 400 mL at a time for 1600 mL, and then 40 mL at a time for the rest 400 mL; when the mixture is cloudy, one should wait until it turns to homogeneous again at 90 °C). The mixture was heated at 90 °C for 10 min before the heat was turned off to allow the temperature to gradually reach to 55 °C (precipitate was observed during the cooling phase). Then 400 mg (m.p. at 155 °C) of the crystalline seed of crystalline Form II was added at the cooling phase (~55 °C). The mixture was allowed to cool down to room temperature. The mixture was stirred continuously at rt for 30 min. Then it was filtered, dried by oven (below 50 °C) to afford the crystalline Form II of 5-[(3,4-difluorophenyl)methyl]-6,9-dioxo-8-[(1r,4r)-4-methylcyclohexyl]-2,5,8-triazaspiro[3.5]nonane-2-carbaldehyde (1979.4 g, 96.5%) (m.p. at 155.6 °C) as off white solid.

### Crystalline Form II characterization

Crystalline Form II of 5-(3,4-difluorobenzyl)-8-((1r,4r)-4-methylcyclohexyl)-6,9-dioxo-2,5,8-triazaspiro[3.5]nonane-2-carbaldehyde was analyzed by XRPD, DSC, TGA, GVS, and HPLC. Table 2-A shows the angles 2-theta and relative peak intensities that were observed for Form II using XRPD. FIG. 2A shows an XRPD pattern of Form II.

**Table 2-A**

| **Angle** / **2θ** | **Intensity** / **%** |
|---|---|
| 5.9 | 100.0 |
| 6.4 | 4.7 |
| 6.7 | 8.2 |
| 7.4 | 4.7 |
| 7.8 | 15.9 |
| 10.4 | 2.6 |
| 11.5 | 8.4 |
| 11.7 | 12.9 |
| 12.4 | 8.4 |
| 12.8 | 6.9 |
| 13.1 | 14.1 |
| 13.7 | 2.3 |
| 14.1 | 2.4 |
| 14.6 | 7.4 |
| 15.1 | 7.8 |
| 15.7 | 8.0 |
| 16.2 | 20.0 |
| 16.6 | 2.3 |
| 16.8 | 10.7 |
| 17.2 | 13.9 |
| 17.9 | 24.5 |
| 18.4 | 4.1 |
| 18.8 | 4.7 |
| 19.1 | 20.9 |
| 19.4 | 8.6 |
| 19.8 | 14.8 |
| 20.3 | 7.9 |
| 20.7 | 11.4 |
| 21.0 | 5.8 |
| 21.3 | 10.6 |
| 21.8 | 11.7 |
| 22.0 | 4.5 |
| 22.4 | 13.9 |
| 23.3 | 8.2 |
| 23.6 | 6.4 |
| 23.9 | 5.5 |
| 24.2 | 9.2 |
| 24.5 | 3.1 |
| 24.8 | 3.8 |
| 25.2 | 3.3 |
| 25.4 | 9.9 |
| 25.9 | 9.1 |
| 26.3 | 4.2 |
| 26.6 | 4.9 |
| 27.3 | 2.4 |
| 27.7 | 2.6 |
| 28.1 | 5.1 |
| 28.4 | 2.8 |
| 28.7 | 7.1 |
| 29.1 | 2.9 |
| 29.6 | 3.2 |
| 30.8 | 2.7 |

FIG. 2B shows DSC and TGA graphs of Form II. As shown in the DSC graph, an endotherm onset at about 154.9 °C and an endotherm peak at about 155.8 °C were observed. As shown in the TGA graph, less than 0.1% weight loss prior to degradation was observed. FIG. 2C shows a GVS graph of Form II.

The purity and stability of crystalline Form II were analyzed by XRPD and HPLC. Table 2-B shows the XRPD analysis results and the purity of crystalline Form II analyzed by HPLC before storage for 8 days at 25°C/97% RH or 8 days at 40°C/75% RH, after storage for 8 days at 25°C/97% RH, and after storage for 8 days at 40°C/75% RH. As shown in Table 2-B, Form II was 5-(3,4-difluorobenzyl)-8-((1r,4r)-4-methylcyclohexyl)-6,9-dioxo-2,5,8-triazaspiro[3.5]nonane-2-carbaldehyde with at least 98.0% purity and the purity remained substantially unchanged when stored for a period of 8 days under 40°C/75% RH and/or under 25°C/97% RH, as determined by HPLC.

**Table 2-B**

| | | |
|---|---|---|
| Before Storage | XRPD | Form II |
| | HPLC | 98.2 % |
| Storage at 25°C/ 97 %RH for 8 days | XRPD | Unchanged by XRPD |
| | HPLC | 98.1 % |
| Storage at 40°C/ 75 %RH for 8 days | XRPD | Unchanged by XRPD |
| | HPLC | 98.0 % |

Table 2-E shows the water content results, XRPD analysis results, assay results, and the purity of crystalline Form II analyzed by HPLC before storage or after storage for 1, 3, or 6 months at 25°C/60% RH or at 40°C/75% RH. As shown in Table 2-E, Form II was 5-(3,4-difluorobenzyl)-8-((1r,4r)-4-methylcyclohexyl)-6,9-dioxo-2,5,8-triazaspiro[3.5]nonane-2-carbaldehyde with at least 99% purity, and the purity remained substantially unchanged when stored for a period of 6 months under 25°C/60% RH or under 40°C/75% RH, as determined by HPLC.

**Table 2-E**

| Condition | Test | Initial | Stability Test Time (months) | | |
|---|---|---|---|---|---|
| | | | 1 | 3 | 6 |
| 25°C/60% RH | Water Content (% w/w) | 0.02 | 0.02 | 0.01 | 0.09 |
| | XRPD | Form II | N/A | N/A | Form II |
| | Assay (% w/w) | 99.1 | 99.0 | 98.8 | 99.2 |
| | Purity (%) | 99.1 | 99.1 | 99.1 | 99.1 |
| | Total Impurities | 0.91 | 0.92 | 0.91 | 0.93 |
| 40°C/75% RH | Water Content (% w/w) | 0.02 | 0.01 | 0.01 | 0.06 |
| | XRPD | Form II | N/A | N/A | Form II |
| | Assay (% w/w) | 99.1 | 98.6 | 97.9 | 99.5 |
| | Purity (%) | 99.1 | 99.1 | 99.1 | 99.1 |
| | Total Impurities | 0.91 | 0.92 | 0.90 | 0.91 |

Single crystals of Form II were obtained by evaporation of a 2-methoxyethanol solution of 5-(3,4-difluorobenzyl)-8-((1r,4r)-4-methylcyclohexyl)-6,9-dioxo-2,5,8-triazaspiro[3.5]nonane-2-carbaldehyde. A crystal of suitable size and quality for analysis by single crystal X-ray diffraction was isolated with approximate dimensions of 0.25 × 0.12 × 0.10 mm. The crystal structure of crystalline Form II was solved in the triclinic centrosymmetric space group P-1 and refined with a final R1 [I>2σ(I)] value of 3.91 %. Single crystal data of Form II is provided in Table 2-D.

**Table 2-D**

| | | | | | |
|---|---|---|---|---|---|
| Crystallization solvents | 2-Methoxyethanol | | | | |
| Crystallization method | Evaporation | | | | |
| Molecular formula | C₂₁H₂₅F₂N₃O₃ | | | | |
| Molecular weight | 405.44 | | | | |
| Crystal system | Triclinic | | | | |
| Space group | *P*-1 | *a* | 14.6622(3) Å | α | 68.271(2)° |
| | | *b* | 14.6944(3) Å | β | 72.639(2)° |
| | | *c* | 16.3761(2) Å | γ | 69.935(2)° |
| V | 3018.67(11) Å³ | | | | |
| Z | 6 | | | | |
| Density (calculated) | 1.338 Mg/m³ | | | | |
| Absorption coefficient, µ | 0.861 mm⁻¹ | | | | |
| Source, λ | 1.54184 Å | | | | |
| *F(000)* | 1284 | | | | |
| *T* | 100(2) K | | | | |
| Crystal | Colorless fragment, 0.250 x 0.120 x 0.100 mm | | | | |
| Wavelength | 1.54184 Å | | | | |

### Example 3

### Kinetic Solubility and Stability of Crystalline Form II in Solvents

The solubility of crystalline Form II was analyzed with 15 types of common solvents. Sufficient amount of Form II was suspended in 0.5 mL solvent for a maximum anticipated concentration of *Ca.* = 10-100 mg/ml of the free Form II. The resulting suspensions were then shaken in a platform shaker at 25 °C/ 750 rpm for 4 hours. After equilibration, the appearance was recorded, and the pH of the saturated solution was measured for aqueous samples only. Samples were then centrifuged for 5 minutes at 13.4 krpm before dilution with MeCN except for the samples that were initially diluted in n-heptane, which were then diluted in 2-propanol rather than MeCN.

All diluted samples were then analyzed by HPLC. Samples were further diluted based on appearance and the maximum anticipated concentration **(Error! Reference source not found.).** All diluted samples were injected at 10, 15 and 20 µL to obtain peak areas within the calibration curve as a diluted concentration of *Ca.* 0.1 mg/ml was trying to be achieved. The details for the dilution used for different solvents are shown in Table 3-A.

**Table 3-A. Form II Dilution Table**

| **Solvent** | **Dilution** |
|---|---|
| DI Water | ×100 |
| MeOH | ×1000 |
| EtOH | ×1000 |
| IPA | ×700 |
| 2-BuOH | ×700 |
| Acetone | ×1000 |
| MIBK | ×1000 |
| DMSO | ×1000 |
| MeCN | ×1000 |
| THF | ×1000 |
| 2-Me-THF | ×1000 |
| Isopropyl acetate | ×700 |
| Toluene | ×500 |
| n-Heptane | ×100 |
| Tert-Butylmetyl ether | ×100 |

Quantitation was measured by HPLC with reference to a standard solution of approximately 0.15 mg/ml in MeCN. Different volumes of the standard diluted and undiluted sample solutions were injected. The solubility was calculated using the peak areas determined by integration of the peak found at the same retention time as the principal peak in the standard injection. Standards prepared in isopropanol were found to be non-linear, however the peak response between standards dissolved in isopropanol versus MeCN were comparable and therefore the MeCN standards were used for quantification for all samples.

The results of the kinetic solubility of Form II after 4 hours in 15 different solvents are shown in Table 3-B.

**Table 3-B**

| **Weight (mg)** | **Media** | **Appearance** | **Final pH** | **Solubility (mg/ml)** | **Average Solubility (mg/ml)** |
|---|---|---|---|---|---|
| 5.3 | DI Water | Suspension with residual solid | 8.7 | 0.50 | *Ca.* 0.41² |
| 5.0 | | Suspension with residual solid | 8.7 | 0.31 | |
| 50.0 | MeOH | Clear Solution | N/A | >100.0 | >100.6³ |
| 50.3 | | Clear Solution | N/A | >100.6 | |
| 50.3 | EtOH | Opaque suspension | N/A | 25 | 24 |
| 50.6 | | Opaque suspension | N/A | 23 | |
| 35.5 | IPA | Opaque suspension | N/A | 9.8 | 9.8 |
| 35.3 | | Opaque suspension | N/A | 9.8 | |
| 35.6 | 2-BuOH | Opaque suspension | N/A | 11 | 11 |
| 35.3 | | Opaque suspension | N/A | 11 | |
| 50.7 | Acetone | Clear Solution | N/A | >101.4 | >101.4⁴ |
| 50.7 | | Clear Solution | N/A | >101.4 | |
| 50.4 | MIBK | Opaque suspension | N/A | 39 | 39 |
| 50.3 | | Opaque suspension | N/A | 39 | |
| 50.4 | DMSO | Clear Solution | N/A | >100.8 | >101.2⁴ |
| 50.6 | | Clear Solution | N/A | >101.2 | |
| 50.7 | MeCN | Clear Solution | N/A | >101.4 | >101.4⁴ |
| 50.3 | | Clear Solution | N/A | >100.6 | |
| 50.8 | THF | Clear Solution | N/A | >101.6 | >101.6⁴ |
| 50.6 | | Clear Solution | N/A | >101.2 | |
| 50.9 | 2-Me-THF | Opaque suspension | N/A | 24 | 24 |
| 50.9 | | Opaque suspension | N/A | 24 | |
| 35.1 | Isopropyl acetate | Opaque suspension | N/A | 22 | 22 |
| 35.5 | | Opaque suspension | N/A | 22 | |
| 25.4 | Toluene | Suspension | N/A | 9.5 | 9.6 |
| 25.4 | | Suspension | N/A | 9.6 | |
| 5.5 | Tert-Butylmethyl ether | Suspension | N/A | 1.12 | 1.12 |
| 5.5 | | Suspension | N/A | 1.12 | |

² Value quoted as an approximate only due to agreement between duplicates; ³ Samples at T=4 hours were a clear solution, therefore reported as a greater than value based upon the initial weight of the sample; ⁴ Samples at T=4 hours were a clear solution, therefore reported as a greater than value based upon the initial weight of the sample.

After injection of the samples to determine solubility, the diluted samples were stored at ambient conditions (25°C) for 24 hours and reinjected in the same order at the same injection volume.

The chromatograms were then overlayed and visual signs of degradation were noted, i.e., loss of compound 1 peak area or increase of other peaks in the chromatography. The instrumental details of HPLC used to determine the stability of Form II are summarized in Table 3-C. The observations of the stability of Form II in a variety of solvents after T = 24 hours are summarized in Table 3-D.

**Table 3-C**

| **Parameter** | **Value** | | | |
|---|---|---|---|---|
| Type of method | Reverse phase with gradient elution | | | |
| Assay type: | Purity | | | |
| Column: | Supelco, Ascentis Express C18, 2.7µm, 100 x 4.6 mm | | | |
| Column Temperature (°C): | 25 | Autosampler temperature (°C) | | Ambient (25) |
| Standard Injections (µl): | 1, 2, 3, 4, 5, 7 | | | |
| Sample Injections (µl): | 1, 2, 3, 10, 15, 20 | | | |
| Detection: | 260, 4 | | | |
| Wavelength & Bandwidth (nm): | | | | |
| Flow Rate (ml/min): | 1.0 | | | |
| Mobile Phase A: | 0.1% Formic Acid in Water | | | |
| Mobile Phase B: | 0.1% Formic Acid in Acetonitrile | | | |
| Timetable: | Time (min) | | % Phase A | % Phase B |
| | 0.0 | | 70 | 30 |
| | 6.0 | | 20 | 80 |
| | 7.0 | | 20 | 80 |
| | 7.1 | | 70 | 30 |
| | 9.0 | | 70 | 30 |
| | 10.0 | | 70 | 30 |

**Table 3-D**

| **Solvent** | **Form II Peak Area** | **Form II Peak Area T= 24 hours** | **Visual Observations** |
|---|---|---|---|
| DI Water | 10.8 | 10.8 | No sign of degradation |
| MeOH | 199.0 | 202.1 | No sign of degradation |
| EtOH | 53.1 | 54.7 | Late eluting impurity peak, present at both timepoints, does not visually increase. |
| IPA | 30.3 | 30.5 | No sign of degradation |
| 2-BuOH | 35.3 | 35.2 | No sign of degradation |
| Acetone | 166.0 | 167.7 | No sign of degradation |
| MIBK | 85.1 | 85.1 | No sign of degradation |
| DMSO | 193.2 | 194.2 | No sign of degradation |
| MeCN | 188.0 | 190.4 | No sign of degradation |
| THF | 177.3 | 177.4 | No sign of degradation |
| 2-Me-THF | 51.7 | 51.5 | No sign of degradation |
| Isopropyl acetate | 66.6 | 66.3 | Impurities eluting after compound 1 peak, present at both timepoints, do not visually increase. |
| Toluene | 41.2 | 40.5 | No sign of degradation |
| Tert-Butylmetyl ether | 24.2 | 24.7 | Large impurity peak eluting after compound 1 peak, present at both timepoints, does not visually increase. |

The analysis above confirmed high solubility (>100 mg/ml) of crystalline Form II in methanol, acetone, DMSO, acetonitrile and THF. Additionally, when these samples were reinjected at T = 24 hours, no visual signs of further degradation were observed in the chromatography. The sample in ethanol displayed a solubility of *Ca.* 24 mg/mL. The replicates were less repeatable than most of the other preparations but did display a late eluting peak at both timepoints, although this sample did not appear to degrade further of the 24 hours.

Apart from the sample prepared in ethanol and tert-butyl methyl ether, only the sample prepared in isopropyl acetate showed any sign of degradation and as in the other two examples, this was seen in the initial injection and did not seem to degrade further during the 24 hours. The solubility in isopropyl acetate was calculated at 22 mg/mL.

### Example 4

### Preparation and Analysis of Form III

30 mg of amorphous 5-(3,4-difluorobenzyl)-8-((1r,4r)-4-methylcyclohexyl)-6,9-dioxo-2,5,8-triazaspiro[3.5]nonane-2-carbaldehyde was treated with 3 vol (90 µL) of 1,4-dioxane:H₂O (v/v 1:1) and the mixture was stirred at 5 °C (300 rpm). After a total of 7 days, the mixture was removed from stirring. An aliquot of the resulting suspension was removed with a spatula and blot dried with filter paper prior to XRPD analysis to give Form III.

Table 4-A shows the angles 2-theta and relative peak intensities that were observed for Form III using XRPD. FIG. 3A shows an XRPD pattern observed for Form III.

**Table 4-A**

| **Angle** / **2θ** | **Intensity** / **%** |
|---|---|
| 5.6 | 60.3 |
| 6.0 | 3.3 |
| 7.2 | 51.4 |
| 11.2 | 46.8 |
| 13.9 | 2.7 |
| 14.9 | 54.8 |
| 15.5 | 3.4 |
| 15.8 | 3.3 |
| 16.4 | 45.8 |
| 16.8 | 100 |
| 17.0 | 3.6 |
| 17.7 | 14.6 |
| 18.7 | 20.4 |
| 18.9 | 4.4 |
| 19.5 | 4.9 |
| 20.1 | 10.7 |
| 20.6 | 12.7 |
| 21.0 | 11.2 |
| 21.4 | 3.8 |
| 21.6 | 14.4 |
| 21.9 | 27.3 |
| 22.5 | 30 |
| 23.2 | 3.4 |
| 23.4 | 12.5 |
| 23.8 | 8.2 |
| 24.3 | 2.2 |
| 24.6 | 8.7 |
| 25.1 | 10.4 |
| 26.2 | 1.5 |
| 26.6 | 4.1 |
| 26.9 | 9.9 |
| 27.3 | 1.8 |
| 27.7 | 21.9 |
| 27.9 | 3.5 |
| 29.0 | 6.6 |
| 29.2 | 8.9 |
| 29.6 | 6.1 |

The crystalline Form III was isolated and subjected to baseline characterization to understand the nature of the solid. Crystalline Form III was assigned as an unstable, hemi-dioxane solvate that converted to crystalline Form II after storage at 40°C/75% RH conditions for 7 days, as determined by XRPD and shown in FIG. 3C. Crystalline Form III was analyzed by ¹HNMR. As determined by ¹HNMR, crystalline Form III was 5-(3,4-difluorobenzyl)-8-((1r,4r)-4-methylcyclohexyl)-6,9-dioxo-2,5,8-triazaspiro[3.5]nonane-2-carbaldehyde with 0.5 mol equiv. of 1,4-dioxane.

FIG. 3B shows DSC and TGA graphs of Form III. As shown in the DSC graph, an endotherm onset at about 81.7 °C, an endotherm onset with two events at about 112.8 °C, and an endotherm onset at about 154.4 °C were observed. In addition, an endotherm peak at about 92.1 °C, an endotherm peak at about 118.2 °C, an endotherm peak at about 130.0 °C, and an endotherm peak at about 155.8 °C were observed in DSC graph. As shown in the TGA graph, a weight loss of about 3.3 % was observed.

### Example 5

### Polymorph Screening

### 5-1 Polymorph screening with Isothermal Maturation at 5 °C

30 mg of amorphous 5-(3,4-difluorobenzyl)-8-((1r,4r)-4-methylcyclohexyl)-6,9-dioxo-2,5,8-triazaspiro[3.5]nonane-2-carbaldehyde was treated with 3 vol (90 µL) of the selected solvent and stirred at 5 °C (300 rpm). After about 1.5 hours, if the resulting suspension was too thick, additional solvent (20 µL) was added to the suspension. After stirring at 5 °C overnight, aliquots of suspensions were removed with a spatula and blot dried with filter paper prior to XRPD analysis. If the resulting suspension was too thin or close to a solution, anti-solvent (3 vol, 90 µL) was added to these solutions, and then these samples were returned to stirring a 5 °C. After stirring at 5 °C overnight, the samples that formed suspensions after anti-solvent addition were analyzed by XRPD. Where solutions persisted, anti-solvent (6 vol, 180 µL) was added. Samples were returned to stirring at 5 °C. After a total of 7 days, the samples were removed from stirring. Aliquots of suspensions were removed with a spatula and blot dried with filter paper prior to XRPD analysis. Samples generated were analyzed by XRPD. The results are provided below in Table 5-A and Table 5-B.

**Table 5-A**

| **Solvent** | **Obs. After stirring overnight at 5 °C** | **XRPD after stirring overnight at 5 °C** | **Obs. after stirring for 7 days at 5 °C** | **XRPD after stirring for 7 days at 5 °C** |
|---|---|---|---|---|
| n-Heptane | Suspension | Form II with an amorphous halo | White suspension | Form II |
| Diethyl ether | Suspension with brittle solids | Form II | White suspension | Form II |
| Propyl acetate | Suspension | Form II | White suspension | Form II |
| Ethyl acetate | Suspension | Form I | White suspension with yellow solid on top of the vial | Form II |
| Isopropyl acetate | Suspension | Form II | White suspension with yellow solid on top of the vial | Form II |
| MIBK | Suspension | Form I | White suspension | Form II |
| 2-Propanol | Suspension | Form I | White suspension | Form II |
| MEK | Suspension | Form II | | |
| 1-Propanol | Suspension | Form I | White suspension | Form I |
| Acetone | Suspension | Form II | Solution with white/ yellow solids on side of vial | N/A |
| Ethanol | Suspension | Form I | White suspension | Form II |
| DMSO:H₂O 1:1 | Suspension | Form I with an amorphous halo | | |
| Water | Gum | N/A | Brittle solids in suspension | Amorphous |
| TBME | Suspension | Form II | White suspension | Form II |
| 2-Methyl-1-propanol | Suspension | Form II | White suspension | Form II |
| Cyclohexane:he ptane 1:1 | Suspension | Form II | White suspension | Form II |
| 1,4-Dioxane:H₂O 1:1 | Suspension | Form I | Thick white paste | Form III |
| Toluene | Suspension | Form II | White suspension emulsion like | Form II |
| 1,2-Dimethoxyethane | Suspension | Form II | White suspension | Form II |
| Tetrahydrofuran | Suspension | Form II | White suspension | Form II |
| 2-Methoxyethanol | Suspension | Form II | White suspension | Form II |
| Methanol | Suspension | Form II | White suspension | Form II |
| Acetonitrile | Emulsion | N/A | Solution with white/ yellow solids on side of vial | N/A |
| Ethyleneglycol | Gum | N/A | Thick white paste | Form I |
| IPA:Water (5%) | Suspension | Form II | White suspension | Form II |
| Acetone: Water (5%) | Emulsion | N/A | Emulsion. Solution at RT | N/A |

**Table 5-B**

| **Solvent** | **Anti-solvent** | **Obs. post anti-solvent addition (1:1)** | **Obs. post stirring at 5 °C** | **Obs. after further anti-solvent addition (1:3)** | **XRPD after adding anti-solvent** | **Obs. after a total of 7 days at 5 °C** | **XRPD after stirring for 7 days at 5 °C** |
|---|---|---|---|---|---|---|---|
| Chloroform | Heptane | Hazy solution followed by solution | Solution | Hazy solution | N/A | | |
| Dichloromethane | Heptane | | Solution | Hazy solution | N/A | White suspensio n | Form II |
| N,N-Dimethylformamide | Water | Suspension | Suspension | N/A | Form I | White suspensio n | Form I |
| Nitrometha ne | Diethyl ether | Solution | Solution | Solution | N/A | Emulsion with solid on side. Solution at RT | N/A |
| N-Methylpyrrolidone | Water | Suspension | Gum | N/A | N/A | | |
| THF:Water (5%) | Water | 2 layers | Hazy solution with material on stirred bar. 2 layers obtained at RT | N/A | N/A | Thin suspensio n | Form II |

### 5-2 Polymorph screening with Temperature Cycling Maturation (RT/50 °C) Screen

30 mg of amorphous 5-(3,4-difluorobenzyl)-8-((1r,4r)-4-methylcyclohexyl)-6,9-dioxo-2,5,8-triazaspiro[3.5]nonane-2-carbaldehyde was treated with 3 vol (90 µL) of the selected solvent and placed for temperature cycling maturation (RT/50 °C, 4 hours per cycle) using a platform shaker incubator. After maturing overnight, aliquots of suspensions were removed with a spatula and blot dried with filter paper prior to XRPD analysis.

Where biphasic layers were observed, the samples were sonicated for 1.5 hours. If the samples remained unchanged, the samples were placed for evaporation. Post evaporation, solids were analysed by XRPD. Gums were treated with cyclohexane (10 vol, 300 µL) and sonicated for 4 hours. Aliquots of suspensions were removed with a spatula and blot dried with filter paper prior to XRPD analysis.

Suspensions obtained were analysed by XRPD. Aliquots of the suspensions were removed with a spatula and blot dried with filter paper prior to analysis.

When solutions are formed, anti-solvent (45 µL) was added to the solutions, which then were returned to temperature cycling maturation (RT/50 °C, 4 hours per cycle). Samples that were suspensions were also returned to temperature cycling maturation. After a total of 7 days, the samples were removed from maturation. Aliquots of suspensions were removed with a spatula and blot dried with filter paper prior to XRPD analysis.

The results of the temperature cycling maturation (RT/50 °C) screen are provided in Table 5-C. Summary of the results from further treatment of solutions is provided in Table 5-D. Summary of additional treatment to biphasic systems is provided Table 5-E.

**Table 5-C**

| **Solvent:anti-solvent v/v** | **Obs. after 3 vol** | **Obs. After stirring overnight** | **XRPD after stirring overnight** | **Obs. after stirring for 7 days** | **XRPD after stirring for 8 days** |
|---|---|---|---|---|---|
| n-Heptane | Thick suspension | Brittle solids (suspension) | Amorphous with a few peaks | | |
| Diethyl ether: heptane 1:1 | Gum/ partial dissolution | Gum | N/A | Suspension | Mixture of Form I & Form II |
| Propyl acetate: heptane 1:1 | Solution with solid on side of vial | Brittle solids (suspension) | Form II | Suspension | Form 2 with extra peak at 5.3 ° 2θ |
| Ethyl acetate: heptane 1:1 | Solution with solid on side of vial | Suspension | Form II | Suspension | Form II |
| Isopropyl acetate: heptane 1:1 | Solution with solid on side of vial with some gumming | Suspension | Form II | Suspension | Form II |
| MIBK: heptane 1:1 | Solution with solid on side of vial | Suspension | Form II | Suspension | Form II |
| 2-Propanol: H₂O 1:1 | Gum/ partial dissolution | Gum followed by brittle solids | Form II | Suspension | Form II with extra peak at 5.6, 6.2 & 7.0 ° 2θ* |
| MEK: heptane 1:1 | Solution with solid on side of vial | Brittle solids (suspension) | Form II with extra peaks | Suspension | Form II |
| 1-Propanol: H₂O 1:1 | 2 layers | 2 layers | N/A. See Table 5-E | | |
| Acetone: H₂O 1:1 | 2 layers | 2 layers | N/A. See Table 5-E | | |
| Ethanol: H₂O 1:1 | 2 layers | Suspension | Form II with extra peaks | Suspension | Form II with extra peak at 5.5 & 6.5° 2θ* |
| DMSO:H₂O 1:1 | Thick suspension | Brittle solids (suspension) | Form I | Suspension | Form I |
| Water | Thick suspension | Gum | N/A | Suspension | Form I |
| tert-Butylmethyl ether: heptane 1:1 | Thick suspension | Suspension | Form I | Suspension | Mixture of Form I & Form II |
| 2-Methyl-1-propanol: heptane 1:1 | Solution | Suspension | Form II | Suspension | Form II |
| Cyclohexane | Thick suspension | Brittle solids (suspension) | Form II with an amorphous halo | Suspension | Form II |
| 1,4-Dioxane:H₂O 1:1 | 2 layers | 2 layers | N/A. See Table 5-E | | |
| Toluene: heptane 1:1 | Gum/ partial dissolution | Brittle solids (suspension) | Form I | Suspension | Form I |
| Chloroform: heptane 1:1 | Solution with solid on side of vial | Solution | N/A. See Table 5-D | | |
| *N,N-*Dimethylformamide: H₂O 1:1 | Gum/ partial dissolution | Suspension | Form I | Suspension | Form II |
| Ethyleneglycol | Thick solution followed by gum/ partial dissolution | Brittle solids (suspension) | Form I with an amorphous halo | Suspension | Form I with low intensity peaks |
| Nitromethane: diethyl ether | Solution with solid on side of vial | Solution | N/A. See Table 5-D | | |
| *N-*Methylpyrrolidone: H₂O 1:1 | Gum/ partial dissolution | Brittle solids (suspension) | Form I with an amorphous halo | Suspension | Form II |
| THF: heptane 1:1 | Solution with solid on side of vial | Suspension | Form II | Suspension | Form II |
| IPA: heptane 1:1 | Solution with gum on side | Suspension | Form II | Suspension | Form II |
| Acetone: heptane 1:1 | Solution with solid on side of vial | Suspension | Form IIwith extra peaks | Suspension | Form II |

| | | | | | |
|---|---|---|---|---|---|
| *These samples were later confirmed to be Form II using high-resolution reflectance geometry XRPD and DSC. | | | | | |

**Table 5-D**

| **Solvent** | **Anti-solvent** | **New Solvent Ratio** | **Obs.** | **Obs. after stirring for 7 days** | **XRPD after stirring for 7 days** |
|---|---|---|---|---|---|
| Chloroform : heptane 1:1 | heptane | Chloroform: heptane 1:3 | Hazy solution with gumming | Suspension | Form II |

**Table 5-E**

| **Solvent** | **Obs. post sonication** | **Treatment** | **Obs.** | **Further treatment** | **XRPD after treatment** |
|---|---|---|---|---|---|
| 1-Propanol: H₂O 1:1 | 2 layers | Evaporation | Gum | Sonication with cyclohexane | Form I |
| 1,4-Dioxane:H₂O 1:1 | 2 layers | Evaporation | White solids. Crystallisation at oil interface | N/A | Mixture of Form I & Form II |

| | | | | | |
|---|---|---|---|---|---|
| *After storage in sealed vial gum was no longer present. | | | | | |

### 5-3 Polymorph screening with High Temperature Slurries (75 °C) Screen

30 mg of amorphous 5-(3,4-difluorobenzyl)-8-((1r,4r)-4-methylcyclohexyl)-6,9-dioxo-2,5,8-triazaspiro[3.5]nonane-2-carbaldehyde was treated with 3 vol (90 µL) of the selected solvent mixture and placed for stirring at 75 °C (500 rpm). After about 5 hours, observations were recorded. Additional solvent was added to all samples.

After stirring overnight at 75 °C, aliquots of suspensions were removed with a spatula and blot dried with filter paper prior to XRPD analysis. Where biphasic solutions remained, additional solvent was added in 150 µL aliquots upon stirring at 75 °C until a solution was obtained (total of 300 µL was added). Solutions were cooled to 5 °C at 0.1 °C/ min. Solutions were stirred at 5 °C. After a total of 15 or 18 days, suspensions obtained were analysed by XRPD. Solutions and biphasic solutions were stirred at RT.

The observations and XRPD analysis are summarized in Table 5-F.

**Table 5-F**

| **Solvent** | **Initial obs.** | **Obs. after a couple of hours** | **Additional treatment** | **Obs.** | **Obs. After stirring overnight** | **Further treatment** | **Obs. after stirring at 5 °C overnight** | **XRPD** |
|---|---|---|---|---|---|---|---|---|
| 50 % v/v water in MeOH | Gum | Biphasic solution | Additional solvent (210 µL) | Biphasic solution | Suspension | N/A | N/A | Form II |
| 85 % v/v water in MeOH | Thick solution. Gum at 75 °C | Solution with material on stirrer bar | Solids broken up and additional solvent (60 µL) | Suspension | Some solids in solution. Solid crown above solvent line | N/A | N/A | Form II |
| 50 % v/v water in EtOH | Gum | Biphasic solution | Additional solvent (210 µL) | Solution | Solution | Slow cooling | Gum | Form II*** |
| 85 % v/v water in EtOH | Thick suspension | Solution with material on stirrer bar | Solids broken up and additional solvent (60 µL) | Suspension | Some solids in solution. Solid crown above solvent line | N/A | N/A | Form II |
| 50 % v/v water in 1-PrOH | Hazy solution with solid on side of vial | Biphasic solution | Additional solvent (210 µL) | Solution with small colorless solid (glass like) | Solution with small colorless material (glass like) | Slow cooling | Solution | Form II**** |
| 85 % v/v water in 1-PrOH | Gum | Solution with material on stirrer bar | Solids broken up and additional solvent (60 µL) | Suspension | Solids stuck to stirrer bar. Solid crown above solvent line | N/A | N/A | Form II |
| 50 % v/v water in 2-PrOH | Hazy solution with solid on side of vial | Biphasic solution | Additional solvent (210 µL) | Solution | Solution | Slow cooling | Hazy solution/ emulsion | Form II*** |
| 85 % v/v water in 2-PrOH | Thick suspension with gum | Solution with material on stirrer bar | Solids broken up and additional solvent (60 µL) | Suspension | Some solids in solution. Solid crown above solvent line | N/A | N/A | Form II |
| 80 % v/v water in 2-BuOH | Gum with material on side of vial | Solution with material on stirrer bar | Solids broken up and additional solvent (60 µL) | Suspension | Solids stuck to stirrer not mobile | N/A | N/A | Form II |
| 50 % v/v water in acetone | Hazy solution with solid on side of vial | Biphasic solution w ith spec of silver material | Additional solvent (210 µL) | Biphasic solution | Biphasic solution | Additional solvent (300 µL) followed by slow cooling | Gum | N/A |
| Heptane | Thick suspension followed by gum* | Material stuck on side of vial | Additional solvent (150 µL) | Suspension | Suspension | N/A | N/A | Form II |
| Cyclohexane | Thick suspension followed by gum** | | Additional solvent (150 µL) | Suspension | Suspension | N/A | N/A | Form II |

| | | | | | | | | |
|---|---|---|---|---|---|---|---|---|
| Key: *gum after additional 30 µL solvent is added; **gum obtained before additional 30 µL solvent is added; *** Suspension obtained after 15 days, **** Suspension obtained after 18 days | | | | | | | | |

### 5-4 Polymorph screening with Liquid Assisted Grinding (LAG)

30 mg of amorphous 5-(3,4-difluorobenzyl)-8-((1r,4r)-4-methylcyclohexyl)-6,9-dioxo-2,5,8-triazaspiro[3.5]nonane-2-carbaldehyde was wetted with solvent (5 µL). Two grinding beads (3 mm diameter) were added and the samples were ground for 2 hours at 500 rpm using a planetary Fritsch Mill (Pulverisette 6). After grinding all solids were analysed by XRPD.

Gums were stored at ambient conditions. Gums and samples containing brittle material were dried *in-vacuo* at R. T. in a vacuum oven overnight (ca. 200 mbar). Observations were recorded and the samples were further dried *in-vacuo* at R. T. in a vacuum oven overnight (ca. 5 mbar). Where gums still persisted, the samples were further dried *in-vacuo* at R. T. in a vacuum oven (ca. 5 mbar) for 3 days. The observations and XRPD analysis are provided in Table 5-G. Further treatment details of gums and brittle materials are summarized in Table 5-H.

**Table 5-G**

| **Solvent** | **Procedure** | **Obs. pre grinding** | **Obs. post grinding** | **XRPD** |
|---|---|---|---|---|
| MeOH **/** water **97:3** v/v | | Gum | White material | Form II |
| 15 % v/v water in MeOH | | Gum | White material | Form II |
| 50 % v/v water in MeOH | | Gum with solids | White material | Mixture of Form I & Form II |
| 85 % v/v water in MeOH | | Solids | Gum | N/A |
| EtOH / water 97:3 v/v | | Solids | Gum | N/A |
| 15 % v/v water in EtOH | | Gum with solids | White material | Form II |
| 50 % v/v water in EtOH | | Gum with solids | White material | Form I |
| 85 % v/v water in EtOH | LAG | Solids | Gum | N/A |
| 1-PrOH / water 98:2 v/v | | Gum with solids | White material | Form I |
| 15 % v/v water in 1-PrOH | | Gum with solids | White material | Form II |
| 50 % v/v water in 1-PrOH | | Solids | White material | Form II |
| 85 % v/v water in 1-PrOH | | Solids | Gum | N/A |
| IPA /water 98:2 v/v | | Gum with solids | Gum | N/A |
| 15 % v/v water in 2-PrOH | | Gum with solids | White material | Form II |
| 50 % v/v water in 2-PrOH | | Gum with solids | Gum | N/A |
| 85 % v/v water in 2-PrOH | | Solids | White material | Form I |
| 2-BuOH / water 98:2 v/v | | Gum with solids | White material | Mixture of Form I & Form II |
| 80 % v/v water in 2-BuOH | | Gum with solids | White material | Form I |
| 95 % v/v water in 2-BuOH | | Solids | White material | Form I |
| Acetone /water 99:1 v/v | | Gum | White material | Form II |
| 15 % v/v water in acetone | LAG | Gum | White material | Form I |
| 50 % v/v water in acetone | | Solids | White material | Amorphous |
| 85 % v/v water in acetone | | Solids | White material | Amorphous |
| 15 % v/v water in DMSO | | Solids | White material | Form I |
| 50 % v/v water in DMSO | | Solids | White material | Form I |
| 85 % v/v water in DMSO | | Solids | Gum | N/A |
| 1,4-dioxane/ water 99:1 v/v | | Gum with solids | White material | Form II |
| 1,4-dioxane/water 1:1 v/v | | Solids | White material | Mixture of Form I & Form II |
| THF / water 99:1 v/v | | Solids | White material | Form II with amorphous halo |
| THF / water 1:1 v/v | | Solids | Gum | N/A |
| Acetonitrile / water 97 v/v:3 | | Solids | Gum | N/A |
| Acetonitrile / water 1:1 v/v | | Gum with solids | White material | Form II |

**Table 5-H**

| **Solvent** | **Obs.** | **Treatment** | **Obs.** | **Treatment** | **Obs.** | **XRPD** |
|---|---|---|---|---|---|---|
| 85 % v/v water in MeOH | Brittle material | Drying *in-vacuo* | Brittle material | Drying *in-vacuo* | Solid material | Mixture of Form I & Form II |
| EtOH / water 97:3 v/v | Brittle material with discolouration | | Brittle material with discolouration | | Solid material | Form II |
| 85 % v/v water in EtOH | Brittle material with discolouration | | Brittle material with discolouration | | Solid material | Mixture of Form I & Form II |
| 85 % v/v water in 1-PrOH | Gum with solids | | Gum with solids | | Solid material | Form I with an amorphous halo |
| IPA /water 98:2 v/v | Brittle material | | Brittle material | | Solid material | Mixture of Form I & Form II |
| 50 % v/v water in 2-PrOH | Brittle material with discolouration | | Brittle material with discolouration | | Solid material | Mixture of Form I & Form II |
| 85 % v/v water in DMSO | Gum with solids | | Gum with solids | | Solid material | Mixture of Form I & Form II |
| THF / water 1:1 v/v | Gum | | Gum | | Gum | N/A |
| Acetonitrile / water 97:3 v/v | Gum | | Gum | | Gum | N/A |

### Example 6

### Competitive slurry experiments between Form I and Form II

A mechanical mixture of Form I and Form II of 5-(3,4-difluorobenzyl)-8-((1r,4r)-4-methylcyclohexyl)-6,9-dioxo-2,5,8-triazaspiro[3.5]nonane-2-carbaldehyde was prepared, which was then used for competitive slurry experiments between Forms I and II to determine the most stable form over a range of temperatures (5-50 °C). From the competitive slurries, Form II was determined to be the most stable form under the investigated conditions and was observed in all experiments where solids were isolated. These results indicate there is monotropic relationship between Form I and Form II and that Form II is the more thermodynamically stable form at all temperatures. The results of the competitive slurry experiments are provided below in Table 6.

**Table 6**

| **Starting Materials** | **Solvent** | **Temperature (°C)** | **XRPD** |
|---|---|---|---|
| | MIBK | | Form II |
| | EtOAc | | Form II |
| | EtOH | | Form II |
| | Toluene | | Low intensity peaks. Diffractogram unchanged after 5 days |
| | acetonitrile | | Form II |
| Forms I and II | EtOH/water (85:15 v/v) | 5 | Form II (low intensity peaks) |
| | EtOH/water (50:50 v/v) | | Form II (low intensity peaks) |
| | EtOH/water (15/85 v/v) | | Form II (low intensity peaks) |
| | EtOAc/cyclohexane (50:50 v/v) | | Form II |
| | EtOAc/cyclohexane (85:15 v/v) | | Form II |
| | MIBK | | Form II |
| | EtOAc | | N/A |
| | EtOH | | Form II |
| | Toluene | | Form II |
| | acetonitrile | | Form II |
| | EtOH/water (85:15 v/v) | 50 | Form II |
| | EtOH/water (50:50 v/v) | | N/A |
| | EtOH/water (15/85 v/v) | | Form II |
| | EtOAc/cyclohexane (50:50 v/v) | | Form II |
| | EtOAc/cyclohexane (85:15 v/v) | | Form II |

### Example 7

### Synthesis of 5-(4-chlorobenzyl)-8-(2,4-difluorophenyl)-6,9-dioxo-2,5,8-triazaspiro[3.5]nonane-2-carbaldehyde (Comparator A)

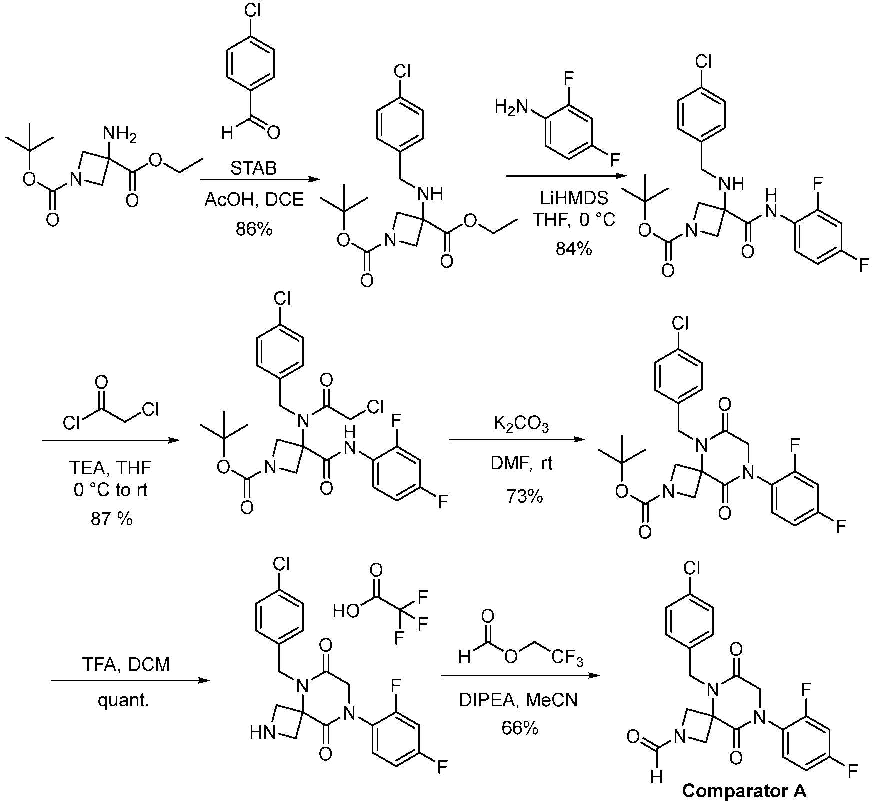

### Step 1: Synthesis of 1-(tert-butyl) 3-ethyl 3-((4-chlorobenzyl)amino)azetidine-1,3-dicarboxylate

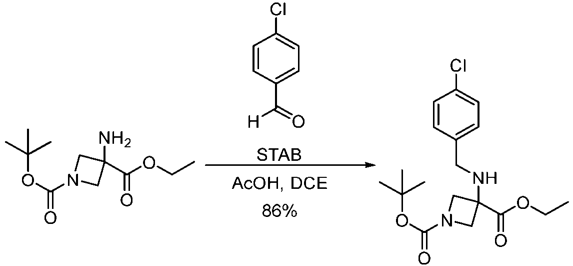

To a solution of 1-tert-butyl 3-ethyl 3-aminoazetidine-1,3-dicarboxylate (10.6 g, 43.3 mmol, 1.0 equiv) and 4-chlorobenzaldehyde (6.1 g, 43.3 mmol, 1.0 equiv) in DCE (120.0 mL) at 0 °C were added AcOH (5.0 mL). Upon warming to r.t. and stirred at rt, to the mixture was added additional AcOH (5.0 mL) twice at 4 h, 8.5 h, and 9 h (total of 20.0 mL). To this mixture was added STAB (11.0 g, 52.0 mmol, 1.2 equiv.). The mixture was stirred at r.t. overnight, diluted with aqueous sodium bicarbonate, and extracted with DCM three times. The combined organic layers were dried over sodium sulfate and concentrated. The obtained oil was dissolved in warmed hexanes and EA (dropwise until the mixture went into homogeneous solution) at 60 °C with stirring. The mixture was then cooled to 0 °C and precipitation was collected by filtration and washed with cold hexanes. The filtrate was concentrated and repeated the process again. The combined solid was dried under vacuum to afford 13.8 g (86%) of *1-(tert-butyl)* 3-ethyl 3-((4-chlorobenzyl)amino)azetidine-1,3-dicarboxylate as a white solid. LRMS (ES) m/z 313.1 (M+H-56).

### Step 2: Synthesis of tert-butyl 3-((4-chlorobenzyl)amino)-3-((2,4-difluorophenyl)carbamoyl)azetidine-1-carboxylate

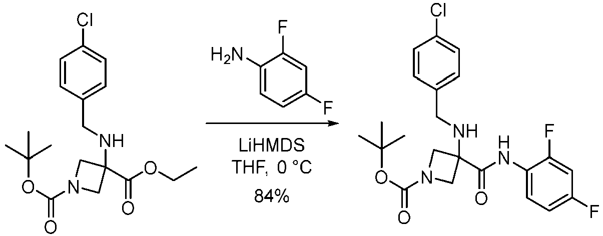

To a stirred solution of *1-(tert-butyl)* 3-ethyl 3-((4-chlorobenzyl)amino)azetidine-1,3-dicarboxylate (10.0 g, 27.1 mmol, 1.0 equiv) and 2,4-difluoroaniline (3.0 mL, 29.8 mmol, 1.1 equiv.) in THF (200.0 mL) at 0 °C under N₂ were added a solution of LHMDS (54.2 mL, 1 M in THF, 54.2 mmol, 2.0 equiv) dropwise over a period of 20 min. The mixture was stirred at 0 °C for 20 min, quenched with water (50 mL, acidified to pH 5 using aqueous HCl (0.5 N), and extracted with EA (100 mL) twice. The combined organic layer were washed with brine, dried over sodium sulfate, concentrated to afford 11.3 g of *tert-butyl 3-((4-*chlorobenzyl)amino)-3-((2,4-difluorophenyl)carbamoyl)azetidine-1-carboxylate, which was used for next step without purification. LRMS (ES) m/z 396.1 (M+H-56).

### Step 3: Synthesis of tert-butyl 3-(2-chloro-N-(4-chlorobenzyl)acetamido)-3-((2,4-difluorophenyl)carbamoyl)azetidine-1-carboxylate

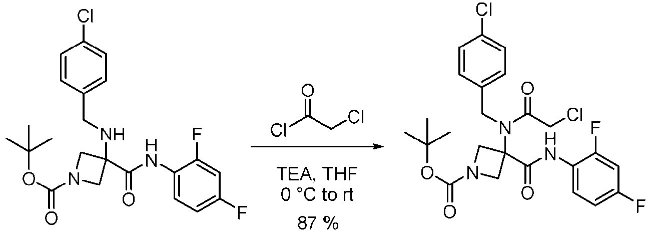

To a solution of *tert-butyl* 3-((4-chlorobenzyl)amino)-3-((2,4-difluorophenyl)carbamoyl)azetidine-1-carboxylate (11.3 g, 23.2 mmol, 1.0 equiv) in THF (20 mL) cooled to 0 °C were added TEA (4.8 mL, 34.7 mmol, 1.5 equiv) and 2-chloroacetyl chloride (1.5 mL, 27.8 mmol, 1.2 equiv). Upon stirring at 0 °C. for 30 min, additional TEA (4.8 mL, 34.7 mmol, 1.5 equiv) and 2-chloroacetyl chloride (1.8 mL, 23.2 mmol, 1.0 equiv) were added into the mixture and the mixture was stirred at 0 °C. for 30 min. To this mixture was added additional 2-chloroacetyl chloride (0.6 mL, 6.9 mmol, 0.3 equiv). The mixture was gradually warmed to rt, stirred for 45min, cooled to 0 °C, and quenched with aqueous sodium bicarbonate. The mixture was extracted with EA three times. The combined organic layers were washed with bring, dried over sodium sulfate, and concentrated to afford 13.2 g of *tert-*butyl 3-(2-chloro-N-(4-(trifluoromethyl)benzyl)acetamido)-3-((2,4-difluorophenyl)carbamoyl)azetidine-1-carboxylate, which was used for next step without further purification. LRMS (ES) m/z 528.1 (M+H).

### Step 4: Synthesis of tert-butyl 5-(4-chlorobenzyl)-8-(2,4-difluorophenyl)-6,9-dioxo-2,5,8-triazaspiro[3.5]nonane-2-carboxylate

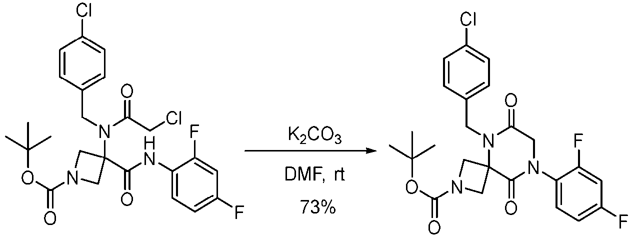

To a stirred solution of tert-butyl 3-(2-chloro-N-(4-(trifluoromethyl)benzyl)acetamido)-3-((2,4-difluorophenyl)carbamoyl)azetidine-1-carboxylate (13.2 g, 20.1 mmol, 1.0 equiv) in DMF (40.0 mL) under N₂ was added K₂CO₃ (4.2 g, 30.1 mmol, 1.5 equiv.). The resulting mixture was stirred at r.t. for 3.5 h, diluted with water, and extracted with EA twice. The combined organic layers were washed with water twice and brine once, dried over sodium sulfate, and concentrated to afford 11.7 g of *tert-*butyl 5-(4-chlorobenzyl)-8-(2,4-difluorophenyl)-6,9-dioxo-2,5,8-triazaspiro[3.5]nonane-2-carboxylate. LRMS (ES) m/z 436.1 (M+H-56).

### Step 5: Synthesis of 5-(4-chlorobenzyl)-8-(2,4-difluorophenyl)-2,5,8-triazaspiro[3.5]nonane-6,9-dione 2,2,2-trifluoroacetate

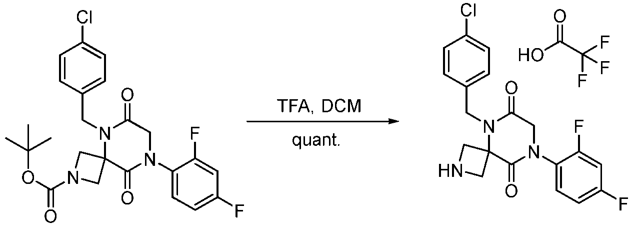

To a stirred solution of *tert-butyl* 5-(4-chlorobenzyl)-8-(2,4-difluorophenyl)-6,9-dioxo-2,5,8-triazaspiro[3.5]nonane-2-carboxylate (14.7 g, 29.8 mmol, 1.0 equiv) in DCM (50.0 mL) was added TFA (25.0 mL). The resulting mixture was stirred at r.t. for 3 h and concentrated to dryness to afford 15.1 g of 5-(4-chlorobenzyl)-8-(2,4-difluorophenyl)-2,5,8-triazaspiro[3.5]nonane-6,9-dione 2,2,2-trifluoroacetate, which was used for next step without further purification. LRMS (ES) m/z 392.1 (M+H).

### Step 6: Synthesis of 5-(4-chlorobenzyl)-8-(2,4-difluorophenyl)-6,9-dioxo-2,5,8-triazaspiro[3.5]nonane-2-carbaldehyde

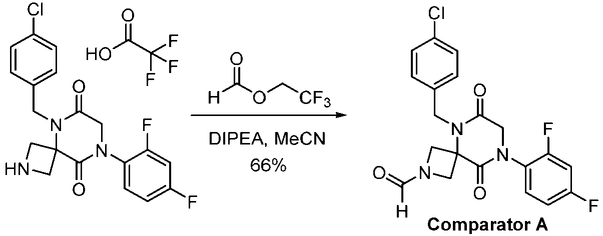

To a solution of 5-(4-chlorobenzyl)-8-(2,4-difluorophenyl)-2,5,8-triazaspiro[3.5]nonane-6,9-dione 2,2,2-trifluoroacetate (15.1 g, 29.9 mmol, 1 equiv) and *N,N-*diisopropylethylamine (15.7 mL, 89.7 mmol, 3 equiv) in ACN (50 mL) was added 2,2,2-trifluoroethyl formate (5.8 mL, 59.8 mmol, 2 equiv). The reaction was stirred at r.t. for 1 hour, diluted with water, and extracted with DCM. The combined organic layers were dried over sodium sulfate, concentrated, and purified by silica gel chromatography using 0 to 100% EtOAc gradient, then 0-10% MeOH, DCM gradient. The solid was suspended in EtOH/MTBE 1:1, heated to 80 °C, cooled on ice, filtered, washed with MTBE, and dried to afford 8.3 g (66%) of 5-(4-chlorobenzyl)-8-(2,4-difluorophenyl)-6,9-dioxo-2,5,8-triazaspiro[3.5]nonane-2-carbaldehyde LRMS (ES) m/z 420.1 (M+H). ¹H NMR (400 MHz, DMSO-*d*₆) δ 7.98 (s, 1H), 7.65 (td, *J* = 8.8, 6.0 Hz, 1H), 7.48 - 7.41 (m, 1H), 7.43 (d, *J* = 8.5 Hz, 2H), 7.34 (d, *J* = 8.4 Hz, 2H), 7.22 (td, *J* = 8.4, 2.4 Hz, 1H), 4.91 (s, 2H), 4.55 (d, *J* = 9.7 Hz, 1H), 4.43 (d, *J* = 1.6 Hz, 2H), 4.37 (d, *J* = 9.8 Hz, 1H), 4.28 (d, *J* = 10.8 Hz, 1H), 4.08 (d, *J =* 10.8 Hz, 1H).

### Example 8

### Synthesis of 8-(2,4-difluorophenyl)-6,9-dioxo-5-(4-(trifluoromethyl)benzyl)-2,5,8-triazaspiro[3.5]nonane-2-carbaldehyde (Comparator B)

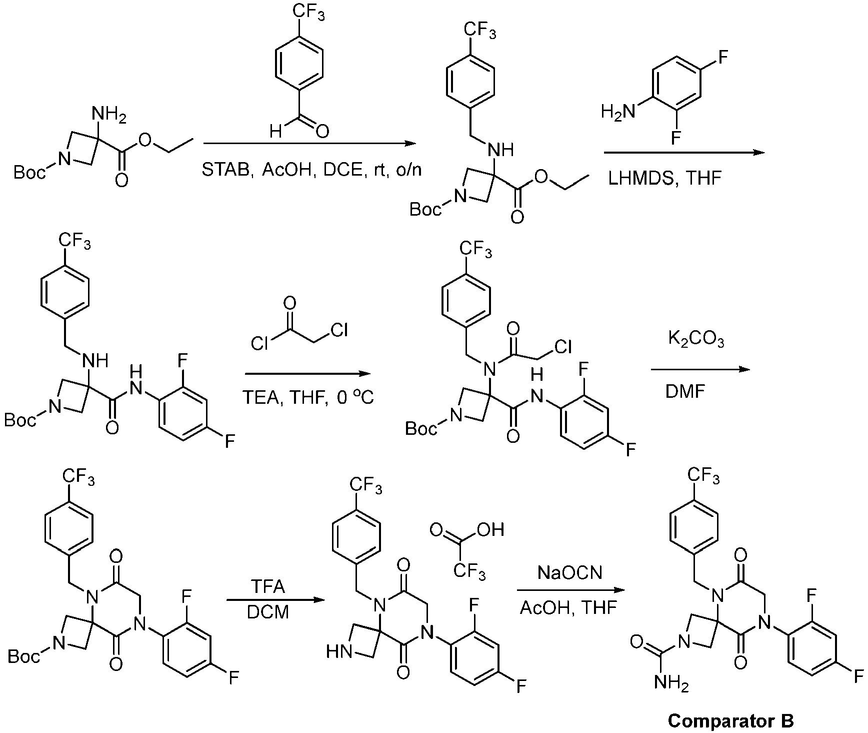

### Step 1. Synthesis of 1-(tert-butyl) 3-ethyl 3-((4-(trifluoromethyl)benzyl)amino)azetidine-1,3-dicarboxylate

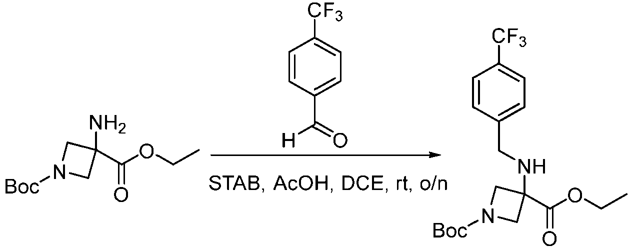

To a solution of 1-tert-butyl 3-ethyl 3-aminoazetidine-1,3-dicarboxylate (15.0 g, 61.4 mmol, 1.0 equiv) and 4-(trifluoromethyl)benzaldehyde (11.8 g, 67.5 mmol, 1.1 equiv) in DCE (60.0 mL) at 0 °C were added AcOH (7.4 g, 122.8 mmol, 2.0 equiv.) and STAB (19.5 g, 92.1 mmol, 1.5 equiv) in portions. The resulting mixture was stirred at r.t. overnight, adjusted the pH to 8 with ammonium hydroxide, added water (100.0 mL) and extracted twice with DCM (300.0 mL). The combined organic layers were washed with brine twice, dried over anhydrous Na₂SO₄, and concentrated under reduced pressure to afford 29.6 g of *1-(tert-butyl)* 3-ethyl 3-((4-(trifluoromethyl)benzyl)amino)azetidine-1,3-dicarboxylate, which was sued for next step without purification. LRMS (ES) m/z 347.1 (M+H-56)

### Step 2. Synthesis of tert-butyl 3-((2,4-difluorophenyl)carbamoyl)-3-((4-(trifluoromethyl)benzyl)amino)azetidine-1-carboxylate

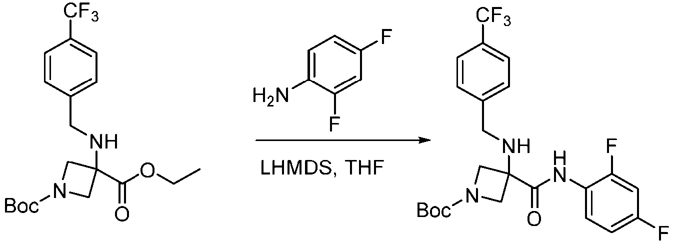

To a stirred solution of *1-(tert-butyl)* 3-ethyl 3-((4-(trifluoromethyl)benzyl)amino)azetidine-1,3-dicarboxylate (29.6 g, 44.1 mmol, 1.0 equiv) and 2,4-difluoroaniline (4.9 mL, 48.5 mmol, 1.1 equiv.) in THF (200.0 mL) at 0 °C under N₂ were added a solution of LHMDS (88.3 mL, 1 M in THF, 88.3 mmol, 2.0 equiv) dropwise over a period of 20 min. The mixture was stirred at 0 °C for 20 min, quenched with water (50 mL, acidified to pH 5 using aqueous HCl (3N), and extracted with EA (100 mL) twice. The combined organic layer were washed with brine, dried over sodium sulfate, concentrated to afford 36.2 g of *tert*-butyl 3-((2,4-difluorophenyl)carbamoyl)-3-((4-(trifluoromethyl)benzyl)amino)azetidine-1-carboxylate, which was used for next step without purification. LRMS (ES) m/z 430.1 (M+H-56).

### Step 3. Synthesis of tert-butyl 3-(2-chloro-N-(4-(trifluoromethyl)benzyl)acetamido)-3-((2,4-difluorophenyl)carbamoyl)azetidine-1-carboxylate

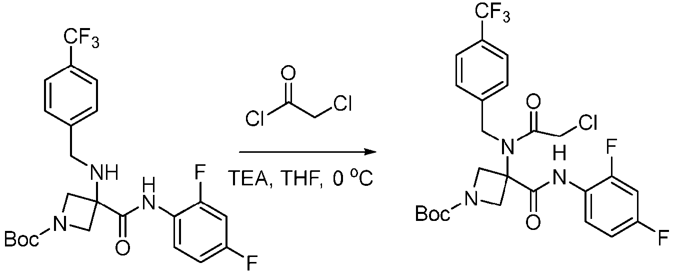

To a solution of *tert*-butyl 3-((2,4-difluorophenyl)carbamoyl)-3-((4-(trifluoromethyl)benzyl)amino)azetidine-1-carboxylate (36.2 g, 40.6 mmol, 1.0 equiv) in THF (100 mL) cooled to 0 °C were added TEA (8.5 mL, 60.9 mmol, 1.5 equiv) and 2-chloroacetyl chloride (3.9 mL, 48.7 mmol, 1.2 equiv). Upon stirring at 0 °C. for 30 min, additional TEA (8.5 mL, 60.9 mmol, 1.5 equiv) and 2-chloroacetyl chloride (3.3 mL, 40.6 mmol, 1.0 equiv) were added into the mixture and the mixture was stirred at 0 °C. for 30 min. To this mixture was added additional 2-chloroacetyl chloride (1.0 mL, 12.2 mmol, 0.3 equiv). The mixture was gradually warmed to rt, stirred for 45min, cooled to 0 °C, and quenched with aqueous sodium bicarbonate. The mixture was extracted with EA three times. The combined organic layers were washed with bring, dried over sodium sulfate, and concentrated to afford 42.0 g of *tert-butyl* 3-(2-chloro-*N*-(4-(trifluoromethyl)benzyl)acetamido)-3-((2,4-difluorophenyl)carbamoyl)azetidine-1-carboxylate, which was used for next step without further purification. LRMS (ES) m/z 562.1 (M+H).

### Step 4. Synthesis of tert-butyl 8-(2,4-difluorophenyl)-6,9-dioxo-5-(4-(trifluoromethyl)benzyl)-2,5,8-triazaspiro[3.5]nonane-2-carboxylate

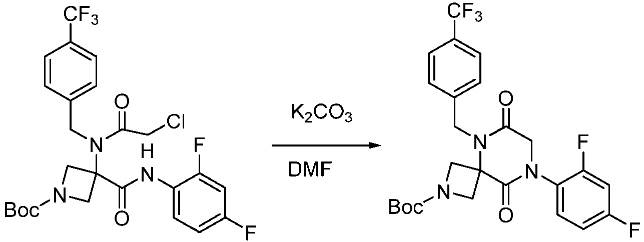

To a stirred solution of *tert-butyl* 3-(2-chloro-*N*-(4-(trifluoromethyl)benzyl)acetamido)-3-((2,4-difluorophenyl)carbamoyl)azetidine-1-carboxylate (42.0 g, 33.4 mmol, 1.0 equiv) in DMF (80.0 mL) under N₂ was added K₂CO₃ (7.0 g, 50.1 mmol, 1.5 equiv.). The resulting mixture was stirred at r.t. for 3.5 h, diluted with water, and extracted with EA twice. The combined organic layers were washed with water twice and brine once, dried over sodium sulfate, concentrated, and purified by silica gel using a gradient of 0-60% EA in hexanes as eluent to afford 12.0 g (37% over 4 steps) of *tert-butyl* 8-(2,4-difluorophenyl)-6,9-dioxo-5-(4-(trifluoromethyl)benzyl)-2,5,8-triazaspiro[3.5]nonane-2-carboxylate. LRMS (ES) m/z 469.7 (M+H-56).

### Step 5. Synthesis of 8-(2,4-difluorophenyl)-5-(4-(trifluoromethyl)benzyl)-2,5,8-triazaspiro[3.5]nonane-6,9-dione 2,2,2-trifluoroacetate

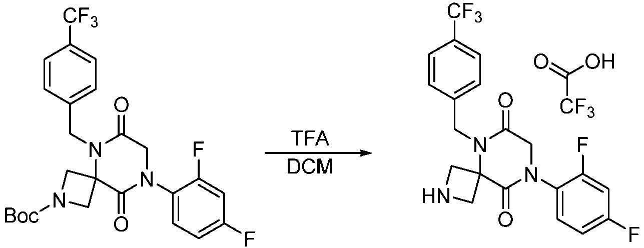

To a stirred solution of tert-butyl 8-(2,4-difluorophenyl)-6,9-dioxo-5-(4-(trifluoromethyl)benzyl)-2,5,8-triazaspiro[3.5]nonane-2-carboxylate (12.0 g, 22.8 mmol, 1.0 equiv) in DCM (12.0 mL) was added TFA (40.0 mL). The resulting mixture was stirred at r.t. for 3 h and concentrated to dryness to afford 12.3 g of 8-(2,4-difluorophenyl)-5-(4-(trifluoromethyl)benzyl)-2,5,8-triazaspiro[3.5]nonane-6,9-dione 2,2,2-trifluoroacetate, which was used for next step without further purification. LRMS (ES) m/z 426.1 (M+H).

### Step 6. Synthesis of 8-(2,4-difluorophenyl)-6,9-dioxo-5-(4-(trifluoromethyl)benzyl)-2,5,8-triazaspiro[3.5]nonane-2-carbaldehyde

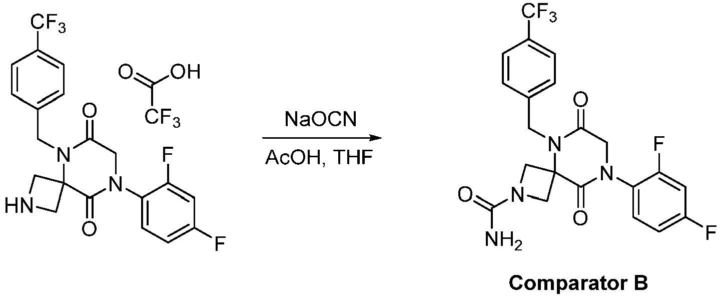

To a stirred solution of 8-(2,4-difluorophenyl)-5-(4-(trifluoromethyl)benzyl)-2,5,8-triazaspiro[3.5]nonane-6,9-dione 2,2,2-trifluoroacete (12.3 g, 22.8 mmol, 1.0 equiv) in THF (50.0 mL) were added sodium cyanate (4.4 g, 68.4 mmol, 3.0 equiv.) and a few drops of acetic acid. The mixture was stirred at r.t. for 30 min, concentrated, and purified by silica column chromatography using a gradient of 0-10% MeOH in DCM as eluent to afford 8.8 g (73% over two steps) of 8-(2,4-difluorophenyl)-6,9-dioxo-5-(4-(trifluoromethyl)benzyl)-2,5,8-triazaspiro[3.5]nonane-2-carbaldehyde. LRMS (ES) m/z 469.1 (M+H); ¹H NMR (400 MHz, Methanol-d4) δ 7.70 (d, J = 8.1 Hz, 2H), 7.61 - 7.53 (m, 3H), 7.20 (ddd, J = 10.4, 8.8, 2.8 Hz, 1H), 7.12 (dddd, J = 9.1, 8.0, 2.8, 1.4 Hz, 1H), 5.15 (s, 2H), 4.53 - 4.49 (m, 4H), 4.20 (d, J = 9.5 Hz, 2H).

### Example 9

### Synthesis of 8-((1r,4r)-4-(difluoromethyl)cyclohexyl)-6,9-dioxo-5-(4-(trifluoromethyl)benzyl)-2,5,8-triazaspiro[3.5]nonane-2-carbaldehyde (Comparator E)

### Step 1: Synthesis of tert-butyl 3-cyano-3-((4-(trifluoromethyl)benzyl)amino)azetidine-1-carboxylate:

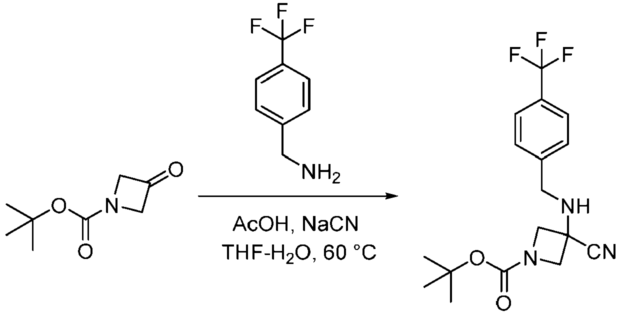

To a solution of *tert-butyl* 3-oxoazetidine-1-carboxylate (25 g, 146.0 mmol, 1.0 equiv) in THF (90 mL) were added acetic acid (10.5 g, 175.2 mmol, 1.2 equiv) and (4-(trifluoromethyl)phenyl)methanamine (31.7 g, 181.1 mmol, 1.2 equiv) in water (40.0 mL). After stirring at r.t. for 5 minutes, to the mixture was added a solution of sodium cyanide (7.2 g, 146.0 mmol, 1.0 equiv) in water (10 mL) was added. The mixture was heated at 60 °C for 18 h in an oil bath, cooled to rt, neutralized by addition of a saturated aqueous solution of sodium bicarbonate, and extracted with ethyl acetate (150 mL x 2). The combined organic layers were washed with brine, dried over magnesium sulfate, and concentrated under reduced pressure. To the resulting yellow solid was added diethyl ether/hexanes (200 mL, 1:2) and the solution was sonicated for a minute, cooled to 0 °C, and filtered. The resulting white precipitate was washed with ice cold diethyl ether (50 mL) and dried overnight to provide *tert-butyl* 3-cyano-3-((4-(trifluoromethyl)benzyl)amino)azetidine-1-carboxylate (43.9 g, 85% yield). LRMS (ES) m/z 329.2 (M+H-27). ¹H NMR (400 MHz, DMSO-*d*₆) δ 7.71 (d, *J* = 8.0 Hz, 2H), 7.60 (d, *J* = 8.0 Hz, 2H), 4.16 (d, *J =* 8.8 Hz, 2H), 3.92 (t, *J* = 7.2 Hz, 1H), 3.84 (d, *J* = 9.2 Hz, 2H), 3.81 (dd, *J* = 7.3 Hz, 2H), 1.39 (s, 9H).

### Step 2: Synthesis of tert-butyl 3-(2-chloro-N-(4-(trifluoromethyl)benzyl)acetamido)-3-cyanoazetidine-1-carboxylate:

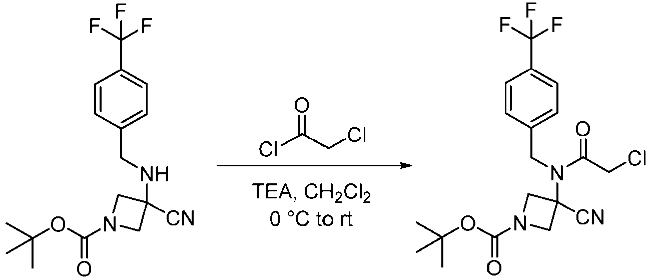

To a solution of *tert-butyl* 3-cyano-3-((4-(trifluoromethyl)benzyl)amino)azetidine-1-carboxylate (3.0 g, 8.4 mmol, 1.0 equiv) and triethylamine (1.3 g, 12.7 mmol, 1.5 equiv) in DCM (0.2 M) cooled to 0 °C was added chloroacetyl chloride (0.95 g, 8.4 mmol, 1.0 equiv). The mixture was stirred at 0 °C for 15 min, warmed to rt, and stirred for 2 h. To the mixture were added additional 2-chloroacetyl chloride (0.95 g, 8.4 mmol, 1.0 equiv) and triethylamine (1.3 g, 12.7 mmol, 1.5 equiv). The reaction was stirred for 2 h, quenched with a saturated aqueous solution of ammonium chloride, and separated the layers. The aqueous layer was extracted with DCM once. The combined organic layers were dried over magnesium sulfate, concentrated, and purified by silica gel column chromatography (5%-70% EtOAc/hexanes, R*_{f}*=0.24 (20% EtOAc/hexanes) to afford 3.4 g (92%) of *tert*-butyl 3-(2-chloro-*N*-(4-(trifluoromethyl)benzyl)acetamido)-3-cyanoazetidine-1-carboxylate. LRMS (ES) m/z 432.1 (M+H). ¹H NMR (400 MHz, DMSO-*d*₆) δ 7.77 (d, *J =* 8.1 Hz, 2H), 7.60 (d, *J* = 8.0 Hz, 2H), 4.95 (s, 2H), 4.52 (s, 2H), 4.15 (s, 4H), 1.35 (s, 9H).

### Step 3: Synthesis of tert-butyl 3-cyano-3-(2-(((1r,4r)-4-(difluoromethyl)cyclohexyl)amino)-N-(4-(trifluoromethyl)benzyl)acetamido)azetidine-1-carboxylate:

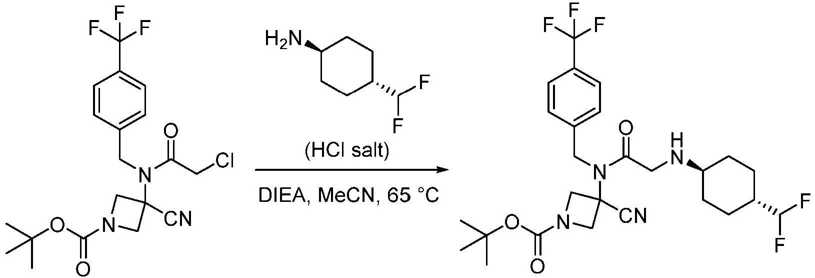

To a solution of *tert*-butyl 3-(2-chloro-N-(4-(trifluoromethyl)benzyl)acetamido)-3-cyanoazetidine-1-carboxylate (0.8 g, 1.9 mmol, 1 equiv) in acetonitrile (15 mL) were added (1r,4r)-4-(difluoromethyl)cyclohexan-1-amine hydrochloride (0.51 g, 2.8 mmol, 1.5 equiv) and DIPEA (1.2 g, 9.3 mmol, 5 equiv). The solution was heated at 65 °C for 4 h at which point LCMS indicated completion of reaction. The reaction was diluted with ethyl acetate and water (1:1, 80 mL) and the aqueous layer was extracted with ethyl acetate. The combined organic layers were washed with brine, dried over magnesium sulfate, concentrated, and purified with silica gel chromatography using a gradient of 25% to 100% ethyl acetate in hexanes as eluent to afford 0.65 g (64%) of *tert-butyl* 3-cyano-3-(2-(((1r,4r)-4-(difluoromethyl)cyclohexyl)amino)-N-(4-(trifluoromethyl)benzyl)acetamido)azetidine-1-carboxylate as a pale yellow oil. R*_{f}* =0.55 (100% ethyl acetate, silica). LRMS (ES) m/z 545.0 (M+H).

### Step 4: Synthesis of tert-butyl 8-((1r,4r)-4-(difluoromethyl)cyclohexyl)-6,9-dioxo-5-(4-(trifluoromethyl)benzyl)-2,5,8-triazaspiro[3.5]nonane-2-carboxylate:

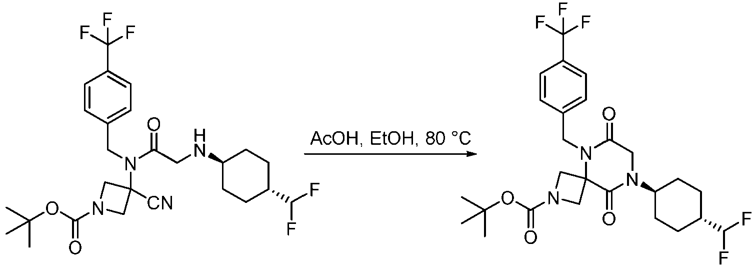

To a solution of *tert*-butyl 3-cyano-3-(2-(((1*r*,4*r*)-4-(difluoromethyl)cyclohexyl)amino)-*N*-(4-(trifluoromethyl)benzyl)acetamido)azetidine-1-carboxylate (0.27 g, 0.50 mmol, 1.0 equiv) in ethanol (2 mL) was added acetic acid (0.18 g, 3.0 mmol, 6.0 equiv). The reaction was heated at 70 °C for 15 hours, cooled to rt, and diluted with hexanes (1.0 mL). The precipitation was collected by filtration, washed with ethanol-hexanes (1:2, 2 mL), and dried to afford 186 mg (69%) of *tert*-butyl 8-((1*r*,4*r*)-4-(difluoromethyl)cyclohexyl)-6,9-dioxo-5-(4-(trifluoromethyl)benzyl)-2,5,8-triazaspiro[3.5]nonane-2-carboxylate as a pale yellow solid. LRMS (ES) m/z 490.2 (M+H-56). ¹H NMR (400 MHz, DMSO-*d*₆) δ 7.71 (d, *J =* 8.1 Hz, 2H), 7.48 (d, *J =* 8.0 Hz, 2H), 5.89 (td, *J =* 56.7, 3.8 Hz, 1H), 4.93 (s, 2H), 4.29 - 4.15 (m, 3H), 4.02 (s, 2H), 3.93 (d, *J* = 9.5 Hz, 2H), 1.91 - 1.55 (m, 7H), 1.35 (s, 9H), 1.38 - 1.20 (m, 2H).

### Step 5: Synthesis of 8-((1r,4r)-4-(difluoromethyl)cyclohexyl)-5-(4-(trifluoromethyl)benzyl)-2,5,8-triazaspiro[3.5]nonane-6,9-dione 2,2,2-trifluoroacetate:

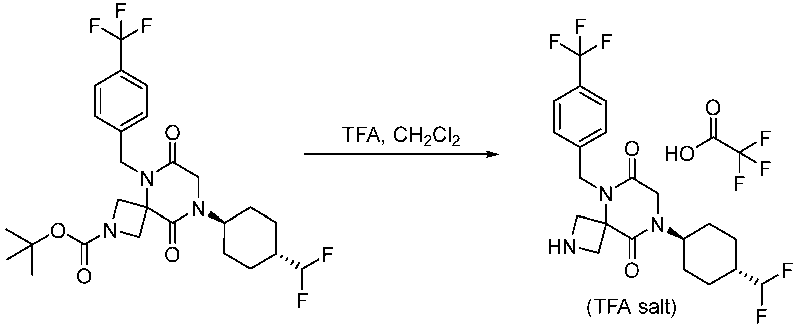

To a solution of *tert-butyl* 8-((1r,4r)-4-(difluoromethyl)cyclohexyl)-6,9-dioxo-5-(4-(trifluoromethyl)benzyl)-2,5,8-triazaspiro[3.5]nonane-2-carboxylate (200 mg, 0.37 mmol, 1.0 equiv) in DCM (1.5mL) was added TFA (1.5mL) at r.t. The mixture was stirred at r.t. for 1 h, concentrated under reduced pressure, and dried under high vacuum to afford 190 mg (94%) of 8-((1r,4r)-4-(difluoromethyl)cyclohexyl)-5-(4-(trifluoromethyl)benzyl)-2,5,8-triazaspiro[3.5]nonane-6,9-dione 2,2,2-trifluoroacetate, which was used without further purification. LRMS (ES) m/z 446.2 (M+H).

### Step 6: Synthesis of 8-((1r,4r)-4-(difluoromethyl)cyclohexyl)-6,9-dioxo-5-(4-(trifluoromethyl)benzyl)-2,5,8-triazaspiro[3.5]nonane-2-carbaldehyde (Comparator E):

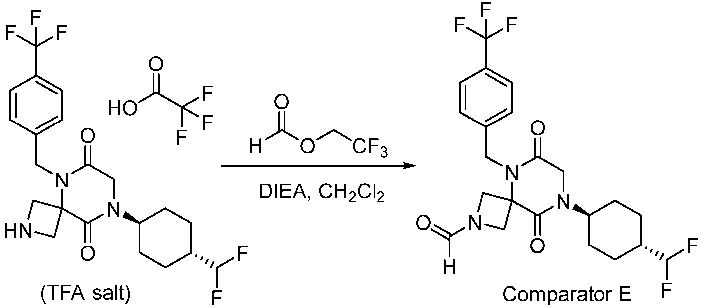

To a solution of 8-((1r,4r)-4-(difluoromethyl)cyclohexyl)-5-(4-(trifluoromethyl)benzyl)-2,5,8-triazaspiro[3.5]nonane-6,9-dione 2,2,2-trifluoroacetate (54.0 mg, 0.10 mmol) in acetonitrile (0.6 mL) were added DIPEA (37.0 mg, 0.29 mmol, 3.0 equiv) and 2,2,2-trifluoroethyl formate (124.0 mg, 0.97 mmol, 10.0 equiv). The mixture was heated in a microwave reactor at 110 °C for 20 min, concentrated, and purified by HPLC using a gradient of 10% to 100% ACN in water (both with 0.1% HCOOH) as eluent to afford 21.0 mg (46%) of 8-((1r,4r)-4-(difluoromethyl)cyclohexyl)-6,9-dioxo-5-(4-(trifluoromethyl)benzyl)-2,5,8-triazaspiro[3.5]nonane-2-carbaldehyde as a foam. LRMS (ES) m/z 473.9 (M+H). ¹H NMR (400 MHz, DMSO-*d*₆) δ 7.96 (s, 1H), 7.71 (d, *J* = 8.0 Hz, 2H), 7.47 (d, *J =* 8.0 Hz, 2H), 5.89 (td, *J* = 56.4, 4.5 Hz, 1H), 4.94 (s, 2H), 4.51 (d, *J* = 9.6 Hz, 1H), 4.30 - 4.15 (m, 3H), 4.04 (s, 2H), 3.96 (d, *J* = 10.7 Hz, 1H), 1.95 - 1.49 (m, 7H), 1.41 - 1.06 (m, 2H).

### Biological Example B-1: Myofibril Assay

To evaluate the effect of the compound 5-(3,4-difluorobenzyl)-8-((1*r*,4*r*)-4-methylcyclohexyl)-6,9-dioxo-2,5,8-triazaspiro[3.5]nonane-2-carbaldehyde on the ATPase activity of full-length cardiac myosin in the context of the native sarcomere, skinned myofibril assays were performed. Bovine cardiac myofibrils were obtained by homogenizing bovine cardiac left ventricular tissue in the presence of a detergent such as triton X-100. Such treatment removes membranes and a majority of the soluble cytoplasmic proteins but leaves intact the cardiac sarcomeric acto-myosin apparatus. Myofibril preparations retain the ability to hydrolyze ATP in a Ca²⁺ regulated manner. ATPase activities of such myofibril preparations in the presence and absence of the compound 5-(3,4-difluorobenzyl)-8-((1r,4r)-4-methylcyclohexyl)-6,9-dioxo-2,5,8-triazaspiro[3.5]nonane-2-carbaldehyde was assayed at Ca²⁺ concentrations activating to a defined fraction of the maximal rate (i.e., 25%, 75%). The compound 5-(3,4-difluorobenzyl)-8-((1*r*,4*r*)-4-methylcyclohexyl)-6,9-dioxo-2,5,8-triazaspiro[3.5]nonane-2-carbaldehyde was assessed for its ability to inhibit the steady-state ATPase activity of bovine cardiac myofibrils using pyruvate kinase and lactate dehydrogenase (PK/LDH)-coupled enzyme system. This assay regenerates myosin-produced ADP into ATP by oxidizing NADH, producing an absorbance change at 340 nm. Prior to testing this compound, the bovine cardiac myofibrils were assessed for their calcium responsiveness and the calcium concentration that achieves either a 50% (pCa₅₀) or 75% (pCa₇₅) activation of the myofibril system was chosen as the final condition for assessing the inhibitory activity of this compound. All enzymatic activity was measured in a buffered solution containing 12 mM PIPES (piperazine-N,N'-bis(2-ethanesulfonic acid), 2 mM magnesium chloride at pH 6.8 (PM 12 buffer). Final assay conditions were 1 mg/mL of bovine cardiac myofibrils, 4 U/mL pyruvate kinase, 6 U/mL lactate dehydrogenase, 50 µM ATP, 0.1 mg/mL BSA (bovine serum albumin), 10 ppm antifoam, 1 mM DTT, 0.5 mM NADH, 1.5 mM PEP, 0.6 mM EGTA, and an amount of CaCl₂ sufficient to achieve either 50% or 75% activation of the myofibril ATPase activity. The compound tested was prepared in accordance with the synthetic procedures described herein. Results for compounds tested are provided in Table B-1.

**Table B-1**

| **Compound** | **CDMF IC₁₅ (µM)** |
|---|---|
| **1** | 1.5 |
| Comparator A | 1.8 |
| Comparator B | 1.1 |
| Comparator C | 1.1 |
| Comparator D | 1.1 |
| Comparator E | 1.3 |

Comparator C and Comparator D have the following structures:

| **Compound** | **Structure** | **Name** |
|---|---|---|
| Comparator C | 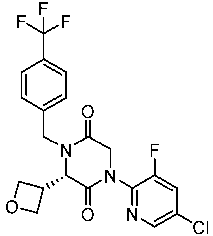 | (S)-1-(5-chloro-3-fluoropyridin-2-yl)-3-(oxetan-3-yl)-4-(4-(trifluoromethyl)benzyl)piperazi ne-2,5-dione |
| Comparator D | 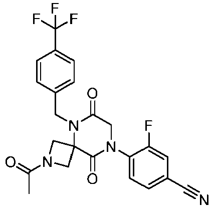 | 4-(2-acetyl-6,9-dioxo-5-(4-(trifluoromethyl)benzyl)-2,5,8-triazaspiro[3.5]nonan-8-yl)-3-fluorobenzonitrile |

The preparation of Comparator C and Comparator D is described in WO2020/047447A1.

### Biological Example B-2: Pharmacokinetics Single Dose Studies

### Mouse Single Dose Studies

Male C57BL/6 mice (18-25g, 6-8 weeks old) were obtained from Zhejiang Vital River Laboratory Animal Technology Co., Ltd. All animals for IV administration had free access to food and water. The IV dosing was performed through tail vein. The IV dose solution for the Test Articles was prepared in 10% DMA/20% PG/70% HPβCD solution (40% w/v aqueous HPβCD) at a concentration of 0.1 mg/mL. The oral dosing suspension was prepared by suspending Test Article in 0.5% HPMC/0.1% Tween 80 in water at a concentration 0.2 mg/mL. Concentrations of IV and PO doses were measured at the end of the study. Pharmacokinetic (PK) parameters were calculated using the nominal dose values if the measured values were within 20% of the nominal values. A group of 15 mice received the IV dose where the volume was 5 mL/kg. Another group of 15 mice received by oral gavage of Test Article at 1 mg/kg. The oral dose volume was 5 mL/kg. Sparse blood samples were collected from groups of three mice via retro-orbital bleeding, placed into a K₂EDTA microtainer tube and maintained on ice until centrifugation to obtain plasma. Each designated group of mice were bled at two-time points. The time points were predose (PO only), 5 (IV only), 15, 30 minutes, 1, 2, 4, 6, 8 and 24 hours postdose. Blood samples were centrifuged and the collected plasma was stored at -80°C until analysis. Plasma samples were analyzed for Test Article concentrations using an LC/MS/MS method. Briefly, a 50 µL aliquot of each plasma sample was mixed with 100 µL of acetonitrile that contained an internal standard (IS). The mixture was vortexed and centrifuged. Ten (10) µL of the resulting solution was injected onto a reverse-phase C18 column and the resultant peaks detected on a LC/MS/MS equipped with a turbo ionspray ionization source. Sample concentrations below the limit of quantification (BLQ) were treated as zero for PK calculations. Composite PK parameters were estimated from a maximum of two sampling points per mouse and three mice per sampling point and the sparse data option of WinNonlin was used for noncompartmental analysis of the concentration-time data (Phoenix WinNonLin software, version 64; Pharsight, Mountain View, CA). The elimination rate constant (k) was calculated as the absolute value of the slope of the linear regression of logarithm of the concentration versus time for the last three data points of the concentration-time profiles. Apparent elimination half-life (t_{½}) values were calculated as ln(2)/k. Area under the concentration-time curve (AUC) values were estimated using linear trapezoidal method. AUCₜ values were calculated from the dosing time to the last measurable concentration. AUC_{∞} values were calculated as the sum of the corresponding AUCₜ and the ratio of the last detectable concentration divided by k. Plasma clearance (CL) was calculated from Dose/AUC_{∞}. Mean resident time (MRT) was estimated by moment analysis. Volume of distribution at steady state (Vₛₛ) was calculated from MRT_{∞}× CL. Maximum concentration (Cₘₐₓ) and time to reach Cₘₐₓ (tₘₐₓ) were recorded as observed. Bioavailability was calculated dAUC_{∞},ₚₒ/dAUC_{∞,iv} ×100% where dAUC was the dose normalized AUC value. Data for compounds tested are provided in Table B-2. Compounds tested were prepared in accordance with the synthetic procedures described herein.

**Table B-2**

| **Compound** | **CL (mL/min/kg)** | **t_{1/2} (h)** | **Vₛₛ (L/kg)** |
|---|---|---|---|
| **1** | 28.4 | 1.77 | 4.2 |
| Comparator B | 21.45 | 2.96 | 4.51 |
| Comparator C | 4.67 | 2.6 | 1.39 |
| Comparator E | 11.6 | 2.8 | 3.28 |

### Rat Single Dose Studies

Male Sprague Dawley rats were obtained from Zhejiang Vital River Laboratory Animal Technology Co., Ltd. The animals in IV group had free access to water and food. The animals in PO group were fasted overnight before dosing and provided with food 2 hours post dosing. The IV dose solution was prepared in 10% DMA/50% PG/40% HPβCD solution (40% w/v aqueous HPβCD) at a concentration of 1 mg/mL. . The oral dosing suspension was prepared by suspending Test Article in 0.5% HPMC/0.1% Tween 80 in water at a concentration 0.2 mg/mL. Concentrations of IV and PO doses were measured at the end of the study. Pharmacokinetic parameters were calculated using the nominal dose values if the measured values were within 20% of the nominal values. Three rats were dosed IV via a bolus injection via caudal vein. Three rats per dose group were dosed by oral gavage. Blood samples were collected from the jugular vein cannula at predose, 5 (IV only),15, 30 minutes, and 1, 2, 4, 6, and 24 hours post-dose. Blood volumes were replaced with an equal amount of sterile 0.9% saline. Blood samples were centrifuged and the collected plasma was stored at - 80°C for subsequent analysis. Plasma samples were analyzed for Test Article concentrations using a LC/MS/MS method. Briefly, a 50 µL aliquot of each plasma sample was mixed with 100 µL of acetonitrile that contained an internal standard. The mixture was vortexed and centrifuged. Ten (10) µL of the resulting solution was injected onto a reverse-phase C18 column and the resultant peaks detected on a LC/MS/MS equipped with a turbo ionspray ionization source. Sample concentrations below the limit of quantification (BLQ) were treated as zero for pharmacokinetic calculations. Pharmacokinetic parameters were estimated from individual animals using noncompartmental analysis of the concentration-time data (Phoenix WinNonLin software, version 64; Pharsight, Mountain View, CA). The elimination rate constant (k) was calculated as the absolute value of the slope of the linear regression of logarithm (log) of the concentration versus time for the last three data points of the concentration-time profiles. Apparent elimination half-life (t_{½}) values were calculated as ln(2)/k. Area under the concentration-time curve (AUC) values were estimated using linear trapezoidal method. AUCₜ values were calculated from the dosing time to the last measurable concentration. AUC_{∞} values were calculated as the sum of the corresponding AUCₜ and the ratio of the last detectable concentration divided by k (AUC_{t-∞}). Plasma clearance (CL) was calculated from Dose/AUC_{∞}. Mean resident time (MRT) was estimated by moment analysis. Volume of distribution at steady state (Vₛₛ) was calculated from MRT_{∞} × CL. Maximum concentration (Cₘₐₓ) and time to reach Cₘₐₓ (tₘₐₓ) were recorded as observed. Bioavailability was calculated from the ratio of dose normalized AUC_{∞,po} of individual rats/mean dAUC_{∞,iv} × 100% where dAUC was the dose normalized AUC value. Data for compounds tested are provided in Table B-3. Compounds tested were prepared in accordance with the synthetic procedures described herein.

**Table B-3**

| **Compound** | **CL (mL/min/kg)** | **t_{1/2} (h)** | **Vₛₛ (L/kg)** |
|---|---|---|---|
| **1** | 52.57 | 3.34 | 12.98 |
| Comparator A | 29.59 | 6.48 | 15.05 |
| Comparator B | 41.86 | 5.87 | 16.58 |
| Comparator C | 20.23 | 3.3 | 5.2 |
| Comparator D | 8.23 | 4.84 | 2.98 |
| Comparator E | 18.75 | 5.4 | 8.23 |

### Dog Single Dose Studies

Non-naive male beagle dogs (8 months -3 years of age, body weight 8-13 kg) were used in this study. All animals for IV administration had free access to food and water; all animals for PO were fasted overnight prior to dosing and were fed approximately 6 hours after dosing. For the animals in PO group, pentagastrin (6.0 µg/kg, i.m.) was administrated 20 minutes before dosing PO formulation and 1.5 hours after the second pentagastrin dosing. The dosing volume was 0.024 mL/kg, the concentration was 250 µg/mL in DMSO/1 N NaOH/PBS. 10 mL of 0.001 N HCl was used to wash the gavage catheter for each animal. The IV dose solution was prepared in 10% DMA/50% PG/40% HPβCD solution (40% w/v aqueous HPβCD) at a concentration of 1.0 mg/mL. The oral dose suspension was prepared by suspending the compound in 0.5% HPMC/0.1% Tween 80 in distilled water at a concentration of 0.2 mg/mL. Concentrations of IV and PO doses were measured at the end of the study. PK parameters were calculated using the nominal dose values if the measured values were within 20% of the nominal values. Blood samples were collected by venipuncture of peripheral veins except the dosing vein at pre-dose, 5 15, 30 min, 1, 2, 4, 6, 8, 24 and 48 h post-dose. Blood samples were centrifuged and resultant plasma was frozen for bioanalysis. Plasma samples were stored at -80°C before analysis. Plasma samples were analyzed for compound concentrations using the LC/MS/MS method. Briefly, a 50 µL aliquot of each plasma sample was mixed with 100 µL of acetonitrile that contained an internal standard. The mixture was vortexed and centrifuged. Ten (10) µL of the resulting solution was injected onto a reverse-phase C18 column and the resultant peaks detected on a LC/MS/MS equipped with a turbo ionspray ionization source. Sample concentrations below the limit of quantification (BLQ) were treated as zero for PK calculations. PK parameters were estimated from individual animals using noncompartmental analysis of the concentration-time data (Phoenix WinNonLin software, version 64; Pharsight, Mountain View, CA). The elimination rate constant (k) was calculated as the absolute value of the slope of the linear regression of logarithm (log) of the concentration versus time for the last three data points of the concentration-time profiles. Apparent elimination half-life (t_{½}) values were calculated as ln(2)/k. Area under the concentration-time curve (AUC) values were estimated using linear trapezoidal method. AUCₜ values were calculated from the dosing time to the last measurable concentration. AUC_{∞} values were calculated as the sum of the corresponding AUCₜ and the ratio of the last detectable concentration divided by k. Plasma clearance (CL) was calculated from Dose/AUC_{∞}. Mean resident time extrapolated to infinity (MRT_{∞}) was estimated by moment analysis. Vₛₛ was calculated from MRT_{∞} × CL. Maximum concentration (Cₘₐₓ) and time to reach Cₘₐₓ (tₘₐₓ) were recorded as observed. As this was a cross-over study, bioavailability was calculated dAUC_{∞,po}/dAUC_{∞,iv} × 100% where dAUC was the dose normalized AUC value from the same animal given IV and PO dose. Data for compounds tested are provided in Table B-4. Compounds tested were prepared in accordance with the synthetic procedures described herein.

**Table B-4**

| **Compound** | **CL (mL/min/kg)** | **t_{1/2} (h)** | **Vₛₛ (L/kg)** |
|---|---|---|---|
| **1** | 7.57 | 9.54 | 4.23 |
| Comparator A | 1.59 | 39.17 | 5.2 |
| Comparator B | 2.39 | 45.88 | 6.51 |
| Comparator C | 4.92 | 14.66 | 5.91 |
| Comparator D | 3.55 | 23.69 | 6.7 |
| Comparator E | 1.49 | 33.1 | 4.1 |

### Monkey Single Dose Studies

Non-naive Male Cynomolgus Monkey (2-5 years of age, body weight 2-5 kg) used in this study were obtained from Topgene Biotechnology. All animals for IV administration had free access to food and water; all animals for PO were fasted overnight prior to dosing and were fed approximately 6 hours after dosing. The IV dose solution was prepared in 10% DMA/50% PG/40% HPβCD solution (40% w/v aqueous HPβCD) at a concentration of 1.0 mg/mL. The oral dose suspension was prepared by suspending the compound in 0.5% HPMC/0.1% Tween 80 in distilled water at a concentration of 0.2 mg/mL. Concentrations of IV and PO doses were measured at the end of the study. PK parameters were calculated using the nominal dose values if the measured values were within 20% of the nominal values. Blood samples were collected by venipuncture of peripheral veins except the dosing vein at pre-dose, 5 15, 30 min, 1, 2, 4, 6, 8, 24 and 48 h post-dose. Blood samples were centrifuged and resultant plasma was frozen for bioanalysis. Plasma samples were stored at -80°C before analysis. Plasma samples were analyzed for compound concentrations using the LC/MS/MS method. Briefly, a 50 µL aliquot of each plasma sample was mixed with 100 µL of acetonitrile that contained an internal standard. The mixture was vortexed and centrifuged. Ten (10) µL of the resulting solution was injected onto a reverse-phase C18 column and the resultant peaks detected on a LC/MS/MS equipped with a turbo ionspray ionization source. Sample concentrations below the limit of quantification (BLQ) were treated as zero for PK calculations. PK parameters were estimated from individual animals using noncompartmental analysis of the concentration-time data (Phoenix WinNonLin software, version 64; Pharsight, Mountain View, CA). The elimination rate constant (k) was calculated as the absolute value of the slope of the linear regression of logarithm (log) of the concentration versus time for the last three data points of the concentration-time profiles. Apparent elimination half-life (t_{½}) values were calculated as ln(2)/k. Area under the concentration-time curve (AUC) values were estimated using linear trapezoidal method. AUCₜ values were calculated from the dosing time to the last measurable concentration. AVC_{∞} values were calculated as the sum of the corresponding AUCₜ and the ratio of the last detectable concentration divided by k. Plasma clearance (CL) was calculated from Dose/AVC_{∞}. Mean resident time extrapolated to infinity (MRT_{∞}) was estimated by moment analysis. Vₛₛ was calculated from MRT_{∞} × CL. Maximum concentration (Cₘₐₓ) and time to reach Cₘₐₓ (tₘₐₓ) were recorded as observed. As this was a cross-over study, bioavailability was calculated dAUC_{∞,po}/dAUC_{∞,iv} × 100% where dAUC was the dose normalized AUC value from the same animal given IV and PO dose. Data for compounds tested are provided in Table B-5. Compounds tested were prepared in accordance with the synthetic procedures described herein.

**Table B-5**

| **Compound** | **CL (mL/min/kg)** | **t_{1/2} (h)** | **Vₛₛ (L/kg)** |
|---|---|---|---|
| **1** | 11.6 | 9.94 | 8.89 |
| Comparator B | 8.72 | 16.22 | 9.96 |
| Comparator C | 13.78 | 11.56 | 12.03 |
| Comparator D | 6.92 | 18.12 | 12.43 |
| Comparator E | 3.4 | 16 | 4.35 |

### Projected Single-Dose CL and Vₛₛ values in Humans

Allometric scaling for the prediction of human Clearance and Volume of Distribution was based on interspecies simple allometric scaling of mouse, rat, dog, and cynomolgus monkey intravenous pharmacokinetic parameters (Boxenbaum, J Pharmacokinet Biopharm 10:201-27, 1982). Prediction of human CL was performed by extrapolating pre-clinical species' plasma intravenous clearance. The 'rule of exponents' (Mahmood & Balian, Life Sci. 59:579-85, 1996) was examined in this prediction, where it has been proposed that, when the exponent of simple allometry lies between 0.71 and 0.99, a correction factor based on Maximum Life Span (MLP) of this species can be applied and, when the exponent of simple allometry is greater than 1.0, a correction factor based on Brain Weight (BrW) can be applied, or a protein binding correction can be applied when available. In a similar manner, simple allometric scaling was employed to predict human Volume of Distribution. This method has been used successfully for varied drugs (Ward & Smith, Drug Metab Dispos 32:612-19, 2004; McGinnity et al., Curr Drug Metab 8:463-79, 2007). Projected data for selected compounds are provided in Table B-6.

**Table B-6**

| **Compound** | **Projected CL (mL/min/kg)** | **Projected t_{1/2} (h)** | **Projected t_{1/2} Method** | **Projected Vₛₛ (L/kg)** |
|---|---|---|---|---|
| **1** | 1.84 | 28.3 | SA, ROE, MLP correction, fu correction | 4.51 |
| Comparator A | 1.44 | 38.7 | SA, fu correction | 41.2 |
| Comparator B | 1.96 | 54.5 | SA, ROE, fu correction | 9.25 |
| Comparator C | 3.9 | 41 | SA, ROE, MLP correction, fu correction | 13.9 |
| Comparator D | 0.96 | 62 | SA, ROE, MLP correction, fu correction | 5.16 |
| Comparator E | 1.2 | 51.7 | SA, ROE, fu correction | 5.5 |

Key for Table B-6: SA = Simple Allometry; ROE = Rule of Exponents; fu correction = function unbound in plasma correction; MLP correction = Maximum Life Span Correction; BrW correction = Brain Weight correction

### Dog Cassette Dosing

Non-naive male beagle dogs (8 months to 3 years of age, body weight 8-14 kg) sourced from Jiangsu Johnsen Bioresource CO. and/or Beijing Rixinkeji CO., LTD and/or Beijing Marshall Biotechnology CO., LTD were used in this study. All animals for IV administration had free access to food and water. The IV dose solution was prepared in 10% DMA/50% PG/40% HPβCD solution (40% w/v aqueous HPβCD) at a concentration of 0.2 mg/mL. Concentrations of IV dose was measured at the end of the study. PK parameters were calculated using the nominal dose values if the measured values were within 20% of the nominal values. Blood samples were collected by venipuncture of peripheral veins except the dosing vein at pre-dose, 5 15, 30 min, 1, 2, 4, 6, 8 and 24 h post-dose. Blood samples were centrifuged and resultant plasma was frozen for bioanalysis. Plasma samples were stored at -80°C before analysis. Plasma samples were analyzed for compound concentrations using the LC/MS/MS method. Briefly, a 50 µL aliquot of each plasma sample was mixed with 100 µL of acetonitrile that contained an internal standard. The mixture was vortexed and centrifuged. Ten (10) µL of the resulting solution was injected onto a reverse-phase C18 column and the resultant peaks detected on a LC/MS/MS equipped with a turbo ionspray ionization source. Sample concentrations below the limit of quantification (BLQ) were treated as zero for PK calculations. PK parameters were estimated from individual animals using noncompartmental analysis of the concentration-time data (Phoenix WinNonLin software, version 64; Pharsight, Mountain View, CA). The elimination rate constant (k) was calculated as the absolute value of the slope of the linear regression of logarithm (log) of the concentration versus time for the last three data points of the concentration-time profiles. Apparent elimination half-life (t_{½}) values were calculated as ln(2)/k. Area under the concentration-time curve (AUC) values were estimated using linear trapezoidal method. AUCₜ values were calculated from the dosing time to the last measurable concentration. AUC_{∞} values were calculated as the sum of the corresponding AUCₜ and the ratio of the last detectable concentration divided by k. Plasma clearance (CL) was calculated from Dose/AUC_{∞}. Mean resident time extrapolated to infinity (MRT_{∞}) was estimated by moment analysis. Vₛₛ was calculated from MRT_{∞} × CL. Maximum concentration (Cₘₐₓ) and time to reach Cₘₐₓ (tₘₐₓ) were recorded as observed. As this was a cross-over study, bioavailability was calculated dAUC_{∞,po}/dAUC_{∞,iv} × 100% where dAUC was the dose normalized AUC value from the same animal given IV and PO dose. Data for the compound 5-(3,4-difluorobenzyl)-8-((1*r*,4*r*)-4-methylcyclohexyl)-6,9-dioxo-2,5,8-triazaspiro[3.5]nonane-2-carbaldehyde (Compound **1)** are provided in Table B-7. The compound tested was prepared in accordance with the synthetic procedures described herein.

**Table B-7**

| **Compound** | **CL (mL/min/kg)** | **t_{1/2} (h)** | **Vₛₛ (L/kg)** |
|---|---|---|---|
| **1** | 5.12 | 11.8 | 4.48 |

### Monkey Cassette Dosing

Non-naive male beagle dogs (2-5 years of age, body weight 2-5 kg) sourced from Topgene Biotechnology were used in this study. All animals for IV administration had free access to food and water. The IV dose solution was prepared in 10% DMA/50% PG/40% HPβCD solution (40% w/v aqueous HPβCD) at a concentration of 0.2 mg/mL. Concentrations of IV dose was measured at the end of the study. PK parameters were calculated using the nominal dose values if the measured values were within 20% of the nominal values. Blood samples were collected by venipuncture of peripheral veins except the dosing vein at pre-dose, 5 15, 30 min, 1, 2, 4, 6, 8 and 24 h post-dose. Blood samples were centrifuged and resultant plasma was frozen for bioanalysis. Plasma samples were stored at -80°C before analysis. Plasma samples were analyzed for compound concentrations using the LC/MS/MS method. Briefly, a 50 µL aliquot of each plasma sample was mixed with 100 µL of acetonitrile that contained an internal standard. The mixture was vortexed and centrifuged. Ten (10) µL of the resulting solution was injected onto a reverse-phase C18 column and the resultant peaks detected on a LC/MS/MS equipped with a turbo ionspray ionization source. Sample concentrations below the limit of quantification (BLQ) were treated as zero for PK calculations. PK parameters were estimated from individual animals using noncompartmental analysis of the concentration-time data (Phoenix WinNonLin software, version 64; Pharsight, Mountain View, CA). The elimination rate constant (k) was calculated as the absolute value of the slope of the linear regression of logarithm (log) of the concentration versus time for the last three data points of the concentration-time profiles. Apparent elimination half-life (t_{½}) values were calculated as ln(2)/k. Area under the concentration-time curve (AUC) values were estimated using linear trapezoidal method. AUCₜ values were calculated from the dosing time to the last measurable concentration. AUC_{∞} values were calculated as the sum of the corresponding AUCₜ and the ratio of the last detectable concentration divided by k. Plasma clearance (CL) was calculated from Dose/AUC_{∞}. Mean resident time extrapolated to infinity (MRT_{∞}) was estimated by moment analysis. Vₛₛ was calculated from MRT_{∞} × CL. Maximum concentration (Cₘₐₓ) and time to reach Cₘₐₓ (tₘₐₓ) were recorded as observed. As this was a cross-over study, bioavailability was calculated dAUC_{∞,po}/dAUC_{∞,iv} × 100% where dAUC was the dose normalized AUC value from the same animal given IV and PO dose. Data for the compound 5-(3,4-difluorobenzyl)-8-((1*r*,4*r*)-4-methylcyclohexyl)-6,9-dioxo-2,5,8-triazaspiro[3.5]nonane-2-carbaldehyde (Compound **1)** are provided in Table B-8. The compound tested was prepared in accordance with the synthetic procedures described herein.

**Table B-8**

| **Compound** | **CL (mL/min/kg)** | **t_{1/2} (h)** | **Vₛₛ (L/kg)** |
|---|---|---|---|
| **1** | 13.1 | 8.69 | 8.8 |

### Projected Cassette-Dosing CL and Vₛₛ Values in Humans

For PK data derived from Cassette IV dosing, predictions of Human Clearance and Volume of Distribution were performed using Single Species allometry. In this case values were predicted from plasma intravenous Clearance of Dog and Monkey PK by applying protein binding correction (Tang, Drug Metab Dispos 33:1294-96, 2005; Patel, Journal of Pharmaceutical Research International, 22(3): 1-7, 2018). Projected data for the compound 5-(3,4-difluorobenzyl)-8-((1*r*,4*r*)-4-methylcyclohexyl)-6,9-dioxo-2,5,8-triazaspiro[3.5]nonane-2-carbaldehyde (Compound **1)** are provided in Table B-9 and Table B-10.

**Table B-9**

| **Compound** | **Projected Human Single Species Allometry: Dog** | **Projected Human Single Species Allometry: Dog** | **Projected Human Single Species Allometry: Dog** |
|---|---|---|---|
| | **CL (mL/min/kg)** | **t_{1/2} (h)** | **Vₛₛ (L/kg)** |
| **1** | 1.5 | 25.8 | 22 |

**Table B-10**

| **Compound** | **Projected Human Single Species Allometry: Monkey CL (mL/min/kg)** | **Projected Human Single Species Allometry: Monkey t_{1/2} (h)** | **Projected Human Single Species Allometry: Monkey Vₛₛ (L/kg)** |
|---|---|---|---|
| **1** | 3.1 | 15.84 | 71 |

### Biological Example B-3

### Echocardiography assessment of acute pharmacodynamic effect in rat cardiac contractility

Assessment of in vivo cardiac function by echocardiography was performed in male Sprague Dawley rats under isoflurane (1-3%) anesthesia. 2-D M-mode images of the left ventricle were acquired in the parasternal long-axis view before, during, and after administration of the compound 5-(3,4-difluorobenzyl)-8-((1*r*,4*r*)-4-methylcyclohexyl)-6,9-dioxo-2,5,8-triazaspiro[3.5]nonane-2-carbaldehyde. *In vivo* fractional shortening was determined by M-mode image analysis with the following calculation: ((End diastolic diameter - end systolic diameter)/ end diastolic diameter x 100). Three pre-dose baseline M-Mode images were taken at 1-minute intervals prior to compound administration. The compound 5-(3,4-difluorobenzyl)-8-((1*r*,4*r*)-4-methylcyclohexyl)-6,9-dioxo-2,5,8-triazaspiro[3.5]nonane-2-carbaldehyde was formulated in a 0.5% hydroxypropyl methylcellulose 2910 (HPMC 2910): 0.1% Tween 80 suspension and delivered as a single dose (5 mL/kg) by oral gavage. At one and four hours post-dose, rats were lightly anesthetized for M-mode echocardiography measurement. Concurrent with echocardiography measurements, blood samples were taken to determine the corresponding compound plasma concentrations. The resulting plasma concentrations were used to estimate the IC₅₀ and IC₁₀ values, which is the concentration at which fractional shortening is 50% and 10% of the pre-dose baseline contractility, respectively. Data for the compound 5-(3,4-difluorobenzyl)-8-((1r,4r)-4-methylcyclohexyl)-6,9-dioxo-2,5,8-triazaspiro[3.5]nonane-2-carbaldehyde (Compound **1)** are provided in Table B-11.

**Table B-11. IC₅₀, IC₁₀ values and IC₅₀/IC₁₀ ratio**

| **Compound** | **IC₁₀ (µM)** | **IC₅₀ (µM)** | **IC₅₀/IC₁₀** |
|---|---|---|---|
| **1** | 0.284 | 2.763 | 9.73 |

### Biological Example B-4

### In Vitro Determination of Time-Dependent inhibition of CYP450 enzymes

An assessment of the time-dependent Inhibitory Potential of the test compounds against Principal Human Cytochrome P450 Isozymes using Human Liver Microsomes was also carried out using standard methods (Grimm et al, Drug Metab. Dispos., Jul; 37(7):1355-70. doi: 10.1124/dmd.109.026716, 2009). Pooled human microsomes and selective CYP probe substrates were used for *in vitro* assessment of test compounds at 25 and 50 µM as time-dependent inhibitors of seven human hepatic cytochrome P450 isozymes (CYP1A2, 2B6, 2C9, 2C19, 2D6, and 3A4). LC-MS/MS was used to quantify metabolite formation. The inhibition of each P450 enzyme in human liver microsomes was measured as the percentage decrease in the activity of marker metabolite formation as measured by LC-MS/MS compared to non-inhibited controls (= 100% activity) at time zero and after 30 minutes incubation. The occurrence of any time dependent inhibition was then expressed as the fold change in enzyme activity at time zero relative to the activity after 30 minutes incubation. The time dependent inhibition for the compound 5-(3,4-difluorobenzyl)-8-((1*r*,4*r*)-4-methylcyclohexyl)-6,9-dioxo-2,5,8-triazaspiro[3.5]nonane-2-carbaldehyde (Compound **1)** against CYP1A2, CYP2B6, CYP2C8, CYP2C9, CYP2C19, CYP2D6, CYP3A4-M and CYP3A4-T is shown in Table B-12.

**Table B-12**

| | | **CYP1A2** | | | **CYP2B6** | | |
|---|---|---|---|---|---|---|---|
| **Compound** | **Conc (µM)** | **% Activity NADPH T0** | **% Activity NADPH T30** | **(% Activity NADPH T0) /(% Activity NADPH T30)** | **% Activity NADPH T0** | **% Activity NADPH T30** | **(% Activity NADPH T0) /(% Activity NADPH T30)** |
| Comparator | 25 | N/A | N/A | N/A | N/A | N/A | N/A |
| C | 50 | N/A | N/A | N/A | N/A | N/A | N/A |
| **1** | 25 | 95 | 90 | 1.1 | 95 | 84 | 1.1 |
| | 50 | 86 | 85 | 1.0 | 76 | 76 | 1.0 |

| | | **CYP2C9** | | | **CYP2C19** | | |
|---|---|---|---|---|---|---|---|
| **Compound No.** | **Conc (µM)** | **% Activity NADPH T0** | **% Activity NADPH T30** | **(% Activity NADPH T0) /(% Activity NADPH T30)** | **% Activity NADPH T0** | **% Activity NADPH T30** | **(% Activity NADPH T0) /(% Activity NADPH T30)** |
| Comparator | 25 | N/A | N/A | N/A | N/A | N/A | N/A |
| C | 50 | N/A | N/A | N/A | N/A | N/A | N/A |
| **1** | 25 | 83 | 84 | 1.0 | 117 | 86 | 1.4 |
| | 50 | 63 | 71 | 0.9 | 84 | 75 | 1.1 |

| | | **CYP2D6** | | | **CYP2C8** | | |
|---|---|---|---|---|---|---|---|
| **Compound** | **Conc (µM)** | **% Activity NADPH T0** | **% Activity NADPH T30** | **(% Activity NADPH T0) /(% Activity NADPH T30)** | **% Activity NADPH T0** | **% Activity NADPH T30** | **(% Activity NADPH T0) /(% Activity NADPH T30)** |
| Comparator | 25 | N/A | N/A | N/A | N/A | N/A | N/A |
| C | 50 | N/A | N/A | N/A | N/A | N/A | N/A |
| **1** | 25 | 118 | 90 | 1.3 | 79 | 80 | 1.0 |
| | 50 | 93 | 88 | 1.1 | 97 | 79 | 1.2 |

| **Compound** | **Conc (µM)** | **CYP3A4-T** | | | **CYP3A4-M** | | |
|---|---|---|---|---|---|---|---|
| | | **% Activity NADPH T0** | **% Activity NADPH T30** | **(% Activity NADPH T0) /(% Activity NADPH T30)** | **% Activity NADPH T0** | **% Activity NADPH T30** | **(% Activity NADPH T0) /(% Activity NADPH T30)** |
| Comparator | 25 | 41 | 23 | 1.8 | 75 | 48 | 1.6 |
| C | 50 | 35 | 19 | 1.8 | 57 | 33 | 1.7 |
| **1** | 25 | 80 | 90 | 0.9 | 70 | 88 | 0.8 |
| | 50 | 83 | 98 | 0.8 | 57 | 78 | 0.7 |

NA: not available; CYP3A4-T : CYP3A4 activity as measured by the testosterone probe substrate; CYP3A4-M : CYP3A4 activity as measured by the midazolam probe substrate

For 3A4, measuring the % activity using both midalzolam and testosterone as probes, 5-(3,4-difluorobenzyl)-8-((1*r*,4*r*)-4-methylcyclohexyl)-6,9-dioxo-2,5,8-triazaspiro[3.5]nonane-2-carbaldehyde (Compound **1)** does not show any indication of time dependent inhibition since the activity of the enzyme did not change by greater than 1.2 fold. Comparator C however did show a change in fold activity of > 1.5 fold for both probes at the 25 and 50 µM concentrations, suggesting that there may be some change in 3A4 activity when tested in time dependent format for Comparator C. Both the compounds tested showed no change in activity for 1A2 and 2B6 when tested in this format. For 2C19, Compound **1** showed a 1.4 fold change in activity at the 25 µM concentration, although no change in activity was observed for Compound **1** at the 50 µM concentration. For 2D6, Compound **1** showed a 1.3 fold change in activity at the 25 µM concentration, although no change in activity was observed for Compound **1** at the 50 µM concentration.

While the foregoing written description of the compounds, uses, and methods described herein enables one of ordinary skill to make and use the compounds, uses, and methods described herein, those of ordinary skill will understand and appreciate the existence of variations, combinations, and equivalents of the specific embodiment, method, and examples herein. The compounds, uses, and methods provided herein should therefore not be limited by the above-described embodiments, methods, or examples, but rather encompasses all embodiments and methods within the scope and spirit of the compounds, uses, and methods provided herein.

All references disclosed herein are incorporated by reference in their entirety.

### Numbered clauses

1. A crystalline form of the compound of Formula 1: 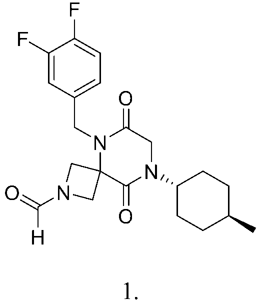
2. The crystalline form of clause 1, characterized by having an XRPD pattern comprising peaks at angles 2-theta of 5.9±0.2, 11.5±0.2, 11.7±0.2, 17.9±0.2, and 19.1±0.2 degrees.
3. The crystalline form of clause 2, characterized by having an XRPD pattern comprising additional peaks at angles 2-theta of 7.8±0.2, and 16.2±0.2 degrees.
4. The crystalline form of any one of clauses 2 or 3, characterized by having an XRPD pattern comprising additional peaks at angles 2-theta of 13.1±0.2, and 19.8±0.2 degrees.
5. The crystalline form of any one of clauses 1-4, characterized by having an XRPD pattern substantially as shown in FIG. 2A.
6. The crystalline form of any one of clauses 1-5, characterized by having a DSC graph substantially as shown in FIG. 2B.
7. The crystalline form of any one of clauses 1-6, characterized by having an endotherm onset at 154.9±2 °C as determined by DSC.
8. The crystalline form of any one of clauses 1-7, characterized by having an endotherm peak at 155.8±2 °C as determined by DSC.
9. The crystalline form of any one of clauses 1-8, characterized by having a TGA graph substantially as shown in FIG. 2B.
10. The crystalline form of any one of clauses 1-9, characterized by having less than 0.1% weight loss prior to degradation as determined by TGA.
11. The crystalline form of any one of clauses 1-10, characterized by having a GVS graph substantially as shown in FIG. 2C.
12. The crystalline form of clause 1, characterized by having an XRPD pattern comprising peaks at angles 2-theta of 6.0±0.2, 10.2±0.2, 21.6±0.2, and 22.1±0.2 degrees.
13. The crystalline form of clause 12, characterized by having an XRPD pattern comprising additional peaks at angles 2-theta of 17.9±0.2, and 24.1±0.2 degrees.
14. The crystalline form of any one of clauses 12 or 13, characterized by having an XRPD pattern comprising additional peaks at angles 2-theta of 16.0±0.2, 16.6±0.2, 17.3±0.2, 17.6±0.2, and 20.5±0.2 degrees.
15. The crystalline form of any one of clauses 1 and 12-14, characterized by having an XRPD pattern substantially as shown in FIG. 1A.
16. The crystalline form of any one of clauses 1 and 12-15, characterized by having a DSC graph substantially a shown in FIG. 1B.
17. The crystalline form of any one of clauses 1 and 12-16, characterized by having an endotherm onset at 125.6±2 °C as determined by DSC.
18. The crystalline form of any one of clauses 1 and 12-17, characterized by having an endotherm peak at 130.2±2 °C as determined by DSC.
19. The crystalline form of any one of clauses 1 and 12-18, characterized by having a TGA graph substantially as shown in FIG. 1B.
20. The crystalline form of any one of clauses 1 and 12-19, characterized by having a weight loss of 0.35%±0.05% between 105 °C and 145 °C, as determined by TGA.
21. The crystalline form of any one of clauses 1 and 12-20, characterized by having a GVS graph substantially as shown in FIG. 1C.
22. A method of preparing the crystalline form of any one of clauses 2-11, comprising (1) forming a mixture of 5-(3,4-difluorobenzyl)-8-((1r,4r)-4-methylcyclohexyl)-6,9-dioxo-2,5,8-triazaspiro[3.5]nonane-2-carbaldehyde and a solvent selected from the group consisting of an alcohol, water and mixtures thereof, and (2) cooling the mixture of step (1).
23. The method of clause 22, wherein the solvent comprises ethanol and water.
24. The method of clause 22 or 23, wherein step (1) comprises stirring the mixture at a temperature of 40 °C to 60 °C.
25. The method of any one of clauses 22-24, wherein the step (2) comprises cooling the mixture of step (1) to a temperature of 0 °C to 10 °C.
26. A method of preparing the crystalline form of any one of clauses 2-11, comprising (1) forming a mixture of 5-(3,4-difluorobenzyl)-8-((1r,4r)-4-methylcyclohexyl)-6,9-dioxo-2,5,8-triazaspiro[3.5]nonane-2-carbaldehyde and a first solvent; (2) heating the mixture of step (1) to a first temperature between 75 °C and 95 °C; (3) adding a second solvent to the mixture of step (2); (4) cooling the mixture of step (3) to a second temperature between 10 °C and 30 °C; and (5) filtering the mixture of step (4) to obtain the crystalline form.
27. The method of clause 26, wherein step (4) further comprises cooling the mixture of step (3) to an intermediate temperature between 50 °C and 75 °C, adding seed crystals of the crystalline form, and further cooling the mixture to the second temperature.
28. The method of clause 26 or 27, wherein the first solvent is ethanol or a mixture of ethanol and water.
29. The method of any one of the clauses 26-28, wherein the second solvent is water.
30. A pharmaceutical composition comprising the crystalline form of any one of clauses 1- 21, and a pharmaceutically acceptable excipient.
31. A method of treating heart disease in a subject in need thereof, comprising administering to the subject the crystalline form of any one of clauses 1-21, or the pharmaceutical composition of clause 30.
32. The method of clause 31, wherein the heart disease is hypertrophic cardiomyopathy or heart failure with preserved ejection fraction.
33. The method of clause 32, wherein the hypertrophic cardiomyopathy is obstructive or nonobstructive or is caused by (i) sarcomeric mutations, (ii) non-sarcomeric mutations, or (iii) both sarcomeric mutations and non-sarcomeric mutations.
34. The method of clause 31, wherein the heart disease is selected from the group consisting of diastolic dysfunction, primary or secondary restrictive cardiomyopathy, myocardial infarction and angina pectoris, left ventricular outflow tract obstruction, hypertensive heart disease, congenital heart disease, cardiac ischemia, coronary heart disease, diabetic heart disease, congestive heart failure, right heart failure, cardiorenal syndrome, and infiltrative cardiomyopathy; or wherein the heart disease is or is related to one or more conditions selected from the group consisting of cardiac senescence, diastolic dysfunction due to aging, left ventricular hypertrophy, and concentric left ventricular remodeling.
35. A method of treating a disease or condition associated with hypertrophic cardiomyopathy, secondary left ventricular wall thickening, small left ventricular cavity and cavity obliteration, hyperdynamic left ventricular contraction, myocardial ischemia, or cardiac fibrosis, or a disease or condition selected from muscular dystrophies and glycogen storage diseases in a subject in need thereof, comprising administering to the subject a crystalline form of any one of clauses 1-21, or a pharmaceutical composition of clause 30.
36. The method of clause 35, wherein the disease or condition is selected from the group consisting of Fabry's Disease, Danon Disease, mitochondrial cardiomyopathies, Noonan Syndrome, hypertension, valvular heart diseases, aortic stenosis, Mitral valve regurgitation, metabolic syndromes, diabetes, obesity, end stage renal disease, scleroderma, sleep apnea, amyloidosis, Friedreich Ataxia, and Pompe disease.
37. A method of inhibiting the cardiac sarcomere, comprising contacting the cardiac sarcomere with the crystalline form of any one of clauses 1-21, or a pharmaceutical composition of clause 30.

## Claims

1. A crystalline form of the compound of Formula 1:

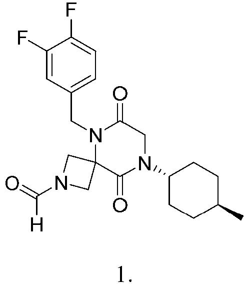

2. The crystalline form of claim 1, **characterized by** having an XRPD pattern comprising peaks at angles 2-theta of 5.9±0.2, 11.5±0.2, 11.7±0.2, 17.9±0.2, and 19.1±0.2 degrees, optionally wherein:
(a) the crystalline form is **characterized by** having an XRPD pattern comprising additional peaks at angles 2-theta of 7.8±0.2, and 16.2±0.2 degrees; and/or
(b) the crystalline form is **characterized by** having an XRPD pattern comprising additional peaks at angles 2-theta of 13.1±0.2, and 19.8±0.2 degrees.

3. The crystalline form of any one of claims 1 or 2, **characterized by**:
(a) having an XRPD pattern substantially as shown in FIG. 2A; and/or
(b) having a DSC graph substantially as shown in FIG. 2B; and/or
(c) having an endotherm onset at 154.9±2 °C as determined by DSC; and/or
(d) having an endotherm peak at 155.8±2 °C as determined by DSC; and/or
(e) having a TGA graph substantially as shown in FIG. 2B; and/or
(f) having less than 0.1% weight loss prior to degradation as determined by TGA;
and/or
(g) having a GVS graph substantially as shown in FIG. 2C.

4. The crystalline form of claim 1, **characterized by** having an XRPD pattern comprising peaks at angles 2-theta of 6.0±0.2, 10.2±0.2, 21.6±0.2, and 22.1±0.2 degrees, optionally wherein:
(a) the crystalline form is **characterized by** having an XRPD pattern comprising additional peaks at angles 2-theta of 17.9±0.2, and 24.1±0.2 degrees; and/or
(b) the crystalline form is **characterized by** having an XRPD pattern comprising additional peaks at angles 2-theta of 16.0±0.2, 16.6±0.2, 17.3±0.2, 17.6±0.2, and 20.5±0.2 degrees.

5. The crystalline form of any one of claims 1 and 4, **characterized by**:
(a) having an XRPD pattern substantially as shown in FIG. 1A; and/or
(b) having a DSC graph substantially a shown in FIG. 1B; and/or
(c) having an endotherm onset at 125.6±2 °C as determined by DSC; and/or
(d) having an endotherm peak at 130.2±2 °C as determined by DSC; and/or
(e) having a TGA graph substantially as shown in FIG. 1B; and/or
(f) having a weight loss of 0.35%±0.05% between 105 °C and 145 °C, as determined by TGA; and/or
(g) having a GVS graph substantially as shown in FIG. 1C.

6. A method of preparing the crystalline form of any one of claims 2 or 3, comprising (1) forming a mixture of 5-(3,4-difluorobenzyl)-8-((1r,4r)-4-methylcyclohexyl)-6,9-dioxo-2,5,8-triazaspiro[3.5]nonane-2-carbaldehyde and a solvent selected from the group consisting of an alcohol, water and mixtures thereof, and (2) cooling the mixture of step (1).

7. The method of claim 6, wherein:
(a) the solvent comprises ethanol and water; and/or
(b) step (1) comprises stirring the mixture at a temperature of 40 °C to 60 °C; and/or
(c) step (2) comprises cooling the mixture of step (1) to a temperature of 0 °C to 10 °C.

8. A method of preparing the crystalline form of any one of claims 2 or 3, comprising (1) forming a mixture of 5-(3,4-difluorobenzyl)-8-((1r,4r)-4-methylcyclohexyl)-6,9-dioxo-2,5,8-triazaspiro[3.5]nonane-2-carbaldehyde and a first solvent; (2) heating the mixture of step (1) to a first temperature between 75 °C and 95 °C; (3) adding a second solvent to the mixture of step (2); (4) cooling the mixture of step (3) to a second temperature between 10 °C and 30 °C; and (5) filtering the mixture of step (4) to obtain the crystalline form.

9. The method of claim 8, wherein:
(a) step (4) further comprises cooling the mixture of step (3) to an intermediate temperature between 50 °C and 75 °C, adding seed crystals of the crystalline form, and further cooling the mixture to the second temperature; and/or
(b) the first solvent is ethanol or a mixture of ethanol and water; and/or
(c) the second solvent is water.

10. A pharmaceutical composition comprising the crystalline form of any one of claims 1-5, and a pharmaceutically acceptable excipient.

11. The crystalline form of any one of claims 1-5, or the pharmaceutical composition of claim 10, for use in a method of treating heart disease in a subject in need thereof, comprising administering to the subject the crystalline form, or the pharmaceutical composition.

12. The crystalline form or the pharmaceutical composition for use according to claim 11, wherein:
(a) the heart disease is hypertrophic cardiomyopathy or heart failure with preserved ejection fraction, optionally wherein the hypertrophic cardiomyopathy is obstructive or nonobstructive or is caused by (i) sarcomeric mutations, (ii) non-sarcomeric mutations, or (iii) both sarcomeric mutations and non-sarcomeric mutations; or
(b) the heart disease is selected from the group consisting of diastolic dysfunction, primary or secondary restrictive cardiomyopathy, myocardial infarction and angina pectoris, left ventricular outflow tract obstruction, hypertensive heart disease, congenital heart disease, cardiac ischemia, coronary heart disease, diabetic heart disease, congestive heart failure, right heart failure, cardiorenal syndrome, and infiltrative cardiomyopathy; or wherein the heart disease is or is related to one or more conditions selected from the group consisting of cardiac senescence, diastolic dysfunction due to aging, left ventricular hypertrophy, and concentric left ventricular remodeling.

13. The crystalline form of any one of claims 1-5, or the pharmaceutical composition of claim 10, for use in a method of treating a disease or condition associated with hypertrophic cardiomyopathy, secondary left ventricular wall thickening, small left ventricular cavity and cavity obliteration, hyperdynamic left ventricular contraction, myocardial ischemia, or cardiac fibrosis, or a disease or condition selected from muscular dystrophies and glycogen storage diseases in a subject in need thereof, comprising administering to the subject the crystalline form, or the pharmaceutical composition.

14. The crystalline form or the pharmaceutical composition for use according to claim 13, wherein the disease or condition is selected from the group consisting of Fabry's Disease, Danon Disease, mitochondrial cardiomyopathies, Noonan Syndrome, hypertension, valvular heart diseases, aortic stenosis, Mitral valve regurgitation, metabolic syndromes, diabetes, obesity, end stage renal disease, scleroderma, sleep apnea, amyloidosis, Friedreich Ataxia, and Pompe disease.

15. The crystalline form of any one of claims 1-5, or the pharmaceutical composition of claim 10, for use in a method of inhibiting the cardiac sarcomere, comprising contacting the cardiac sarcomere with the crystalline form, or the pharmaceutical composition.
